# EUROPÄISCHE PATENTSCHRIFT

(11) **EP 2 235 011 B1**
(45) Veröffentlichungstag und Bekanntmachung des Hinweises auf die Patenterteilung: **25.01.2012**
(21) Anmeldenummer: 08864918.1
(22) Anmeldetag: 09.12.2008
(51) Int. Cl.: C07D 471/04, A61P 9/04, A61P 9/10, A61P 3/10, A61K 31/437

(54) **SUBSTITUIERTE PYRROLO[2, 3-B]- UND PYRAZOLO[3, 4-B]PYRIDINE ALS ADENOSIN REZEPTOR LIGANDEN**
SUBSTITUTED PYRROLO[2, 3-B]AND PYRAZOLO[3, 4-B]PYRIDINES FOR USE AS ADENOSINE RECEPTOR LIGANDS
PYRROLO[2,3-B]- ET PYRAZOLO[3,4-B]-PYRIDINES SUBSTITUÉES COMME LIGANDS DE RÉCEPTEUR DE L'ADÉNOSINE

(30) Priorität: 20.12.2007 DE 102007061763
(43) Veröffentlichungstag der Anmeldung: 06.10.2010
(73) Patentinhaber: Bayer Pharma Aktiengesellschaft, 13353 Berlin (DE)
(72) Erfinder: NELL, Peter, 42115 Wuppertal (DE); VAKALOPOULOS, Alexandros, 40721 Hilden (DE); SÜSSMEIER, Frank, 42277 Wuppertal (DE); ALBRECHT-KÜPPER, Barbara, 42289 Wülfrath (DE); ZIMMERMANN, Katja, 40470 Düsseldorf (DE); KELDENICH, Jörg, 42113 Wuppertal (DE); MEIBOM, Daniel, 51373 Leverkusen (DE)
(86) Internationale Anmeldenummer: PCT/EP2008/010409
(87) Internationale Veröffentlichungsnummer: WO 2009/080197

(56) Entgegenhaltungen:
- WO-A-01/25210
- WO-A-2004/074218
- KARL-NORBERT KLOTZ: "Adenosine receptors and their ligands" NAUNYN-SCHMIEDEBERGS ARCHIVES OF PHARMACOLOGY, Bd. 362, Nr. 4-5, 21. Oktober 2000 (2000-10-21), Seiten 382-391, XP002520638

## Beschreibung

Die vorliegende Anmeldung betrifft neue substituierte Pyrrolopyridin-, und Pyrazolopyridin Derivate, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, vorzugsweise zur Behandlung und/oder Prävention von kardiovaskulären Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung. In Adipozyten ist Adenosin in der Lage, die Lipolyse zu hemmen und somit die Konzentration an freien Fettsäuren und Triglyzeriden im Blut zu senken.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosin-rezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über A1-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A1-Rezeptoren durch spezifische A1-Agonisten führt beim Menschen zu einer frequenzabhängigen Senkung der Herzfrequenz, ohne einen Einfluss auf den Blutdruck zu haben. Selektive A1-Agonisten könnten somit unter anderem für die Behandlung von Angina pectoris und Vorhofflimmern geeignet sein.

Die kardioprotektive Wirkung der A1-Rezeptoren im Herzen kann unter anderem durch die Aktivierung dieser A1-Rezeptoren durch spezifische A1-Agonisten für die Behandlung und Organprotektion bei akutem Myokardinfarkt, akutem Koronarsyndrom, Herzinsuffizienz, Bypass Operationen, Herzkatheteruntersuchungen und Organtransplantationen genutzt werden.

Die Aktivierung von A2b-Rezeptoren durch Adenosin oder spezifische A2b-Agonisten führt über die Erweiterung von Gefäßen zu einer Blutdrucksenkung. Die Blutdrucksenkung ist von einem reflektorischen Herzfrequenzanstieg begleitet. Der Herzfrequenzanstieg kann durch die Aktivierung von A1-Rezeptoren durch spezifische A1-Agonisten reduziert werden.

Die kombinierte Wirkung von selektiven A1/A2b-Agonisten auf das Gefäßsystem und die Herzfrequenz resultiert somit in einer systemischen Blutdrucksenkung ohne relevanten Herzfrequenzanstieg. Mit einem solchen pharmakologischen Profil könnten duale A1/A2b-Agonisten zur Behandlung z.B. der Hypertonie beim Menschen eingesetzt werden.

Die Hemmung von A1-Rezeptoren durch spezifische A1-Antagonisten wirkt beim Menschen urikosurisch, natriuretisch sowie Kalium sparend diuretisch ohne Beeinflussung der glomerulären Filtrationsrate und somit nierenprotektiv. Selektive A1-Antagonisten können somit unter anderem zur Behandlung von akut dekompensierter Herzinsuffizienz und chronischer Herzinsuffizienz geeignet sein. Desweiteren können sie zur Nierenprotektion bei Nephropathie und anderen Nierenerkrankungen eingesetzt werden.

In Adipozyten bewirkt die Aktivierung von A1- und A2b-Rezeptoren eine Inhibition der Lipolyse. Die kombinierte Wirkung von A1/A2b-Agonisten auf den Lipidstoffwechsel führt somit zu einer Senkung von freien Fettsäuren und Triglyzeriden. Eine Senkung der Lipide wiederum führt bei Patienten mit Metabolischem Syndrom und bei Diabetikern zur Verringerung der Insulinresistenz und zur Verbesserung der Symptomatik.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis", J. Biol. Chem. 267 (1992), Seiten 10764-1077.

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells", Naunyn Schmiedebergs Arch. Pharmacol. 357 (1998), Seiten 1-9 .

Bei den aus dem Stand der Technik bekannten, als "Adenosinrezeptor-spezifisch" geltenden Liganden handelt es sich überwiegend um Derivate auf Basis des natürlichen Adenosins [S.-A. Poulsen und R. J. Quinn, "Adenosine receptors: New opportunities for future drugs", Bioorganic and Medicinal Chemistry 6 (1998), Seiten 619-641]. Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet. In klinischer Entwicklung befindliche Verbindungen dieser Art eignen sich bislang nur zur intravenösen Applikation.

In WO 95/34563 werden substituierte Pyrazolo- und Pyrrolopyridine als CRF Antagonisten für die Behandlung von stressbedingten Erkrankungen beschrieben. WO 2004/014368 offenbart 3-Pyrrolylpyridopyrazole und 3-Pyrrolylindazole als Kinase-Inhibitoren zur Behandlung von Krebs. In WO 2004/035740 werden substituierte heterobicyclische Verbindungen zur Behandlung von In WO 2004/035740 werden substituierte heterobicyclische Verbindungen zur Behandlung von unter anderem Gelenkrheumatismus, Sepsis und Multipler Sklerose beansprucht. WO 2004/058767, WO 2006/001501 und WO 2006/001511 offenbaren Pyrrolopyridine und - pyrimidine als CRF Liganden zur Behandlung von ZNS-Erkankungen und Bluthochdruck.

In WO 2001/25210 werden substituierte, nicht annellierte 2-Benzylthio-3,5-dicyano-4-aryl-6-aminopyridine als Adenosin Rezeptorliganden zur therapeutischen Anwendung beschrieben. In der WO 2004/074218 werden benzo-annellierte 3-Cyanopyridine beschrieben. In der Druckschrift K.-N. Klotz, "Adenosine receptors and their ligands", Naunyn-Schmiedebergs Archives of Pharmacology bd. 362, Nr. 4-5, 21.10. 2000 (2000-10-21), Seiten 382-391 werden bicyclische Heterocyclen als Adenosin Rezeptor Liganden offenbart, jedoch keine annellierten 3-Cyanopyridine.

Aufgabe der vorliegenden Erfindung ist die Bereitstellung neuer Verbindungen, die als selektive Liganden des Adenosin A1- und/oder des Adenosin A2b-Rezeptors wirken und als solche zur Behandlung und/oder Prävention von Krankheiten, insbesondere zur Behandlung und/oder Prävention von kardiovaskulären Erkrankungen geeignet sind.

Gegenstand der vorliegenden Erfindung sind Verbindungen der Formel (I) 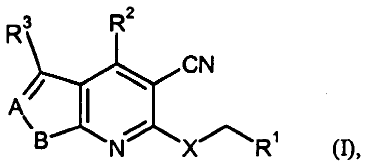 in welcher
entweder
- A: für CR⁴ oder N steht,
wobei
R⁴ für (C₁-C₄)-Alkoxycarbonyl, Aminocarbonyl, Mono-(C₁-C₄)-alkylaminocarbonyl oder Di-(C₁-C₄)-alkylaminocarbonyl steht, und
- B: für NR⁵ steht,
wobei
R³ für Wasserstoff oder (C₁-C₄)-Alkyl steht,
- X: für O oder S steht,
- R¹: für (C₆-C₁₀)-Aryl oder 5- bis 10-gliedriges Heteroaryl steht,
wobei (C₆-C₁₀)-Aryl und 5- bis 10-gliedriges Heteroaryl mit 1 oder 2 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Hydroxycarbonyl, (C₁-C₆)-Alkoxycarbonyl, Aminocarbonyl, Mono-(C₁-C₆)-alkylaminocarbonyl, Di-(C₁-C₆)-alkylaminocarbonyl, Pyrrolidino, Piperidino, Morpholino, Piperazino und *N'*-(C₁-C₄)-Alkylpiperazino, Phenyl und 5- oder 6-gliedriges Heteroaryl, substituiert sein können,
worin Phenyl und 5- oder 6-gliedriges Heteroaryl mit 1 bis 3 Substituenten unabhängig voneinander ausgewählt aus der Gruppe Halogen, Nitro, Cyano, (C₁-C₆)-Alkyl, Difluormethyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl substituiert sein können,
- R²: für (C₅-C₆)-Cycloalkyl, 5- oder 6-gliedriges Heterocyclyl, Phenyl oder 5- oder 6-gliedriges Heteroaryl steht,
wobei (C₅-C₆)-Cycloalkyl mit 1 oder 2 Substituenten unabhängig voneinander ausgewählt aus der Gruppe (C₁-C₆)-Alkyl, Hydroxy, Oxo, (C₁-C₆)-Alkoxy, Amino, Mono-(C₁-C₆)-alkylamino und Di-(C₁-C₆)-alkylamino substituiert sein kann,
und
wobei 5- oder 6-gliedriges Heterocyclyl mit 1 bis 3 Substituenten unabhängig voneinander ausgewählt aus der Gruppe Oxo, Thioxo, Hydroxy, (C₁-C₆)-Alkyl, (C₁-C₆)-Alkoxy, (C₁-C₆)-Alkylcarbonyl, Amino, Mono-(C₁-C₆)-alkylamino, Di-(C₁-C₆)-alkylamino und (C₃-C₇)-Cycloalkyl substituiert sein kann,
worin (C₁-C₆)-Alkyl mit 1 bis 3 Substituenten unabhängig voneinander ausgewählt aus der Gruppe Fluor, Oxo, Hydroxy, Trifluormethyl, (C₁-C₄)-Alkoxy, (C₁-C₄)-Alkylcarbonyloxy, Amino, Mono-(C₁-C₄)-alkylamino, Di-(C₁-C₄)-alkylamino und (C₃-C₇)-Cycloalkyl substituiert sein kann,
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
worin (C₁-C₆)-Alkylcarbonyl mit einem Substituenten ausgewählt aus der Gruppe Hydroxy und (C₁-C₄)-Alkoxy substituiert sein kann,
und
worin (C₃-C₇)-Cycloalkyl mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
wobei Phenyl und 5- oder 6-gliedriges Heteroaryl mit 1 bis 3 Substituenten unabhängig voneinander ausgewählt aus der Gruppe Halogen, Cyano, Hydroxy, (C₁-C₆)-Alkyl, (C₁-C₆)-Alkoxy, (C₃-C₇)-Cycloalkoxy und -NR^{A}R^{B} substituiert sein können,
worin (C₁-C₆)-Alkyl mit 1 bis 3 Substituenten Fluor substituiert sein kann,
und
worin (C₁-C₆)-Alkoxy mit 1 bis 3 Substituenten unabhängig voneinander ausgewählt aus der Gruppe Fluor, Trifluormethyl, (C₃-C₇)-Cycloalkyl, Oxo, Hydroxy, (C₁-C₄)-Alkoxy, Hydroxycarbonyl, Amino, Mono-(C₁-C₄)-alkylamino und Di-(C₁-C₄)-alkylamino substituiert sein kann,
und
worin (C₃-C₇)-Cycloalkoxy mit 1 oder 2 Substituenten unabhängig voneinander ausgewählt aus der Gruppe (C₁-C₄)-Alkyl, Hydroxy, Oxo und (C₁-C₄)-Alkoxy substituiert sein können,
und
worin
R^{A} für Wasserstoff oder (C₁-C₆)-Alkyl steht,
worin (C₁-C₆)-Alkyl seinerseits mit einem Substituenten ausgewählt aus der Gruppe Hydroxy und (C₁-C₄)-Alkoxy substituiert sein kann,
R^{B} für Wasserstoff, (C₁-C₆)-Alkyl, (C₃-C₇)-Cycloalkyl, (C₁-C₄)-Alkylsulfonyl oder (C₃-C₇)-Cycloalkylsulfonyl steht,
worin (C₁-C₆)-Alkyl seinerseits mit 1 oder 2 Substituenten unabhängig voneinander ausgewählt aus der Gruppe (C₃-C₇)-Cycloalkyl, Oxo, Hydroxy, (C₁-C₄)-Alkoxy, Hydroxycarbonyl; Amino, Mono-(C₁-C₄)-alkylamino und Di-(C₁-C₄)-alkylamino substituiert sein kann,
und
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
oder
worin zwei benachbarte Substituenten am Phenyl zusammen mit den Kohlenstoffatomen, an die sie gebunden sind, ein 1,3-Dioxolan oder 2,2-Difluor-1,3-dioxolan bilden können,
- R³: für Wasserstoff, (C₁-C₄)-Alkyl, (C₁-C₄)-Alkoxy, Amino, Mono-(C₁-C₄)-alkylamino, Di-(C₁-C₄)-alkylamino oder (C₁-C₄)-Alkylcarbonyl steht
sowie ihre *N*-Oxide, Salze, Solvate, Salze der *N*-Oxide und Solvate der *N*-Oxide.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.
die Koordination mit Wasser erfolgt. Als Solvate sind im Rahmen der vorliegenden Erfindung Hydrate bevorzugt.

Die erfindungsgemäßen Verbindungen können auch als Prodrugs vorliegen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 6 bzw. 1 bis 4 Kohlenstoffatomen. Bevorzugt ist ein linearer oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
Cycloalkyl steht im Rahmen der Erfindung für einen monocyclischen, gesättigten Carbocyclus mit 3 bis 7 bzw. 5 bis 6 Ring-Kohlenstoffatomen. Beispielhaft und vorzugsweise seien genannt: Cyclopropyl, Cyclobutyl, Cyclopentyl, Cyclohexyl und Cycloheptyl.
Alkylcarbonyl steht im Rahmen der Erfindung für einen linearen oder verzweigten Alkylrest mit 1 bis 6 bzw. 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.
Alkylcarbonyloxy steht im Rahmen der Erfindung für einen linearen oder verzweigten Alkylrest mit 1 bis 4 Kohlenstoffatomen und einer in 1-Position angebundenen Carbonylgruppe, der über ein Sauerstoffatom verknüpft ist. Beispielhaft und vorzugsweise seien genannt: Methylcarbonyloxy, Ethylcarbonyloxy, n-Propylcarbonyloxy, Isopropylcarbonyloxy und tert.-Butylcarbonyloxy.
Alkoxy steht im Rahmen der Erfindung für einen linearen oder verzweigten Alkoxyrest mit 1 bis 6 bzw. 1 bis 4 bzw. 2 bis 4 Kohlenstoffatomen. Bevorzugt ist ein linearer oder verzweigter Alkoxyrest mit 1 bis 4 bzw. 2 bis 4 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.
Cycloalkoxy steht im Rahmen der Erfindung für einen monocyclischen, gesättigten Alkoxyrest mit 3 bis 7 Kohlenstoffatomen. Beispielhaft und vorzugsweise seien genannt: Cyclopropyloxy, Cyclobutyloxy, Cyclopentyloxy, Cyclohexyloxy und Cycloheptyloxy.
Alkoxycarbonyl stehen im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Bevorzugt ist ein linearer oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-alkylamino steht im Rahmen der Erfindung für eine Amino-Gruppe mit einem linearen oder verzweigten Alkylsubstituenten, der 1 bis 6 bzw. 1 bis 4 bzw. 2 bis 4 Kohlenstoffatome aufweist. Bevorzugt ist ein linearer oder verzweigter Monoalkylamino-Rest mit 1 bis 4 bzw. 2 bis 4 Kohlenstoffatomen. Beispielhaft und vorzugsweise seien genannt: Methylamino, Ethylamino, n-Propylamino, Isopropylamino, n-Butylamino, tert.-Butylamino, n-Pentylamino und n-Hexylamino.
Di-alkylamino steht im Rahmen der Erfindung für eine Amino-Gruppe mit zwei gleichen oder verschiedenen linearen oder verzweigten Alkylsubstituenten, die jeweils 1 bis 6 bzw. 1 bis 4 Kohlenstoffatome aufweisen. Bevorzugt sind lineare oder verzweigte Dialkylamino-Reste mit jeweils 1 bis 4 Kohlenstoffatomen. Beispielhaft und vorzugsweise seien genannt: *N,N-*Dimethylamino, *N,N*-Diethylamino, *N*-Ethyl-*N*-methylamino, *N*-Methyl-*N*-n-propylamino, *N*-Isopropyl-*N*-n-propylamino, *N,N*-Diisopropylamino, *N*-n-Butyl-*N*-methylamino, *N*-tert.-Butyl-*N-*methylamino, *N*-Ethyl-*N*-n-pentylamino und *N*-n-Hexyl-*N*-methylamino.
Mono-alkylaminocarbonyl steht im Rahmen der Erfindung für eine Amino-Gruppe, die über eine Carbonylgruppe verknüpft ist und die einen linearen oder verzweigten Alkylsubstituenten mit 1 bis 6 bzw. 1 bis 4 Kohlenstoffatomen aufweist. Bevorzugt ist ein Mono-alkylaminocarbonyl-Rest mit 1 bis 4 Kohlenstoffatomen in der Alkylgruppe. Beispielhaft und vorzugsweise seien genannt: Methylaminocarbonyl, Ethylaminocarbonyl, *n*-Propylaminocarbonyl, Isopropylaminocarbonyl, *n-*Butylaminocarbonyl und *tert*.-Butylaminocarbonyl.
Di-alkylaminocarbonyl steht im Rahmen der Erfindung für eine Amino-Gruppe, die über eine Carbonylgruppe verknüpft ist und die zwei gleiche oder verschiedene lineare oder verzweigte Alkylsubstituenten mit jeweils 1 bis 6 bzw. 1 bis 4 Kohlenstoffatomen aufweist. Bevorzugt ist ein Dialkylaminocarbonyl-Rest mit jeweils 1 bis 4 Kohlenstoffatomen pro Alkylgruppe. Beispielhaft und vorzugsweise seien genannt: *N,N*-Dimethylaminocarbonyl, *N,N*-Diethylaminocarbonyl, *N-*Ethyl-*N*-methylaminocarbonyl, *N*-Methyl-*N*-*n*-propylaminocarbonyl, *N*-*n*-Butyl-*N*-methylaminocarbonyl und *N*-tert.-Butyl-*N*-methylaminocarbonyl.
Alkylsulfonyl steht in Rahmen der Erfindung für einen linearen oder verzweigten Alkylrest mit 1 bis 4 Kohlenstoffatomen, der über eine Sulfongruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.
Cycloalkylsulfonyl steht in Rahmen der Erfindung für einen monocyclischen, gesättigten Alkylrest mit 3 bis 7 Kohlenstoffatomen, der über eine Sulfongruppe gebunden ist. Beispielhaft und vorzugsweise seinen genannt: Cyclopropylsulfonyl, Cyclobutylsulfonyl, Cyclopentylsulfonyl, Cyclohexylsulfonyl und Cycloheptylsulfonyl.
Heterocyclyl steht im Rahmen der Erfindung für einen gesättigten Heterocyclus mit insgesamt 5 oder 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Pyrrolidinyl, Pyrazolidinyl, Tetrahydrofuranyl, Piperidinyl, Piperazinyl, Tetrahydropyranyl, Morpholinyl und Thiomorpholinyl. Bevorzugt sind Pyrrolidinyl, Tetrahydrofuranyl, Piperidinyl, Piperazinyl, Tetrahydropyranyl und Morpholinyl.
(C₆-C₁₀)-Aryl steht im Rahmen der Erfindung für einen aromatischen Carbocyclus mit 6 oder 10 Ring-Kohlenstoffatomen. Bevorzugte Arylreste sind Phenyl und Naphthyl.
Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind monocyclische 5- oder 6-gliedrige Heteroaryl-Reste mit bis zu drei Ring-Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl, Triazinyl.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Bevorzugt sind im Rahmen der vorliegenden Erfindung Verbindungen der Formel (I), in welcher
- A: für CR⁴ oder N steht,
wobei
R⁴ für (C₁-C₄)-Alkoxycarbonyl, Aminocarbonyl oder Mono-(C₁-C₄)-alkylaminocarbonyl steht,
- B: für NR⁵ steht,
wobei
R⁵ für Wasserstoff, Methyl oder Ethyl steht,
worin Methyl und Ethyl mit einem Substituenten ausgewählt aus der Gruppe Methoxycarbonyl und Ethoxycarbonyl substituiert sein können,
- X: für O oder S steht,
- R¹: für Phenyl oder 5- oder 6-gliedriges Heteroaryl steht,
wobei Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, Hydroxy, (C₁-C₄)-Alkoxy, Amino, Hydroxycarbonyl, (C₁-C₄)-Alkoxy-carbonyl, Aminocarbonyl, Phenyl und 5- oder 6-gliedriges Heteroaryl substituiert sein können,
worin Phenyl und 5- oder 6-gliedriges Heteroaryl mit 1 bis 3 Substituenten unabhängig voneinander ausgewählt aus der Gruppe Fluor, Chlor, Nitro, Cyano, (C₁-C₄)-Alkyl, Difluormethyl, Trifluormethyl, Hydroxy, Methoxy, Ethoxy, Amino, Hydroxycarbonyl und (C₁-C₄)-Alkoxycarbonyl substituiert sein können,
- R²: für Cyclohexyl, Tetrahydropyranyl, Piperidinyl, Piperazinyl, Morpholinyl, Phenyl, Pyrazolyl, Imidazolyl, Oxazolyl, Thiazolyl oder Pyridyl steht,
wobei Cyclohexyl mit einem Substituenten ausgewählt aus der Gruppe Hydroxy und (C₁-C₄)-Alkoxy substituiert sein kann,
worin (C₂-C₄)-Alkoxy mit 1 oder 2 Substituenten unabhängig voneinander ausgewählt aus der Gruppe Hydroxy und Methoxy substituiert sein kann,
und
wobei Piperidinyl, Piperazinyl und Morpholinyl mit einem Substituenten ausgewählt aus der Gruppe (C₁-C₄)-Alkyl, Hydroxy, (C₁-C₄)-Alkoxy und (C₁-C₄)-Alkylcarbonyl substituiert sein können,
worin (C₁-C₄)-Alkyl mit 1 oder 2 Substituenten unabhängig voneinander ausgewählt aus der Gruppe Hydroxy, Methoxy, Ethoxy, Methylcarbonyloxy und Ethylcarbonyloxy substituiert sein kann,
und
worin (C₁-C₄)-Alkylcarbonyl mit einem Substituenten ausgewählt aus der Gruppe Hydroxy, Methoxy und Ethoxy substituiert sein kann,
und
wobei Phenyl und Pyridyl mit 1 bis 3 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus der Gruppe Oxo, Hydroxy, (C₁₋C₄)-Alkoxy, Hydroxycarbonyl und Amino substituiert sein kann,
und
wobei Pyrazolyl, Imidazolyl, Oxazolyl und Thiazolyl mit 1 oder 2 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können, worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
- R³: für Wasserstoff, Amino, Methylamino oder Dimethylamino steht,
sowie ihre Salze, Solvate und Solvate der Salze.

Bevorzugt sind auch im Rahmen der vorliegenden Erfindung Verbindungen der Formel (I), in welcher
- A: für CR⁴ oder N steht,
wobei
R⁴ für (C₁-C₄)-Alkoxycarbonyl, Aminocarbonyl oder Mono-(C₁-C₄)-alkylaminocarbonyl steht,
und
- B: für NR⁵ steht, wobei
R⁵ für Wasserstoff, Methyl oder Ethyl steht,
worin Methyl und Ethyl mit einem Substituenten ausgewählt aus der Gruppe Methoxycarbonyl und Ethoxycarbonyl substituiert sein können,
- X: für O oder S steht,
- R¹: für Phenyl oder 5- oder 6-gliedriges Heteroaryl steht, wobei Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, Hydroxy, (C₁-C₄)-Alkoxy, Amino, Hydroxycarbonyl, (C₁-C₄)-Alkoxy-carbonyl, Aminocarbonyl, Phenyl und 5- oder 6-gliedriges Heteroaryl substituiert sein können,
worin Phenyl und 5- oder 6-gliedriges Heteroaryl mit 1 bis 3 Substituenten unabhängig voneinander ausgewählt aus der Gruppe Fluor, Chlor, Nitro, Cyano, (C₁-C₄)-Alkyl, Difluormethyl, Trifluormethyl, Hydroxy, Methoxy, Ethoxy, Amino, Hydroxycarbonyl und (C₁-C₄)-Alkoxycarbonyl substituiert sein können,
- R²: für eine Gruppe der Formel 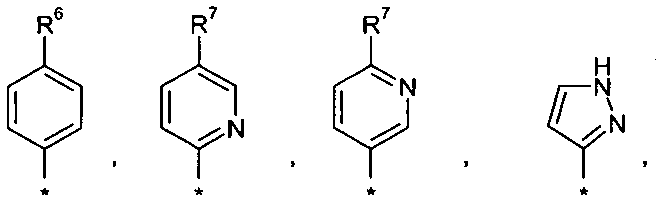 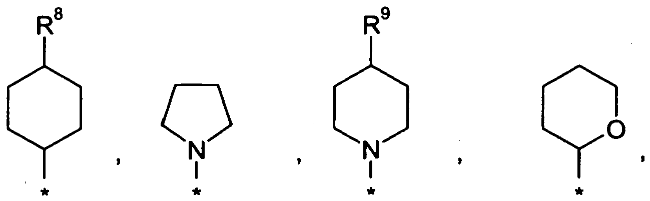 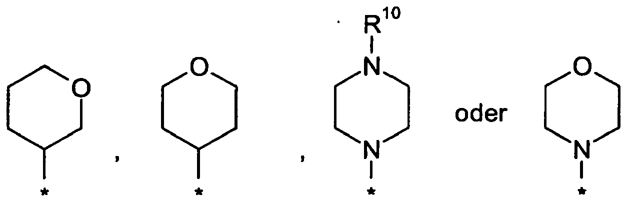 steht, wobei
* die Anknüpfstelle an den Bicyclus bedeutet,
R⁶ für Wasserstoff oder (C₁-C₄)-Alkoxy steht,
worin (C₂-C₄)-Alkoxy mit 1 oder 2 Substituenten Hydroxy substituiert sein kann,
R⁷ für Wasserstoff oder (C₁-C₄)-Alkoxy steht,
worin (C₂-C₄)-Alkoxy mit 1 oder 2 Substituenten Hydroxy substituiert sein kann,
R⁸ ür Wasserstoff, Hydroxy, Methoxy, Ethoxy oder 2-Hydroxyethoxy steht,
R⁹ für Wasserstoff oder Hydroxy steht,
und
R¹⁰ für Wasserstoff oder Methyl steht,
- R³: für Wasserstoff, Amino, Methylamino oder Dimethylamino steht,
sowie ihre Salze, Solvate und Solvate der Salze.

Besonders bevorzugt sind im Rahmen der vorliegenden Erfindung Verbindungen der Formel (I), in welcher
- A: für CR⁴ oder N steht,
wobei
R⁴ für Methoxycarbonyl, Aminocarbonyl oder Methylaminocarbonyl steht,
und
- B: für NR⁵ steht,
wobei
R⁵ für Wasserstoff oder Methyl steht,
worin Methyl mit einem Substituenten Methoxycarbonyl substituiert sein kann
- X: für O oder S steht
- R¹: für Thiazolyl, Oxazolyl, Phenyl oder Pyridyl steht,
wobei Phenyl und Pyridyl mit 1 oder 2 Substituenten unabhängig voneinander ausgewählt aus der Gruppe Fluor, Chlor, Cyano, Methyl, Ethyl, Methoxy, Amino, Hydroxycarbonyl, Methoxycarbonyl, Ethoxycarbonyl und Aminocarbonyl substituiert sein können,
und
wobei Thiazolyl und Oxazolyl mit einem Substituenten Gruppe Phenyl substituiert sind, worin Phenyl mit einem Substituenten ausgewählt aus der Gruppe Fluor, Chlor, Cyano, Methyl, Methoxy und Hydroxycarbonyl substituiert sein kann,
und
wobei Thiazolyl und Oxazolyl mit einem Substituenten ausgewählt aus der Gruppe Fluor, Chlor, Cyano, Methyl, Ethyl, Methoxy, Amino, Hydroxycarbonyl, Methoxycarbonyl, Ethoxycarbonyl und Aminocarbonyl substituiert sein können, für eine Gruppe der FormelR² 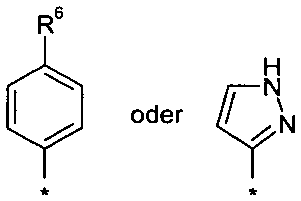 steht, wobei
* die Anknüpfstelle an den Bicyclus bedeutet,
R⁶ für Wasserstoff oder (C₁-C₄)-Alkoxy steht, worin (C₂-C₄)-Alkoxy mit 1 oder 2 Substituenten Hydroxy substituiert sein kann, R³ für Amino steht,
sowie ihre Salze, Solvate und Solvate der Salze.
- R³: für Amino steht,
sowie ihre Salze, Solvate und Solvate der Salze.

Bevorzugt sind im Rahmen der vorliegenden Erfindung auch Verbindungen der Formel (I), in welcher
- R²: für eine Verbindung der Formel 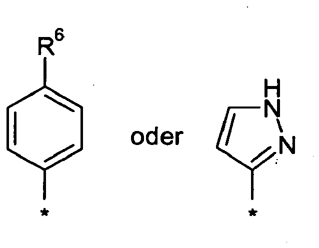 steht, wobei
* die Anknüpfstelle an den Bicyclus bedeutet,
R⁶ für Wasserstoff oder (C₁-C₄)-Alkoxy steht,
worin (C₂-C₄)-Alkoxy mit 1 oder 2 Substituenten Hydroxy substituiert sein kann.

Bevorzugt sind im Rahmen der vorliegenden Erfindung auch Verbindungen der Formel (I), in welcher
- R¹: für Thiazolyl, Oxazolyl, Phenyl oder Pyridyl steht,
wobei Phenyl und Pyridyl mit 1 oder 2 Substituenten unabhängig voneinander ausgewählt aus der Methoxycarbonyl, Gruppe Fluor, Chlor, Cyano, Methyl, Ethyl, Methoxy, Amino, Hydroxycarbonyl, Ethoxycarbonyl und Aminocarbonyl substituiert sein können,
und
wobei Thiazolyl und Oxazolyl mit Phenyl substituiert sind,
worin Phenyl mit einem Substituenten ausgewählt aus der Gruppe Fluor, Chlor, Cyano, Methyl, Methoxy und Hydroxycarbonyl substituiert sein kann,
und/ oder
wobei Thiazolyl und Oxazolyl mit einem Substituenten ausgewählt aus der Gruppe Fluor, Chlor, Cyano, Methyl, Ethyl, Methoxy, Amino, Hydroxycarbonyl, Methoxycarbonyl, Ethoxycarbonyl und Aminocarbonyl substituiert sein können.

Bevorzugt sind im Rahmen der vorliegenden Erfindung auch Verbindungen der Formel (I), in welcher R³ für Amino steht.

Weiterer Gegenstand der vorliegenden Erfindung ist ein Verfahren zur Herstellung der erfindungsgemäßen Verbindungen der Formel (I), in welcher R³ für Amino steht, dadurch gekennzeichnet, dass man eine Verbindung der Formel (II) 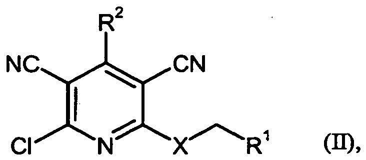 in welcher X, R¹ und R² jeweils die oben angegebenen Bedeutungen haben,
[A] in einem inerten Lösungsmittel in Gegenwart einer geeigneten Base mit einer Verbindung der Formel (III-A) 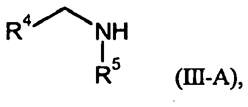 in welcher R⁴ und R⁵ jeweils die oben angegebenen Bedeutungen haben,
   zu einer Verbindung der Formel (IV-A) 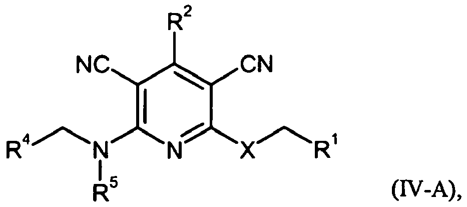 in welcher X, R¹, R², R⁴ und R⁵ jeweils die oben angegebenen Bedeutungen haben,
   umsetzt, diese anschliessend in einem inerten Lösungsmittel und in Gegenwart einer geeigneten Base zu Verbindungen der Formel (I-A) 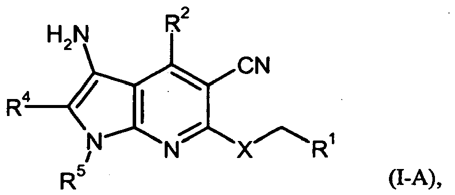 in welcher X, R¹, R², R⁴ und R⁵ jeweils die oben angegebenen Bedeutungen haben,
   cyclisiert,
   oder
[B] in einem inerten Lösungsmittel in Gegenwart einer geeigneten Base mit einer Verbindung der Formel (III-B) 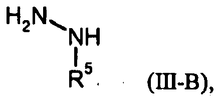 in welcher R⁵ die oben angegebene Bedeutung hat,
   zu Verbindungen der Formel (I-B) 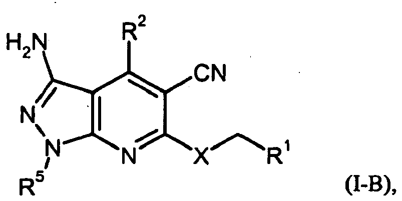 in welcher X, R¹, R² und R⁵ jeweils die oben angegebenen Bedeutungen haben,
   cyclisiert,
anschließend gegebenenfalls vorhandene Schutzgruppen abspaltet und die resultierenden Verbindungen der Formeln (I-A), (I-B) gegebenenfalls mit den entsprechenden (*i*) Lösungsmitteln und/oder (*ii*) Basen oder Säuren in ihre Solvate, Salze und/oder Solvate der Salze überführt.

In den Verbindungen der Formel (II) bzw. im Rest R² gegebenenfalls vorhandene funktionelle Gruppen - wie insbesondere Amino-, Hydroxy- und Carboxylgruppen - können bei diesem Verfahren, falls zweckmäßig oder erforderlich, auch in temporär geschützter Form vorliegen. Die Einführung und Entfernung solcher Schutzgruppen erfolgt hierbei nach üblichen, dem Fachmann bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Bei Vorhandensein mehrerer Schutzgruppen kann die Entfernung gegebenenfalls simultan in einer Eintopf-Reaktion oder in separaten Reaktionsschritten vorgenommen werden.

Die Verbindungen der Formel (III-A), (III-B) sind kommerziell erhältlich, literaturbekannt oder können in Analogie zu literaturbekannten Verfahren hergestellt werden.

Das zuvor beschriebene Verfahren kann durch die folgenden Reaktionsschemata 1 bis 2 erläutert werden:

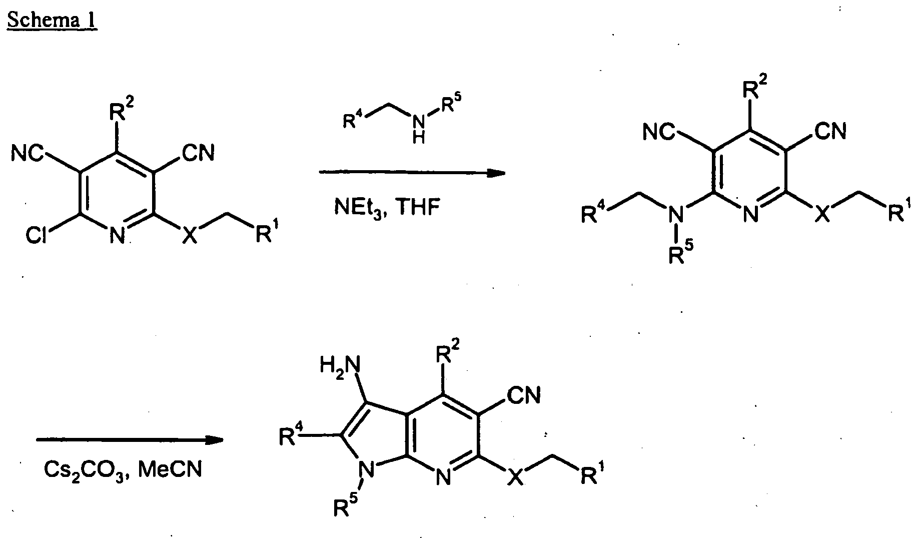

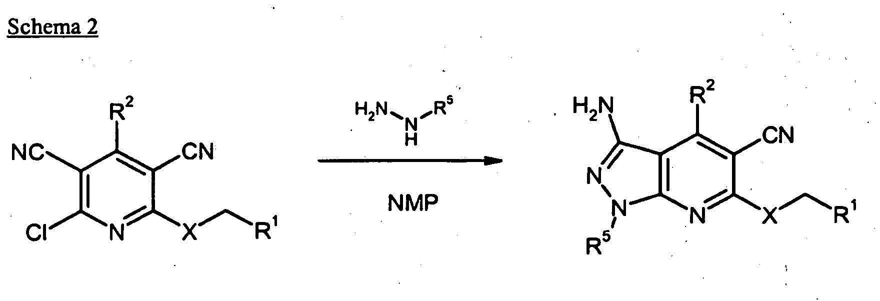

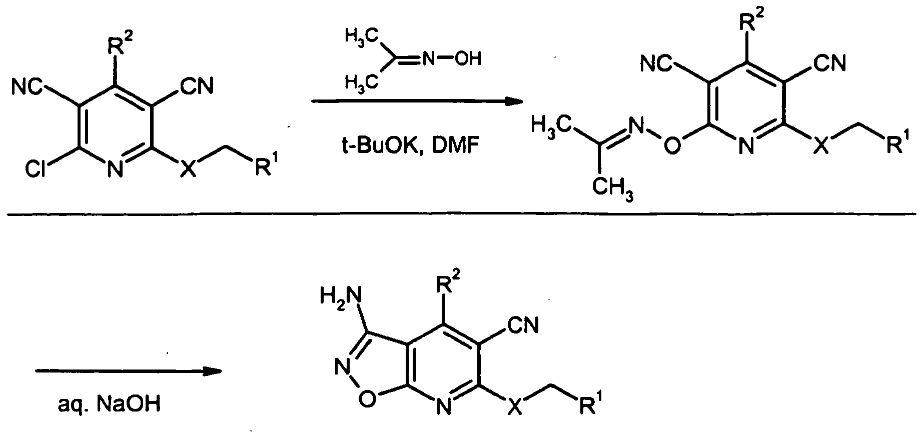

Inerte Lösungsmittel für die Umsetzungen (II) + (III-A) **→** (IV-A), (IV-A) → (I-A), (II) + (III-B) → (I-B), sind beispielsweise Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), *N*-Methylpyrrolidinon (NMP), Acetonitril, Pyridin oder Wasser. Ebenso ist es möglich, Gemische der zuvor genannten Lösungsmittel einzusetzen. Bevorzugt werden Dimethylformamid, Acetonitril, Tetrahydrofuran und Dioxan als Lösungsmittel verwendet.

Als Base für die Umsetzung (II) + (III-A) → (IV-A) → (I-A) eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat oder Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Triethylamin und Alkalicarbonate.

Die Base kann hierbei jeweils in einer Menge von 1 bis 10 Mol, bevorzugt von 1 bis 5 Mol, insbesondere von 1 bis 4 Mol, bezogen auf 1 Mol der Verbindung der Formel (II), eingesetzt werden.

Die Reaktionen erfolgen im Allgemeinen in einem Temperaturbereich von -78°C bis +140°C, bevorzugt im Bereich von -20°C bis +80°C, insbesondere bei 0°C bis +50°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Weitere erfindungsgemäße Verbindungen können ausgehend von den nach obigen Verfahren erhaltenen Verbindungen der Formel (I), worin R³ für Amino steht, hergestellt werden, indem man diese in Analogie zu dem in Ortega, M.A. et al., Bioorg. Med. Chem. 2002, 10 (7), 2177-2184 beschriebenen Verfahren in Verbindungen der Formel (V), 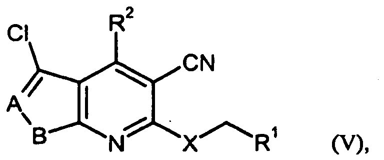 in welcher A, B, X, R¹ und R² jeweils die oben angegebenen Bedeutungen haben,
überführt und diese Verbindungen anschliessend in Analogie zu literaturbekannten Verfahren weiter umsetzt [vgl. Fischer E. et al., Chem. Ber. 1901, 34, 798; Zhu, G. et al., Bioorg. Med. Chem. 2007, 15 (6), 2441-2452; Vasudevan A. et al., Bioorg. Med. Chem. Lett. 2005, 15 (23), 5293-5297; Pillai P. et al., Indian J. Chem. Sect. B, 1989, 28, 1026-1030].

Weitere erfindungsgemäße Verbindungen können gegebenenfalls auch hergestellt werden durch Umwandlungen von funktionellen Gruppen einzelner Substituenten, insbesondere den unter R², R³ R⁴ und R⁵ aufgeführten, ausgehend von den nach obigen Verfahren erhaltenen Verbindungen der Formel (I). Diese Umwandlungen werden nach üblichen, dem Fachmann bekannten Methoden bekannten Methoden durchgeführt und umfassen beispielsweise Reaktionen wie nukleophile und elektrophile Substitutionen, Oxidationen, Reduktionen, Hydrierungen, Übergangsmetallkatalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die Verbindungen der Formel (II) können hergestellt werden, indem man eine Verbindung der Formel (VI) 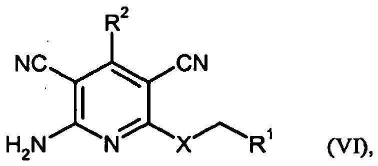 in welcher X, R¹ und R² jeweils die oden angegebenen Bedeutungen haben,
in einem inerten Lösungsmittel mit Kupfer(II)chlorid und Isopentylnitrit umsetzt.

Das zuvor beschriebene Verfahren kann durch das folgende Reaktionsschema erläutert werden:

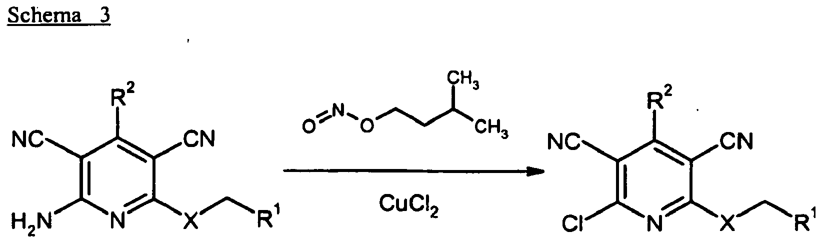

Die Umsetzung (VI) → (II) erfolgt im Allgemeinen in einem Molverhältnis von 2 bis 12 Mol Kupfer(II)chlorid und 2 bis 12 Mol Isopentylnitrit bezogen auf 1 Mol der Verbindung der Formel (VI).

Als Lösungsmittel für den Verfahrensschritt (VI) → (II) eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylsulfoxid (DMSO), Dimethylformamid, Acetonitril oder Pyridin. Ebenso ist es möglich, Gemische der zuvor genannten Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C, bevorzugt im Bereich von 0°C bis +100°C, insbesondere bei +20°C bis +80°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Verbindungen der Formel (VI), in welcher X für S steht, können hergestellt werden, indem man eine Verbindung der Formel (VII-A) 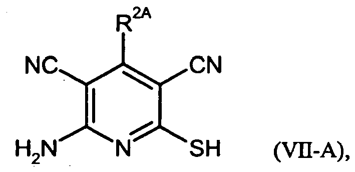 in welcher
- R^{2A}: für (C₅-C₆)-Cycloalkyl, C-gebundenes 5- oder 6-gliedriges Heterocyclyl, Phenyl oder C-gebundenes 5- oder 6-gliediges Heteroaryl steht, welche jeweils wie oben unter R² beschrieben substituiert sein können,
in einem inerten Lösungsmittel in Gegenwart einer Base mit einer Verbindung der Formel (VIII) 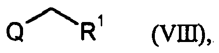 in welcher R¹ die oben angegebene Bedeutung hat, und
- Q: für eine geeignete Abgangsgruppe, vorzugsweise für Halogen, insbesondere Chlor, Brom oder Iod, oder für Mesylat, Tosylat oder Triflat steht,
zu Verbindungen der Formel (VI-A) 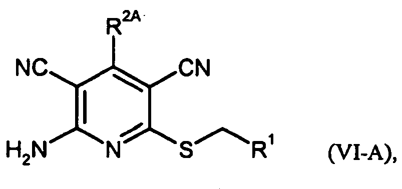 in welcher R¹ und R^{2A} jeweils die oben angegebenen Bedeutungen haben,
umsetzt.

Die Verbindungen der Formel (VIII) sind kommerziell erhältlich, literaturbekannt oder nach literaturbekannten Methoden herstellbar. So können beispielsweise durch Reaktion von Amiden bzw. Thioamiden mit einem 1,3-Dihalogenaceton substituierte Oxazol- und Thiazol-Derivate der Formel (VIII-A) und (VIII-B) erhalten werden (siehe Schema 4): 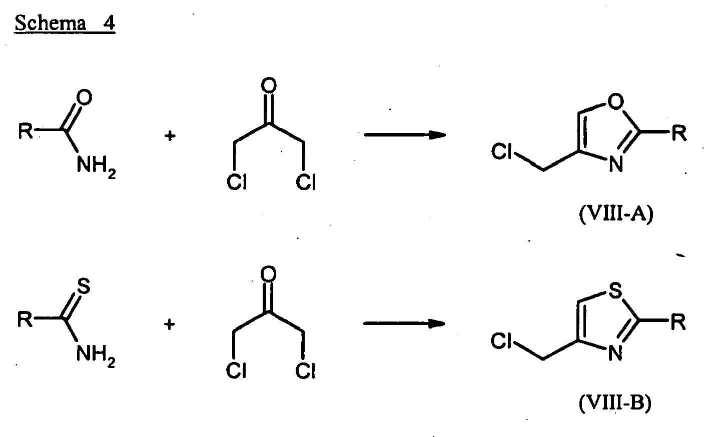 Inerte Lösungsmittel für die Umsetzung (VII-A) + (VIII) → (VI-A) sind beispielsweise Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), *N*-Methylpyrrolidinon (NMP), Acetonitril oder Pyridine Wasser ist als lösungsmittel ebenfalls geeignet. Ebenso ist es möglich Gemische der zuvor genannten Lösungsmittel einzusetzen. Bevorzugt wird Dimethylformamid als Lösungsmittel verwendet.

Als Base für die Umsetzung (VII-A) + (VIII) → (VI-A) eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Alkalicarbonate und Alkalihydrogencarbonate.

Die Base kann hierbei in einer Menge von 1 bis 10 Mol, bevorzugt von 1 bis 5 Mol, insbesondere von 1 bis 4 Mol, bezogen auf 1 Mol der Verbindung der Formel (II), eingesetzt werden.

Die Reaktion erfolgt im Allgemeinen in einem Temperaturbereich von -78°C bis +140°C, bevorzugt im Bereich von -20°C bis +80°C, insbesondere bei 0°C bis +50°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Verbindungen der Formel (VII-A), können in Analogie zu literaturbekannten Methoden beispielsweise dadurch hergestellt werden, dass man Aldehyde der Formel (IX) 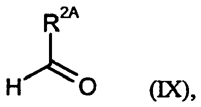 in welcher R^{2A} die oben angegebene Bedeutung hat,
in Gegenwart einer Base mit zwei Äquivalenten Cyanothioacetamid umsetzt [vgl. z.B. Dyachenko et al., Russ. J. Chem. 33 (7), 1014-1017 (1997), 34 (4), 557-563 (1998); Dyachenko et al., Chemistry of Heterocyclic Compounds 34 (2), 188-194 (1998); Qintela et al., Eur. J. Med. Chem. 33, 887-897 (1998); Kandeel et al., Z. Naturforsch. 42b, 107-111 (1987); Reddy et al., J. Med. Chem. 49, 607-615 (2006); Evdokimov et al., Org. Lett. 8, 899-902 (2006)].

Die Verbindungen der Formel (IX) sind kommerziell erhältlich, literaturbekannt oder können in Analogie zu literaturbekannten Verfahren hergestellt werden.

Weitere Verbindungen der Formel (VI), in welcher X für S steht, können hergestellt werden, indem man die Verbindung der Formel (X) 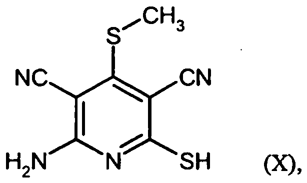 in einem inerten Lösungsmittel in Gegenwart einer Base mit einer Verbindung der Formel (VIII) in eine Verbindung der Formel (XI) 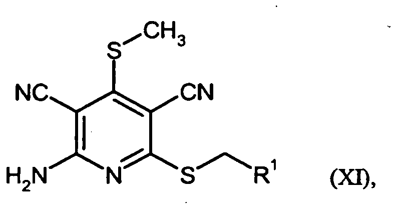 in welcher R¹ die oben angegebene Bedeutung hat,
überführt und diese dann in einem inerten Lösungsmittel oder ohne Lösungsmittel mit einer Verbindung der Formel (XII)

R^{2B}-H (XII),

in welcher
- R^{2B}: für N-gebundenes 5- oder 6-gliedriges Heterocyclyl oder N-gebundenes 5- oder 6-gliedriges Heteroaryl steht,
welche jeweils wie oben für R² beschrieben substituiert sein können,
zu Verbindungen der Formel (VI-B) 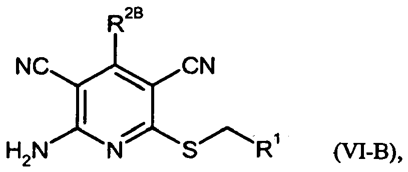 in welcher R¹ und R^{2B} jeweils die oben angegebenen Bedeutungen haben,
umsetzt.

Als Lösungsmittel für den Verfahrensschritt (X) + (VIII) → (XI) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), N-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Bevorzugt wird Dimethylformamid als Lösungsmittel verwendet.

Als Base für den Verfahrensschritt (X) + (VIII) → (XI) eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Alkalicarbonate und -hydrogencarbonate.

Die Base kann hierbei in einer Menge von 1 bis 10 Mol, bevorzugt von 1 bis 5 Mol, insbesondere von 1 bis 4 Mol, bezogen auf 1 Mol der Verbindung der Formel (II), eingesetzt werden.

Die Reaktion erfolgt im Allgemeinen in einem Temperaturbereich von -78°C bis +140°C, bevorzugt im Bereich von -20°C bis +80°C, insbesondere bei 0°C bis +50°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Lösungsmittel für den Verfahrensschritt (XI) + (XII) → (VI-B) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan oder Chlorbenzol, oder andere Lösungsmittel wie Dimethylformamid (DMF), Dimethylsulfoxid (DMSO), *N*-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Gegebenenfalls kann die Reaktion auch vorteilhaft in Gegenwart eines Überschusses der Verbindung (XII) ohne Zusatz eines weiteren Lösungsmittels durchgeführt werden. Bevorzugt wird die Umsetzung in Aceton oder *N*-Methylpyrrolidinon als Lösungsmittel durchgeführt.

Der Verfahrensschritt (XI) + (XII) → (VI-B) erfolgt im Allgemeinen in einem Temperaturbereich von 0°C bis +180°C, bevorzugt im Bereich von +20°C bis +100°C, insbesondere bei +60°C bis +100°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (XII) sind kommerziell erhältlich, literaturbekannt oder können in Analogie zu literaturbekannten Verfahren hergestellt werden.

Die Verbindung der Formel (X) lässt sich auf einfache Weise durch Umsetzung von [Bis(methyl-thio)methylen]malononitril mit Cyanothioacetamid in Gegenwart einer Base wie Triethylamin erhalten.

Verbindungen der Formel (VI), in welcher X für O steht, können hergestellt werden, indem man eine Verbindung der Formel (XIII) 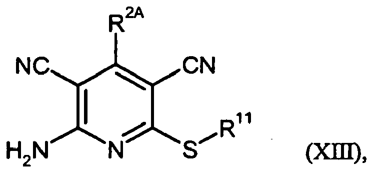 in welcher R^{2A} die oben angegebene Bedeutung hat,
und
- R¹¹: für (C₁-C₄)-Alkyl oder Phenyl steht,
in einem inerten Lösungsmittel in Gegenwart einer Base mit einer Verbindung der Formel (XIV) 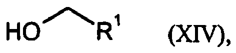 in welcher R¹ die oben angegebene Bedeutung hat, und
zu Verbindungen der Formel (VI-C) 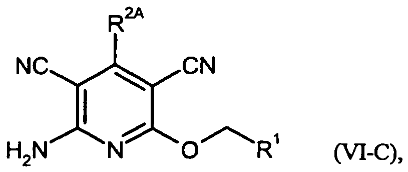 in welcher R¹ und R^{2A} jeweils die oben angegebenen Bedeutungen haben,
umsetzt.

Die Verbindungen der Formel (XIII) können in Analogie zu literaturbeschriebenen Verfahren hergestellt werden [vgl. z.B. Kambe et al., Synthesis, 531-533 (1981); Elnagdi et al., Z. Naturforsch. 47b, 572-578 (1991); Reddy et al., J. Med. Chem. 49, 607-615 (2006); Evdokimov et al., Org. Lett. 8, 899-902 (2006)] oder durch Umsetzung von Verbindungen der Formel (VII-A) Analogie zu literaturbeschriebenen Verfahren [vgl. z. B. Fujiwara, H. et al., Heterocycles 1993, 36 (5),1105-1113, Su et al., J. Med Chem. 1988, 31, 1209-1215].

Weitere Verbindungen der Formel (VI), in welcher X für O steht, können hergestellt werden, indem man die Verbindung der Formel (XV) 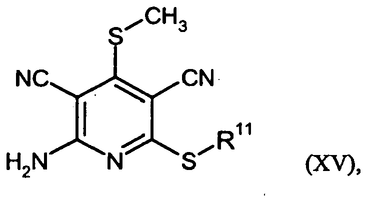 in welcher R¹¹ die oben angegebene Bedeutung hat,
in einem inerten Lösungsmittel in Gegenwart einer Base mit einer Verbindung der Formel (XIV) in eine Verbindung der Formel (XVI) 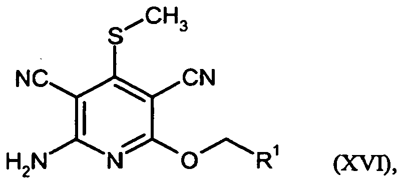 in welcher R¹ die oben angegebene Bedeutung hat,
überführt und diese dann in einem inerten Lösungsmittel oder ohne Lösungsmittel mit einer Verbindung der Formel (XII) zu Verbindungen der Formel (VI-D) 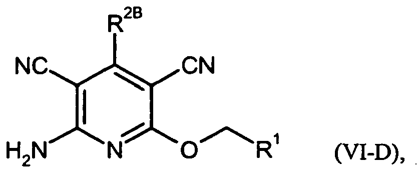 in welcher R¹ und R^{2B} jeweils die oben angegebenen Bedeutungen haben,
umsetzt, oder
alternativ eine Verbindung der Formel (XV) zuerst in einem inerten Lösungsmittel oder ohne Lösungsmittel mit einer Verbindung der Formel (XII) zu Verbindungen der Formel (XVII) 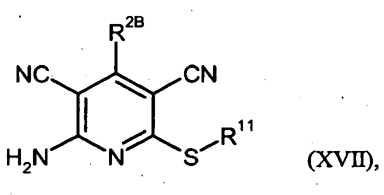 in welcher R^{2B} und R¹¹ jeweils die oben angegebenen Bedeutungen haben,
umsetzt, und diese anschliessend mit einer Verbindung der Formel (XIV) in Verbindungen der Formel (VI-D) überfährt.

Die Verbindungen der -Formel (XV), in welcher R¹¹ für Phenyl steht, lassen sich aus der Verbindung der Formel (X) in Analogie zu dem in Fujiwara, H. et al., Heterocycles 1993, 36 (5), 1105-1113 beschriebenen Verfahren herstellern.

Die Verbindungen der Formel (XV), in welcher R¹¹ für (C₁-C₄)-Alkyl steht, lassen sich aus der Verbindung der Formel (X) in Analogie zu dem in Su et al., J. Med Chem. 1988, 31, 1209-1215 beschriebenen Verfahren herstellen.

Als inerte Lösungsmittel für die Reaktionen (XIII) + (XIV), (XV) + (XIV) und (XVII) + (XIV) eignen sich insbesondere acyclische und cyclische Ether wie Diethylether, Methyl-tert.-butylether, 1,2-Dimethoxyethan, Tetrahydrofuran und Dioxan, Kohlenwasserstoffe wie Benzol, Toluol, Xylol, Hexan und Cyclohexan, oder andere Lösungsmittel wie Dimethylformamid (DMF), Dimethylsulfoxid (DMSO), *N*-Methylpyrrolidinon (NMP) und Pyridin. Ebenso ist es möglich, Gemische dieser Lösungsmittel einzusetzen. Bevorzugt wird 1,2-Dimethoxyethan verwendet.

Als Basen für diese Umsetzungen sind insbesondere Alkalialkoholate wie Natrium- oder Kaliummethanolat, Natrium- oder Kaliumethanolat oder Natrium- oder Kalium-tert.-butylat, Alkalihydride wie Lithium-, Natrium- oder Kaliumhydrid, Amide wie Natriumamid, Lithium-, Natrium- oder Kalium-bis-(trimethylsilyl)amid oder Lithiumdiisopropylamid, oder metallorganische Verbindungen wie Butyllithium oder Phenyllithium geeignet. Bevorzugt wird Kalium-tert.-butylat verwendet.

Die Base wird hierbei in der Regel in einer Menge von 1 bis 1.25 Mol, bevorzugt in äquimolarer Menge, bezogen auf 1 Mol der Verbindung der Formel (XIV), eingesetzt.

Die Reaktionen (XIII) + (XIV), (XV) + (XIV) und (XVII) + (XIV) erfolgen im Allgemeinen in einem Temperaturbereich von -20°C bis +120°C, bevorzugt bei +20°C bis +100°C, gegebenenfalls in der Mikrowelle. Die Umsetzungen können bei normalem, erhöhtem oder erniedrigtem Druck durchgeführt werden (z.B. im Bereich von 0.5 bis 5 bar). Im Allgemeinen arbeitet man bei Normaldruck.

Als Lösungsmittel für die Verfahrensschritte (XV) bzw. (XVI) + (XII) → (VI-D) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan oder Chlorbenzol, oder andere Lösungsmittel wie Dimethylformamid (DMF), Dimethylsulfoxid (DMSO), N-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Gegebenenfalls kann die Reaktion auch vorteilhaft in Gegenwart eines Überschusses der Verbindung (XII) ohne Zusatz eines weiteren Lösungsmittels durchgeführt werden. Bevorzugt wird die Umsetzung in Aceton oder N-Methylpyrrolidinon als Lösungsmittel durchgeführt.

Die Verfahrensschritte (XV) bzw. (XVI) + (XII) → (VI-D) erfolgen im Allgemeinen in einem Temperaturbereich von 0°C bis +180°C, bevorzugt im Bereich von +20°C bis +100°C, insbesondere bei +60°C bis +100°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die zuvor beschriebenen Herstellungsverfahren können durch die folgenden Reaktionsschemata beispielhaft erläutert werden:

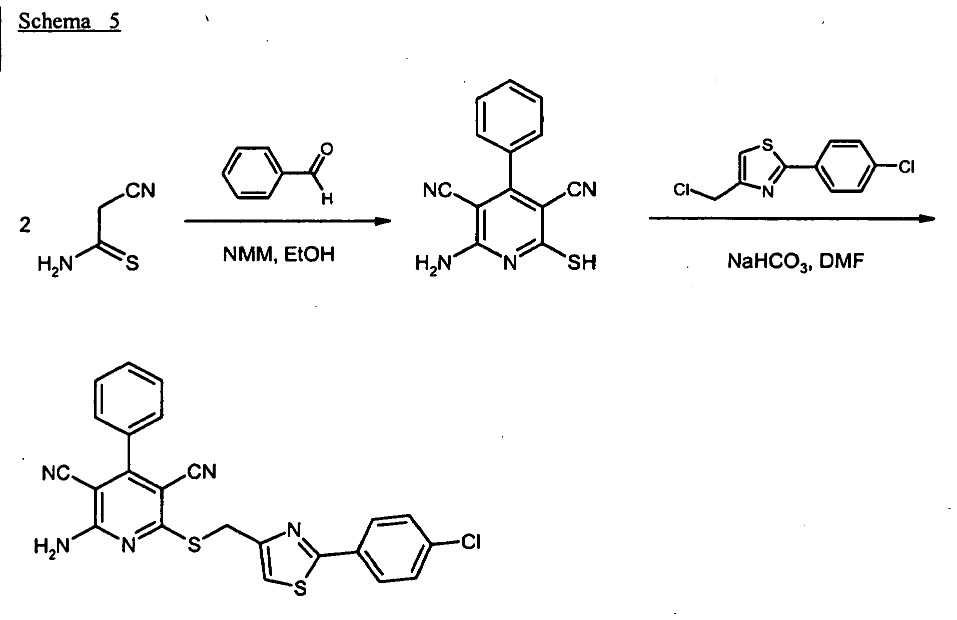

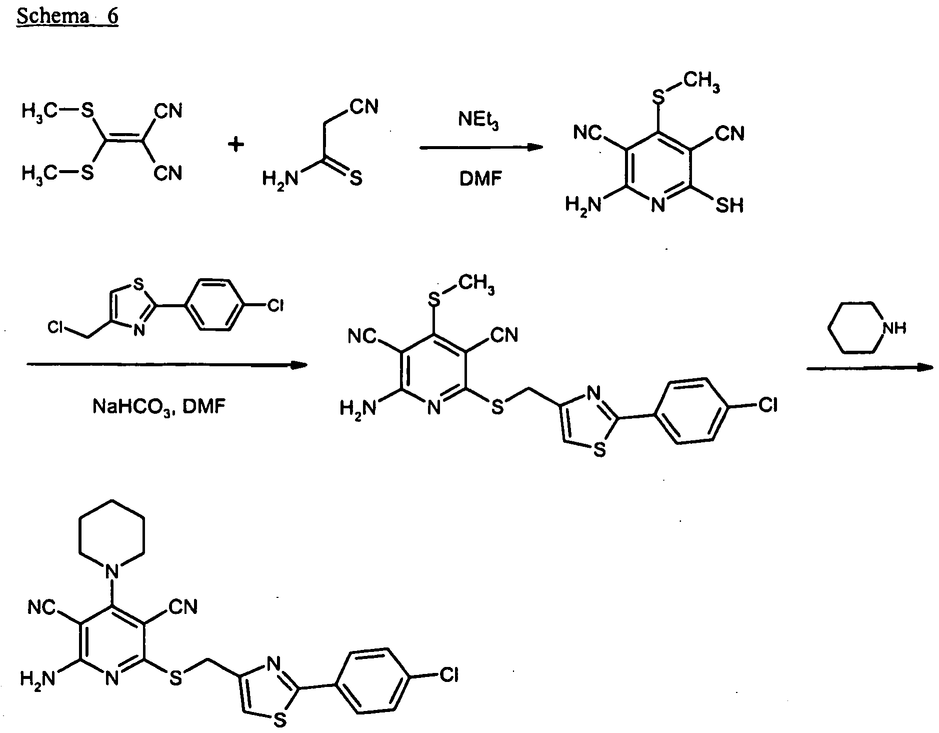

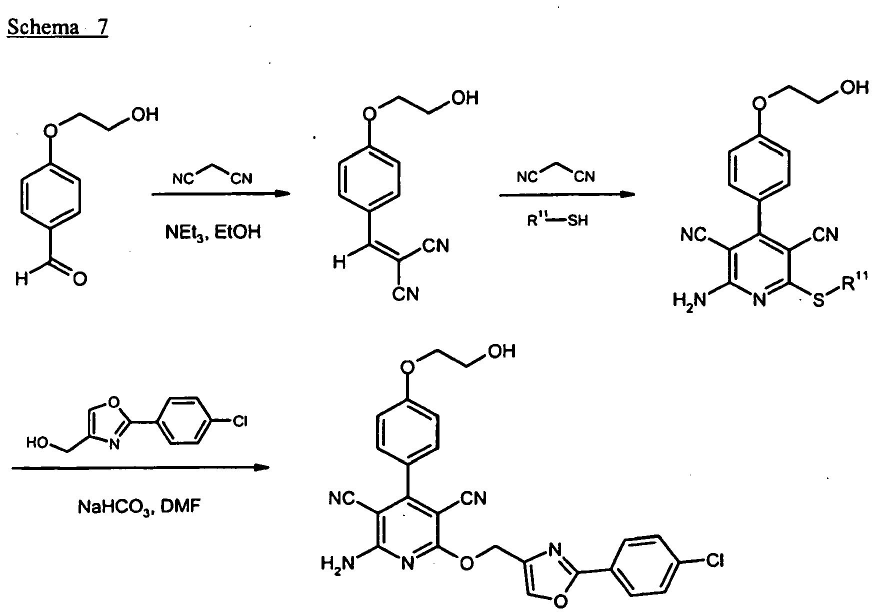

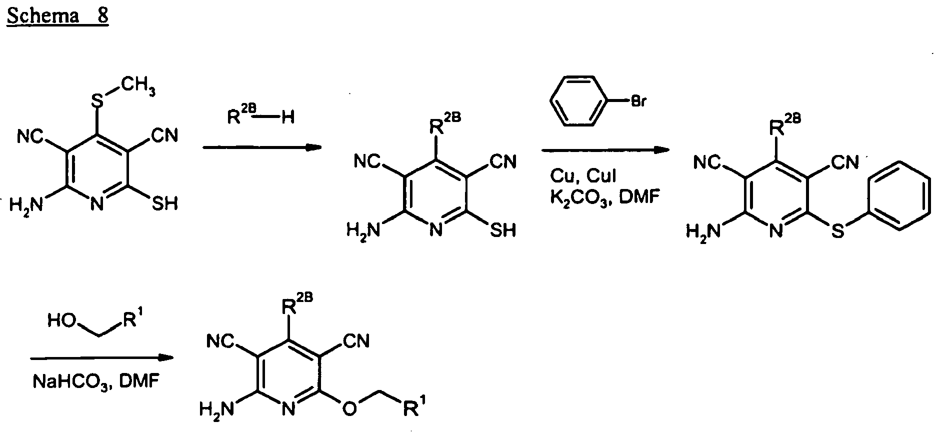

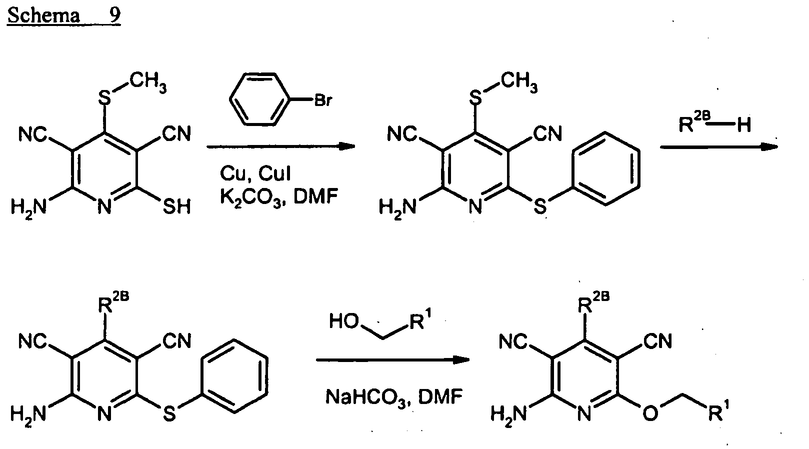

Überraschenderweise zeigen die erfindungsgemäßen Verbindungen ein nicht vorhersehbares, wertvolles pharmakologisches Wirkspektrum und sind daher insbesondere zur Prävention und/oder Behandlung von Erkrankungen geeignet.

Die pharmazeutische Wirksamkeit der erfindungsgemäßen Verbindungen lässt sich durch ihre Wirkung als potente, selektive Liganden an einzelnen oder mehreren Subtypen der Adenosin-Rezeptoren, insbesondere als selektive Liganden an Adenosin A1- und/oder A2b-Rezeptoren erklären. Sie wirken hierbei als selektive A1-Agonisten, selektive Al-Antagonisten oder als selektive duale A1/A2b-Agonisten.

Die erfindungsgemäßen Verbindungen wirken vorwiegend als selektive Adenosin A1-Agonisten.

Als "selektive Liganden an Adenosin A1- und/oder A2b-Rezeptoren" werden im Rahmen der vorliegenden Erfindung solche Adenosin-Rezeptorliganden bezeichnet, bei denen einerseits eine deutliche Wirkung an A1- und/oder A2b-Adenosinrezeptor-Subtypen und andererseits keine oder eine deutliche schwächere Wirkung (Faktor 10 oder höher) an A2a- und A3-Adenosinrezeptor-Subtypen zu beobachten ist, wobei bezüglich der Testmethoden für die Wirk-Selektivität Bezug genommen wird auf die im Abschnitt B-1. beschriebenen Tests.

Die erfindungsgemäßen Verbindungen können in Abhängigkeit von ihrer jeweiligen Struktur als volle, als partielle Adenosinrezeptor-Agonisten oder als Adenosinrezeptor-Antagonisten fungieren. Partielle Adenosinrezeptor-Agonisten sind hierbei definiert als Rezeptorliganden, die eine funktionelle Antwort an Adenosinrezeptoren auslösen, welche geringer ist als bei vollen Agonisten (wie beispielsweise Adenosin selbst). Partielle Agonisten weisen infolgedessen eine geringere Wirksamkeit bezüglich der Rezeptoraktivierung auf als volle Agonisten.

Die Verbindungen der Formel (I) sind allein oder in Kombination mit einem oder mehreren anderen Wirkstoffen zur Prävention und/oder Behandlung verschiedener Erkrankungen geeignet, so beispielsweise insbesondere von Hypertonie und anderen Erkrankungen des Herzkreislauf-Systems (kardiovaskulären Erkrankungen), zur Kardioprotektion nach Schädigungen des Herzens sowie von Stoffwechsel- und Nierenerkrankungen.

Im Sinne der vorliegenden Erfindung sind unter Erkrankungen des Herzkreislauf-Systems bzw. kardiovaskulären Erkrankungen neben der Hypertonie beispielsweise die folgenden Erkrankungen zu verstehen: periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, stabile und instabile Angina pectoris, Herzinsuffizienz, Tachykardien, Arrhythmien, Vorhof- und Kammerflimmern, periphere Durchblutungsstörungen, erhöhte Spiegel von Fibrinogen und von LDL geringer Dichte, sowie erhöhte Konzentrationen von Plasminogenaktivator-Inhibitor 1 (PAI-1), insbesondere koronare Herzerkrankung, akutes Koronarsyndrom, Angina pectoris, Herzinsuffizienz, Myokardinfarkt, Vorhofflimmern und Hypertonie

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische sowie systolische Herzinsuffizienz.

Weiterhin eignen sich die erfindungsgemäßen Verbindungen auch zur Reduktion des von einem Infarkt betroffenen Myokardbereichs sowie zur Prävention von Sekundärinfarkten.

Des weiteren sind die erfindungsgemäßen Verbindungen zur Prävention und/oder Behandlung von thromboembolischen Erkrankungen, Reperfusionsschäden nach Ischämie, mikro- und makrovaskulären Schädigungen (Vaskulitis), Ödemen, Ischämien wie Myokardinfarkt, Hirnschlag und transitorischen ischämischen Attacken, sowie zur Organprotektion bei Transplantationen, Bypass Operationen, Herzkatheteruntersuchungen und anderen operativen Eingriffen geeignet.

Weiterhin eignen sich die erfindungsgemäßen Verbindungen zur Behandlung und/oder Prävention von Nierenerkrankungen, insbesondere von Niereninsuffizienz. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, obstruktive Uropathie, Glomerulonephritis, akute Glomerulonephritis, tubulointerstitielle Erkrankungen, nephropathische Erkrankungen wie primäre und angeborene Nierenerkrankung, Nierenentzündung, durch toxische Substanzen induzierte Nephropathie, diabetische Nephropathie, Pyelonephritis, Nierenzysten und Nephrosklerose, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weitere Indikationsgebiete, für die die erfindungsgemäßen Verbindungen eingesetzt werden können, sind beispielsweise die Prävention und/oder Behandlung von Erkrankungen des Urogenitalbereiches, wie z.B. Reizblase, erektile Dysfunktion und weibliche sexuelle Dysfunktion, daneben aber auch die Prävention und/oder Behandlung von inflammatorischen Erkrankungen, wie z.B. entzündliche Dermatosen (Psoriasis, Akne, Ekzeme, Neurodermitis, Dermatitis, Keratitis, Narbenbildung, Warzenbildung, Frostbeulen), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), von Schmerzzuständen, Krebserkrankungen (Hautkrebs, Liposarcome, Karzinome des Magen-Darm-Traktes, der Leber, Bauchspeicheldrüse, Lunge, Niere, Harnleiter, Prostata und des Genitaltraktes) sowie von Übelkeit und Erbrechen in Verbindung mit Krebstherapien.

Weitere Indikationsgebiete sind beispielsweise die Prävention und/oder Behandlung von Entzündungs- und Immunerkrankungen (Morbus Crohn, Colitis ulcerosa, Lupus erythematodes, rheumatoide Arthritis) und von Erkrankungen der Atemwege, wie beispielsweise chronischobstruktive Atemwegserkrankungen (chronische Bronchitis, COPD), Asthma, Lungenemphysem, Bronchiektasien, zystische Fibrose (Mukoviszidose) und pulmonale Hypertonie, insbesondere pulmonale arterielle Hypertonie.

Schließlich kommen die erfindungsgemäßen Verbindungen auch für die Prävention und/ oder Behandlung von Diabetes, insbesondere Diabetes mellitus, Gestationsdiabetes, Insulin-abhängiger Diabetes und Nicht-Insulin-abhängiger Diabetes, von diabetischen Folgeerkrankungen wie z.B. Retinopathie, Nephropathie und Neuropathie, von metabolischen Erkrankungen (Metabolisches Syndrom, Hyperglykämie, Hyperinsulinämie, Insulinresistenz, Glukose-Intoleranz, Fettsucht (Adipositas)) sowie von Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien, in Betracht

Darüber hinaus können die erfindungsgemäßen Verbindungen auch zur Behandlung und/oder Prävention von Schilddrüsenerkrankungen (Hyperthyreose), Erkrankungen der Bauchspeicheldrüse (Pankreatitis), Leberfibrose, viralen Erkrankungen (HPV, HCMV, HIV), Kachexie, Osteoporose, Gicht, Inkontinenz sowie zur Wundheilung und Angiogenese eingesetzt werden.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

Weiterer Gegenstand der vorliegenden Erfindung sind die erfindungsgemäßen Verbindungen zur Verfahren zur Behandlung und/oder Prophylaxe von Diabetes, Metabolischem Syndrom und Dyslipidämien.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/ oder antithrombotisch wirkende Mittel,

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylharnstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/ oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Rezeptoren-Blocker, alpha-Rezeptoren-Blocker, Aldosteron-Antagonisten, Mineralocorticoid-Rezeptor-Antagonisten, ECE-Inhibitoren, ACE/NEP Inhibitoren sowie der Vasopeptidase-Inhibitoren; und/oder
- antithrombotisch wirkenden Mitteln, beispielhaft und vorzugsweise aus der Gruppe der Thrombozytenaggregationshemmer oder der Antikoagulantien
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Torcetrapib, JTT-705, BAY 60-5521, BAY 78-7499 oder CETP vaccine (Avant), verabreicht.

Bei einer bevorzugten Ausführungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren und PPAR-gamma-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin und Vildagliptin, verabreicht.

Bei einer bevorzugten Ausführungsform der Erfindung werden die erfindungsgemäßen Verbindungen in Kombination mit einem PPAR-gamma-Agonisten beispielsweise aus der Klasse der Thiazolidindione, wie beispielhaft und vorzugsweise Pioglitazone oder Rosiglitazone, verabreicht.

Unter den Blutdruck senkenden Mitteln werden vorzugsweise Verbindungen aus der Gruppe der Calcium-Antagonisten, Angiotensin AII-Antagonisten, ACE-Hemmer, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralokortikoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Nitroglycerin, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, mit Kaliumkanal-Agonisten wie Minoxidil, Diazoxid, Dihydralazin oder Hydralazin, oder mit Stickoxid freisetzenden Stoffen wie Glycerinnitrat oder Nitroprussidnatrium verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Rezeptoren-Blocker, organische Nitrate und NO-Donatoren, ACE-Inhibitoren, Angiotensin AII-Antagonisten, Aldosteron- und Mineralokortikoid-Rezeptor-Antagonisten, Vasopressin-Rezeptor-Antagonisten, Thrombozytenaggregationshemmer und Antikoagulantien, sowie deren Verwendung zur Behandlung und/oder Prävention der zuvor genannten Erkrankungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- aq.: wässrig
- br s: breites Singulett (bei NMR)
- Bsp.: Beispiel
- c: Konzentration
- d: Dublett (bei NMR)
- dd: Dublett von Dublett (bei NMR)
- DC: Dünnschichtchromatographie
- DCI: direkte chemische Ionisation (bei MS)
- DMF: *N,-N*-Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ent: Enantiomer / enantiomerenrein
- ESI: Elektrospray-Ionisation (bei MS)
- Et: Ethyl
- Fp.: Schmelzpunkt
- GC-MS: Gaschromatographie-gekoppelte Massenspektrometrie
- h: Stunde(n)
- HATU: O-(7-Azabenzotriazol-1-yl)-*N*,*N*,*N'*,*N'*-tetramethyluronium-Hexafluorphosphat
- HPLC: Hochdruck-, Hochleistungsflüssigchromatographie
- kat.: katalytisch
- konz.: konzentriert
- LC-MS: Flüssigchromatographie-gekoppelte Massenspektrometrie
- Lit.: Literatur(stelle)
- MeCN: Acetonitril
- min: Minute(n)
- MS: Massenspektrometrie
- NMP: N-Methylpyrrolidon
- NMR: Kernresonanzspektrometrie
- q: Quartett (bei NMR)
- rac.: racemisch
- RP-HPLC: reverse phase HPLC
- RT: Raumtemperatur
- Rₜ: Retentionszeit (bei HPLC)
- s: Singulett (bei NMR)
- t: Triplett (bei NMR)
- t-Bu: tert.-Butyl
- TFA: Trifluoressigsäure
- THF: Tetrahydrofuran
- verd.: verdünnt

### HPLC-, LC-MS- und GC-MS-Methoden:

Methode 1 (HPLC): Instrument: Hewlett Packard Series 1050; Säule: Symmetry TM C18 3.9 x 150 mm; Fluss: 1.5 ml/min; Eluent A: Wasser, Eluent B: Acetonitril; Gradient: → 0.6 min 10% B → 3.8 min 100% B → 5.0 min 100% B → 5.5 min 10% B; Stopzeit: 6.0 min; Injektionsvolumen: 10 µl; Diodenarraydetektor-Signal: 214 und 254 nm.
Methode 2 (LC-MS): Gerätetyp MS: Micromass ZQ; Gerätetyp HPLC: Waters Alliance 2795; Säule: Merck Chromolith SpeedROD RP-18e 100 mm x 4.6 mm; Eluent A: 11 Wasser + 0.5 ml 50%-ige Ameisensäure, Eluent B: 11 Acetonitril + 0.5 ml 50%-ige Ameisensäure; Gradient: 0.0 min 10% B → 7.0 min 95% B → 9.0 min 95% B; Ofen: 35°C; Fluss: 0.0 min 1.0 ml/min → 7.0 min 2.0 ml/min → 9.0 min 2.0 ml/min; UV-Detektion: 210 nm.
Methode 3 (LC-MS): Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min. 2 ml/min; Ofen: 50°C; UV-Detektion: 210 nm.
Methode 4 (LC-MS): Instrument: Micromass Quattro LCZ mit HPLC Agilent Serie 1100; Säule: Phenomenex Onyx Monolithic C18, 100 mm x 3 mm. Eluent A: 1.1 Wasser + 0.5 ml 50%ige Ameisensäure, Eluent B: 1 1 Acetonitril + 0.5 ml 50%ige Ameisensäure; Gradient: 0.0 min 90% A → 2 min 65% A → 4.5 min 5% A → 6 min 5% A; Fluss: 2 ml/min; Ofen: 40°C; UV-Detektion: 208- 400 nm.
Methode 5 (LC-MS): Gerätetyp MS: Micromass ZQ; Gerätetyp HPLC: Waters Alliance 2795; Säule: Phenomenex Synergi 2.5 µ MAX-RP 100A Mercury 20 mm x 4mm; Eluent A: 11 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.
Methode 6 (LC-MSI): Instrument: Micromass QuattroPremier mit Waters UPLC Acquity ; Säule: Thermo Hypersil GOLD 1,9µ 50 x 1mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 1.5 min 10% A → 2.2 min 10% A; Ofen: 50°C; Fluss: 0.33 ml/min; UV-Detektion: 210 nm.
Methode 7 (LC-MS): Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.
Methode 8 (LC-MS): Instrument: Micromass Quattro LCZ mit HPLC Agilent Serie 1100; Säule: Phenomenex Synergi 2.5 µ MAX-RP 100A Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208- 400 nm.
Methode 9 (LC-MS): Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 11 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 208- 400 nm.
Methode 10 (LC-MS): Gerätetyp MS: Micromass ZQ; Gerätetyp HPLC: Waters Alliance 2795; Säule: Merck Chromolith SpeedROD RP-18e 100 x 4.6mm; Eluent A: 11 Wasser + 0.5 ml 50%ige Ameisensäure; Eluent B: 1 1 Acetonitril + 0.5 ml 50%ige Ameisensäure; Gradient: 0.0 min 10% B → 7.0 min 95% B → 9.0 min 95% B; Ofen: 35°C; Fluss: 0.0 min 1.0 ml/min→ 7.0 min 2.0 ml/min→ 9.0 min 2.0 ml/min; UV-Detektion: 210 nm
Methode 11 (LC-MS): Instrument: Micromass Quattro LCZ mit HPLC Agilent Serie 1100; Säule: Phenomenex Gemini 3µ 30 mm x 3.00 mm; Eluent A: 11 Wasser + 0.5 ml 50%ige Ameisensäure, Eluent B: 11 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 208- 400 nm.
Methode 12 (LC-MS): Gerätetyp MS: M-40 DCI (NH₃); Gerätetyp HPLC: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Ausgangsverbindungen und Intermediate:

### Beispiel 1A

### 2-Amino-4-[4-(2-hydroxyethoxy)phenyl]-6-sulfanylpyridin-3,5-dicarbonitril

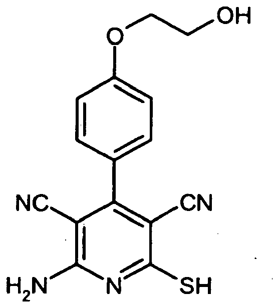

Die Darstellung wurde, wie in WO 03/053441 für Beispiel 6 (1. Stufe) beschrieben, durchgeführt.

LC-MS (Methode 4): Rₜ = 1.73 min; MS (ESIpos): m/z = 313 [M+H]⁺.

### Beispiel 2A

### 2-Amino-4-phenyl-6-sulfanylpyridin-3,5-dicarbonitril

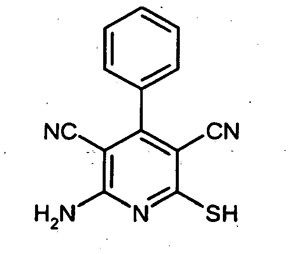

Die Darstellung erfolgt in Analogie zu Beispiel 1A.

MS (ESIpos): m/z = 253 (M+H)⁺

### Beispiel 3A

### 2-Amino-4-(1H-pyrazol-3-yl)-6-sulfanylpyridin-3,5-dicarbonitril

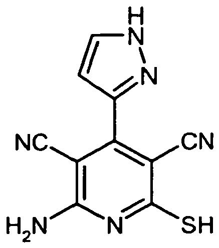

Die Darstellung wurde, wie in WO 03/053441 für Beispiel 6 (1. Stufe) beschreiben aus Pyrazol-3-carbaldehyd, Cyanothioacetamid und 4-Methylmorpholin, durchgeführt.

LC-MS (Methode 6): Rₜ = 0.44 min; MS (ESIpos): m/z = 243 [M+H]⁺.

### Beispiel 4A

### 2'-Amino-6'-sulfanyl-3,4'-bipyridin-3',5'-dicarbonitril

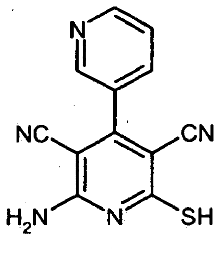

Die Darstellung erfolgte in Analogie zu Beispiel 1 A.

LC-MS (Methode 3): Rₜ = 1.26 min; MS (ESIpos): m/z = 254 [M+H]+.

### Beispiel 5A

### 3-[({6-Amino-3,5-dicyano-4-[4-(2-hydroxyethoxy)phenyl]pyridin-2-yl}sulfanyl)methyl]-benzoesäure

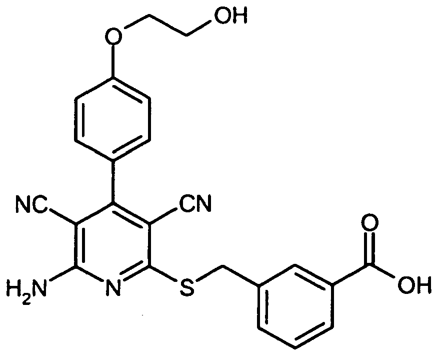

6.50 g (20.81 mmol) der Verbindung aus Beispiel 1A, 5.24 g (62.43 mmol) Natriumhydrogencarbonat und 3.91 g (22.89 mmol) 3-(Chlormethyl)benzoesäure wurden in 100 ml absolutem DMF zusammengegeben und 1.5h bei Raumtemperatur gerührt. Das Reaktionsgemisch wurde auf 700 ml Wasser gegossen, mit 1 N Salzsäure versetzt und 1h gerührt. Der entstandene Niederschlag wurde über eine Glasfritte abgesaugt und mit Wasser gewaschen. Der Rückstand wurde im Vakuum getrocknet.

Ausbeute: 8.56 g (92% d. Th.)

LC-MS (Methode 7): Rₜ = 2.84 min; MS (ESIpos): m/z = 447 [M+H]⁺.

Die in Tabelle 1 aufgeführten Beispiele wurden analog zu Beispiel 5A aus den entsprechenden Edukten mit anschließender Aufreinigung [präparative HPLC (Chroinasil, Wasser/Acetonitril + 0.15% konz. Salzsäure)] hergestellt:

**Tabelle 1:**

| **Beispiel Nr.** | **Struktur (Ausbeute)** | **LC-MS: Rₜ [min] (Methode); MS (ESI): m/z [M+H]⁺** |
|---|---|---|
| **6A** | 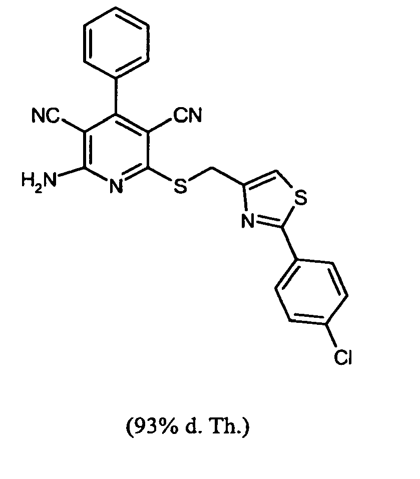 | 4.26 min (Methode 4); m/z = 460 |
| **7A** | 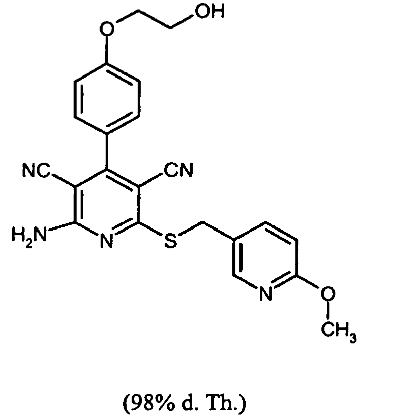 | 3.14 min (Methode 4); m/z = 434 |
| **8A** | 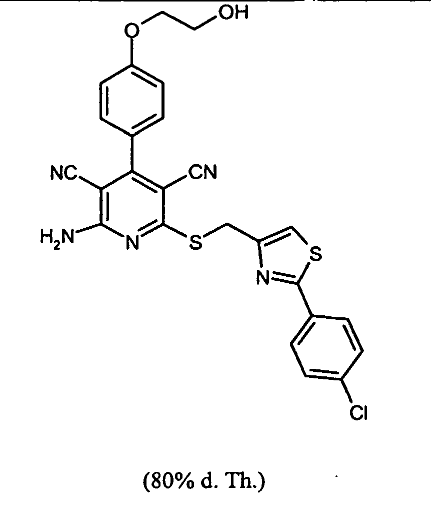 | 5.69 min (Methode 10); m/z = 520 |
| **9A** | 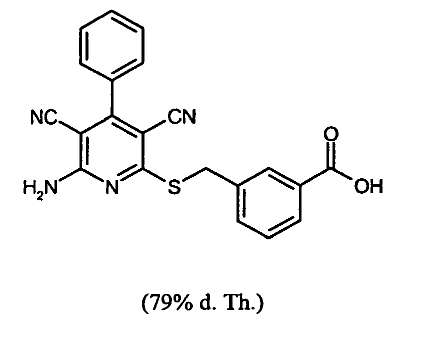 | 3.42 min (Methode 4); m/z = 387 |
| **10A** | 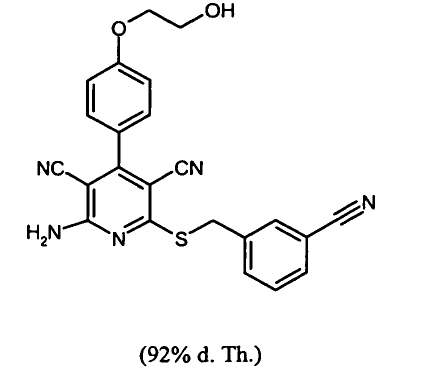 | 3.29 min (Methode 4); m/z = 428 |
| **11A** | 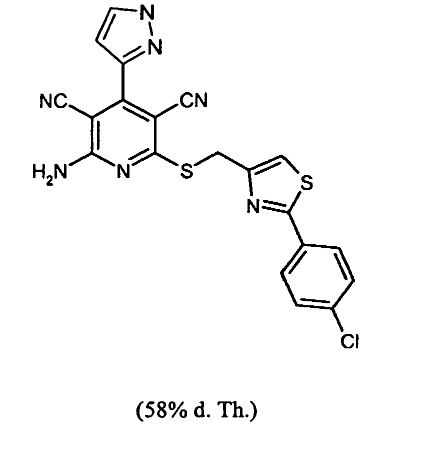 | 2.08 min (Methode 5); m/z = 450 |
| **12A** | 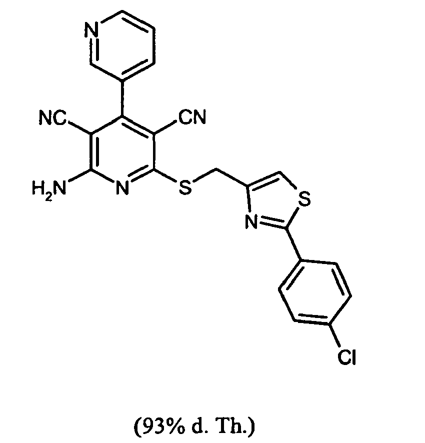 | 2.60 min (Methode 5); m/z = 461 |

### Beispiel 13A

### 3-[({6-Chlor-3,5-dicyano-4-[4-(2-hydroxyethoxy)phenyl]pyridin-2-yl}sulfanyl)methyl]-benzoesäure

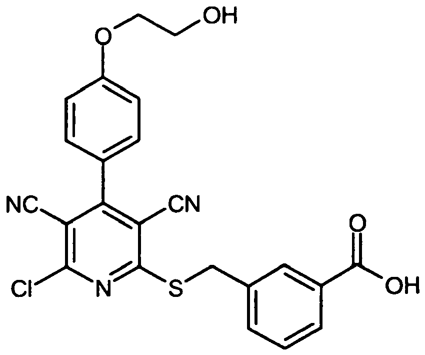

262 mg (2.24 mmol) Isopentylnitrit und 301 mg (2.24 mmol) Kupfer(II)-chlorid wurden in 10.4 ml Acetonitril vorgelegt. Nach Versetzen mit 500 mg (1.12 mmol) der Verbindung aus Beispiel 5A wurde 4h bei 60°C gerührt. Nach Abkühlen auf RT wurden 2.2 ml 1N Salzsäure zugegeben. Die wässrige Phase mit dreimal mit je 30 ml Essigsäureethylester extrahiert. Die vereinigten organischen Phasen wurden einmal mit gesättigter wässriger Natriumhydrogencarbonat-Lösung, zweimal mit Wasser und einmal mit gesättigter wässriger Natriumchlorid-Lösung gewaschen und über Natriumsulfat getrocknet. Nach Entfernen des Lösungsmittels wurde das Produkt ohne weitere Reinigung in der Folgereaktion eingesetzt.

Ausbeute: 600 mg (86% d. Th., Reinheit 75%)

LC-MS (Methode 7): Rₜ = 3.23 min; MS (ESIpos): m/z = 466 [M+H]⁺.

Die in Tabelle 2 aufgeführten Beispiele wurden analog zu Beispiel 13A aus den entsprechenden Edukten mit anschließender Aufreinigung [präparative HPLC (Chromasil, Wasser/Acetonitril + 0.15% konz. Salzsäure)] hergestellt:

**Tabelle 2:**

| **Beispiel Nr.** | **Struktur (Ausbeute)** | **LC-MS: Rₜ [min] (Methode); MS (ESI): m/z [M+H]⁺** |
|---|---|---|
| **14A** | 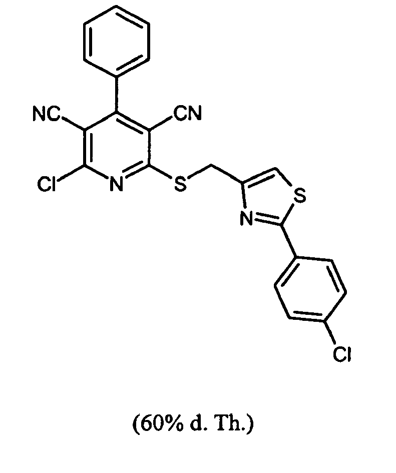 | 3.15 min (Methode 2); m/z = 479 |
| **15A** | 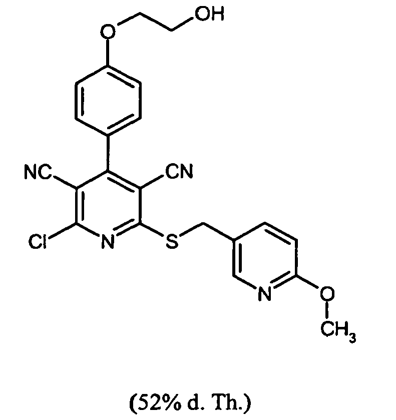 | 3.66 min (Methode 4); m/z = 453 |
| **16A** | 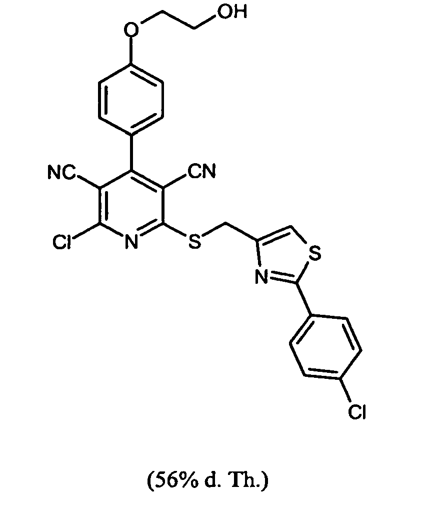 | 3.01 min (Methode 11); m/z = 539 |
| **17A** | 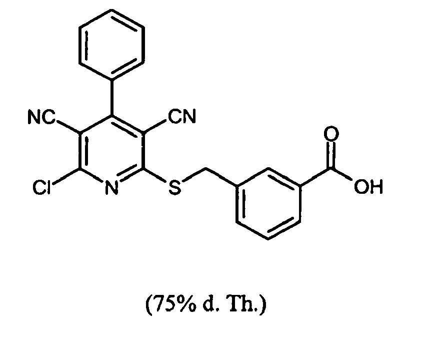 | 3.75 min (Methode 4); m/z = 406 |
| **18A** | 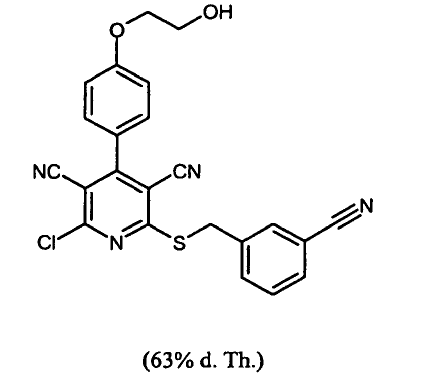 | 4.99 min (Methode 10); m/z = 447 |
| **19A** | 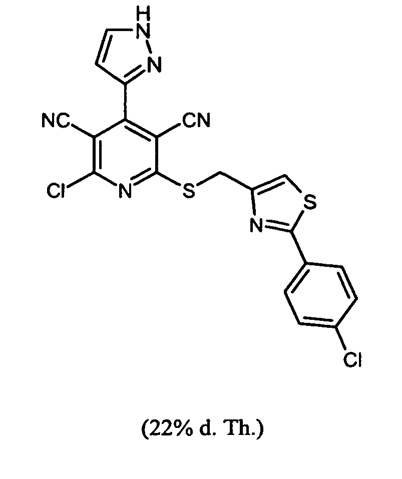 | 1.49 min (Methode 6); m/z = 469 |
| **20A** | 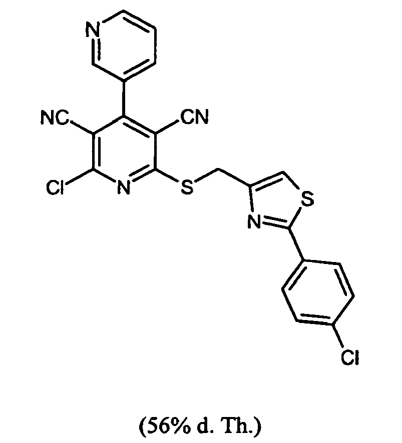 | 2.94 min (Methode 3); m/z = 480 |

### Beispiel 21A

### Methyl-N-{6-({[2-(4-chlorphenyl)-1,3-thiazol-4-yl]methyl}thio)-3,5-dicyano-4-[4-(2-hydroxyethoxy)phenyl]pyridin-2-yl}-N-methylglycinat

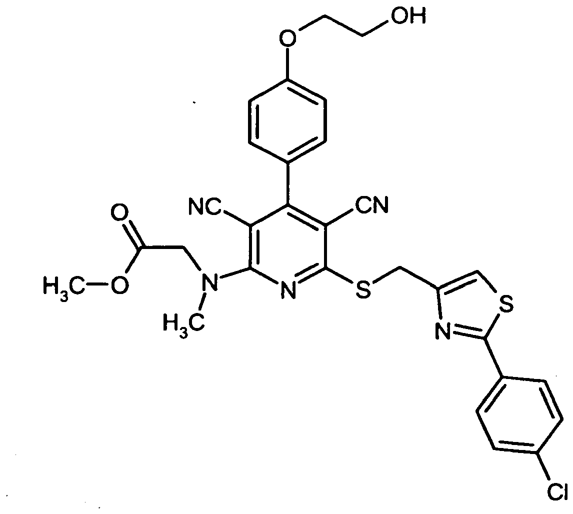

60 mg (0.111 mmol) der Verbindung aus Beispiel 16A, 31 mg (0.222 mmol) Sarcosinmethylester-Hydrochlorid und 0.031 ml (0.222 mmol) Triethylamin wurden in 1.5 ml THF über Nacht bei RT gerührt. Das Reaktionsgemisch wurde mit Wasser versetzt und dreimal mit Essigsäureethylester extrahiert. Die vereinigten organischen Phasen wurden mit gesättigter wässriger Natriumchlorid-Lösung gewaschen, über Natriumsulfat getrocknet und eingeengt. Der Rückstand wurde über eine präparative HPLC (Chromasil, Wasser/Acetonitril + 0.3% konz. Salzsäure) gereinigt.

Ausbeute: 50 mg (74% d. Th.)

¹H-NMR (400 MHz, DMSO-d₆): δ = 7.98 (d, 2H), 7.70 (s, 1H), 7.61-7.52 (m, 4H), 7.11 (d, 2H), 4.91 (t, 1H), 4.62-4.57 (m, 4H), 4.09 (t, 2H), 3.74 (q, 2H), 3.66 (s, 3H), 3.48 (s, 3H).

LC-MS (Methode 4): Rₜ = 4.13 min; MS (ESIpos): m/z = 606 [M+H]⁺.

Die in Tabelle 3 aufgeführten Beispiele wurden analog zu Beispiel 21A aus den entsprechenden Edukten mit anschließender Aufreinigung [präparative HPLC (Chromasil, Wasser/Acetonitril + 0.15% konz. Salzsäure)] hergestellt:

**Tabelle 3:**

| **Beispiel Nr.** | **Struktur (Ausbeute)** | **LC-MS: Rₜ [min] (Methode) ; MS (ESI): m/z [M+H]⁺** | **¹H-NMR (DMSO-d₆):** |
|---|---|---|---|
| **22A** | 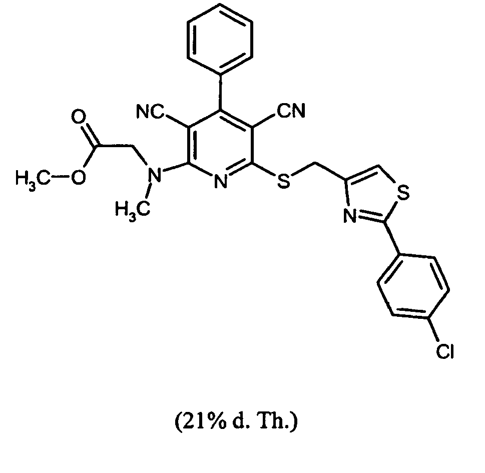 | 4.47 min (Methode 4); m/z = 546 | - |
| **23A** | 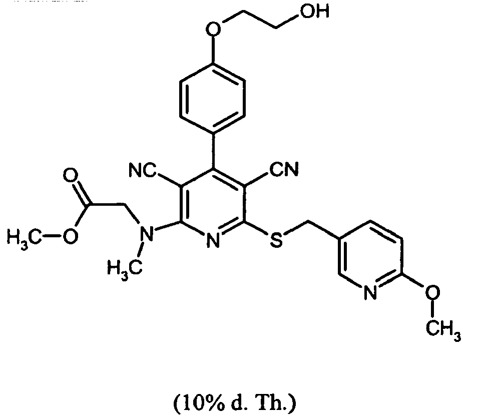 | 2.38 min (Methode 3); m/z = 520 | - |
| **24A** | 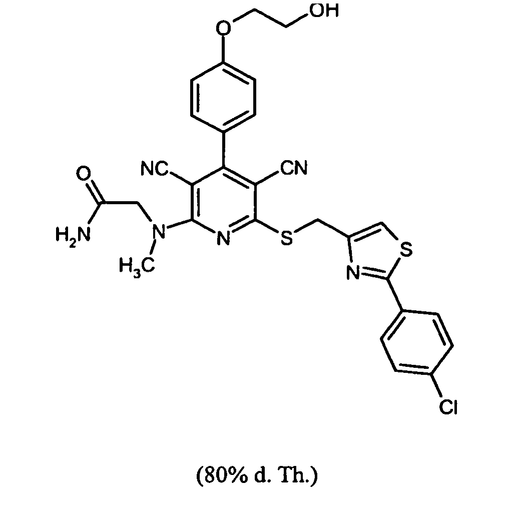 | 2.28 min (Methode 2); m/z = 591 | δ (400 MHz) = 7.98 (d, 2H), 7.71 (s, 1H), 7.61-7.55 (m, 3H), 7.51 (d, 2H), 7.25 (s, 1H), 7.11 (d, 2H), 4.90 (t, 1H), 4.68 (s, 2H), 4.39 (s, 2H), 4.09 (t, 2H), 3.75 (q, 2H), 3.46 (s, 3H). |
| **25A** | 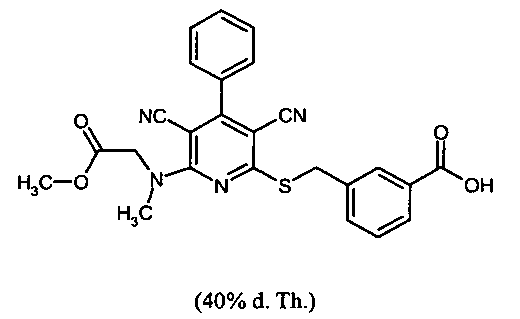 | 2.53 min (Methode 3); m/z= 473 | δ (400 MHz) = 13.08 (s, 1H), 8.00 (s, 1H), 7.87 (d, 1H), 7.65 (d, 1H), 7.61-7.52 (m, 5H), 7.49 (t, 1H), 4.58-4.52 (m, 4H), 3.62 (s, 3H), 3.47 (s, 3H). |
| **26A** | 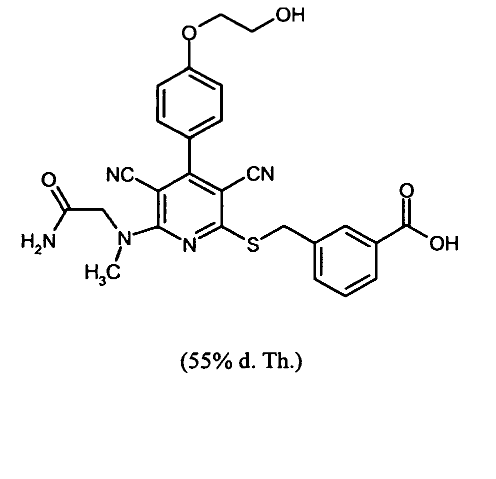 | 1.56 min (Methode 8); m/z= 518 | δ (400 MHz) = 13.06 (s, 1H), 8.00 (s, 1H), 7.87 (d, 1H), 7.68 (d, 1H), 7.59-7.48 (m, 4H), 7.21 (s, 1H), 7.11 (d, 2H), 4.90 (br s, 1H), 4.59 (s, 2H), 4.33 (s, 2H), 4.09 (t, 2H), 3.73 (t, 2H), 3.42 (s, 3H). |
| **27A** | 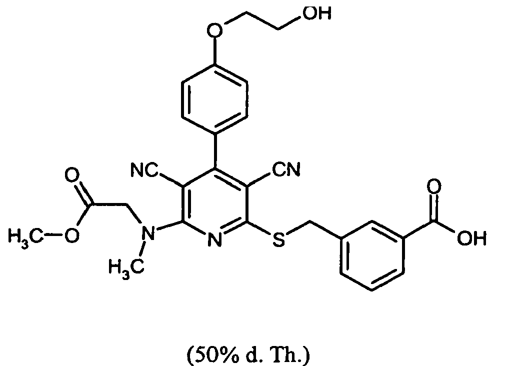 | 1.87 min (Methode 8); m/z = 533 | δ (400 MHz) = 13.06 (s, 1H), 8.00 (s, 1H), 7.87 (d, 1H), 7.66 (d, 1H), 7.58 (d, 2H), 7.50 (t, 1H), 7.11 (d, 2H), 4.90 (br s, 1H), 4.58-4.49 (m, 4H), 4.09 (t, 2H), 3.73 (t, 2H), 3.62 (s, 3H), 3.44 (s, 3H). |
| **28A** | 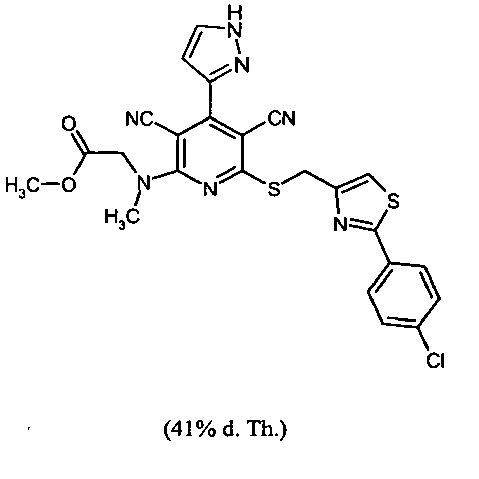 | 1.38 min (Methode 6); m/z = 536 | δ (400 MHz) = 13.61 (s, 1H), 7.99-7.92 (m, 3H), 7.70 (s, 1H), 7.58 (d, 2H), 6.81 (t, 1H), 4.63-54 (m, 4H), 3.67 (s, 3H), 3.47 (s, 3H). |
| **29A** | 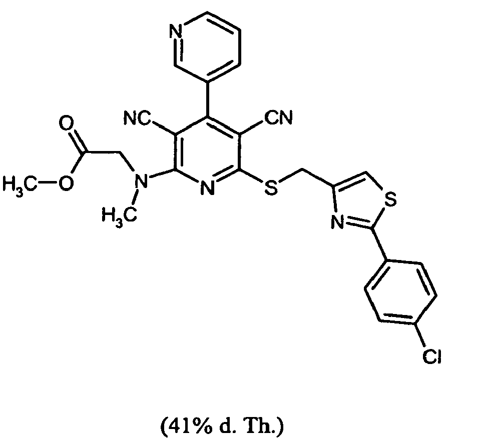 | 1.42 min (Methode 6); m/z = 547 | δ (400 MHz) = 8.82-8.76 (m, 2H), 8.09 (dt, 1H), 7.95 (d, 2H), 7.70 (s, 1H), 7.66-7.56 (m, 3H), 4.64-4.59 (m, 4H), 3.67 (s, 3H), 3.49 (s, 3H). |

### Beispiel 30A

### Methyl-N-{6-({[2-(4-chlorphenyl)-1,3-thiazol-4-yl]methyl}thio)-3,5-dicyano-4-[4-(2-hydroxyethoxy)phenyl]pyridin-2-yl}glycinat

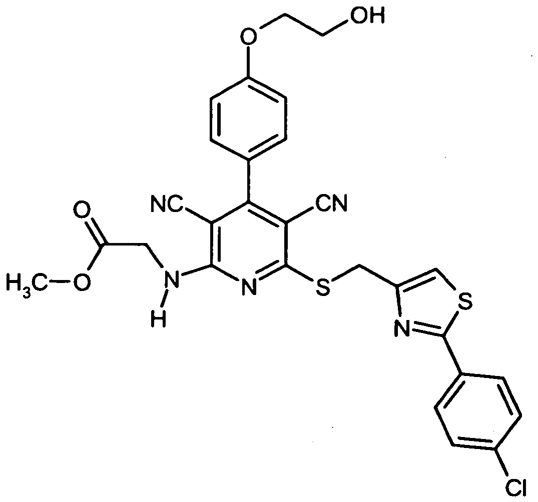

97 mg (0.18 mmol) der Verbindung aus Beispiel 16A; 45 mg (0.36 mmol) Glycinmethylester-Hydrochlorid und 0.05 ml (0.36 mmol) Triethylamin wurden in 2 ml THF 30 min bei RT gerührt. Nach Zusatz von 23 mg (0.18 mmol) Glycinmethylester-Hydrochlorid und 0.025 ml (0.18 mmol) Triethylamin wurde das Reaktionsgemisch 1h bei RT gerührt und anschließend mit Wasser versetzt und vom gebildeten Niederschlag abfiltriert. Dieser wurde im Hochvakuum getrocknet.

Ausbeute: 68 mg (63% d. Th.)

¹H-NMR (400 MHz, DMSO-d₆): δ = 8.58 (t, 1H), 7.98 (d, 2H), 7.68 (s, 1H), 7.60 (d, 2H), 7.53 (d, 2H), 7.12 (d, 2H), 4.91 (t, 1H), 4.61 (s, 2H), 4.25 (d, 2H), 4.09 (t, 2H), 3.74 (q, 2H), 3.62 (s, 3H).

LC-MS (Methode 2): Rₜ = 2.64 min; MS (ESIpos): m/z = 592 [M+H]⁺.

### Beispiel 31A

### Methyl-N-(tert.-butyloxycarbonyl)-N-[4-(4-{2-[(tert.-butyloxycarbonyl)oxy]ethoxy}phenyl)-6-({[2-(4-chlorphenyl)-1,3-thiazol-4-yl]methyl}thio)-3,5-dicyanopyridin-2-yl]glycinat

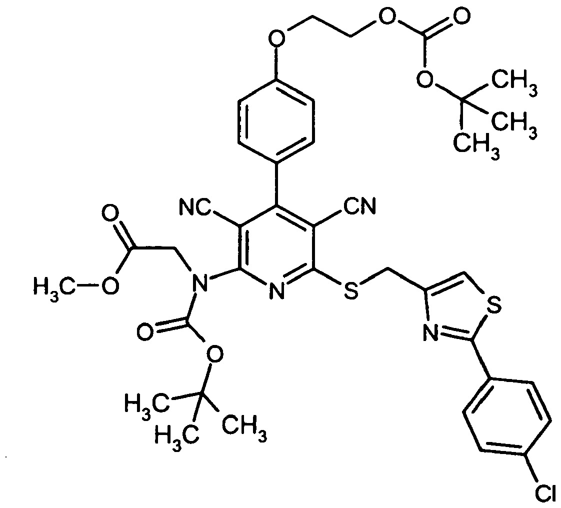

100 mg (0.17 mmol) der Verbindung aus Beispiel 30A wurden in 0.5 ml absolutem Dichlormethan vorgelegt, mit 0.024 ml (0.17 mmol) Triethylamin, 44 mg (0.203 mmol) Di-tert.-butyldicarbonat und 2 mg (0.017 mmol) 4-Dimethylaminopyridin versetzt und über Nacht bei RT gerührt. Das Reaktionsgemisch wurde mit Dichlormethan verdünnt und mit Wasser gewaschen. Die organische Phase wurde über Natriumsulfat getrocknet und eingedampft. Der Rückstand wurde mittels Säulenchromatographie an Kieselgel 60 (Laufmittel: Cyclohexan:Essigsäureethylester = 9:1) aufgereinigt.

Ausbeute: 92 mg (69% d. Th.)

¹H-NMR (400 MHz, DMSO-d₆): δ = 7.96 (d, 2H), 7.70 (s, 1H), 7.60-7.52 (m, 4H), 7.21 (d, 2H), 4.62 (s, 2H), 4.60 (s, 2H), 4.40-4.35 (m, 2H), 4.33-4-28 (m, 2H), 3.60 (s, 3H), 1.49 (s, 9H), 1.42 (s, 9H).

LC-MS (Methode 4): Rₜ = 4.89 min; MS (ESIpos): m/z = 792 [M+H]⁺.

### Beispiel 32A

### 2-Amino-4-[4-(2-{[tert.-butyl(dimethyl)silyl]oxy}ethoxy)phenyl]-6-({[2-(4-chlorphenyl)-1,3-thiazol-4-yl]methyl}thio)pyridin-3,5-dicarbonitril

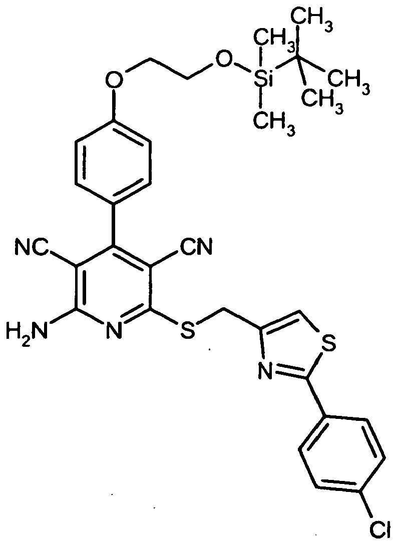

200 mg (0.385 mmol) Beispiel 8A wurden in 4 ml Dichlormethan vorgelegt und mit 161 µl (1.154 mmol) Triethylamin versetzt. Zu der entstandenen Suspension wurde bei RT eine Lösung von 90 mg (0.577 mmol) tert.-Butyl-dimethylsily1chlorid in 1 ml Dichlormethan zugetropft und über Nacht bei RT gerührt. Das Reaktionsgemisch wurde mit 2 ml DMF versetzt und zur entstandenen Lösung wurden nochmals 29 mg (0.19 mmol) tert.-Butyl-dimethylsilylchlorid und 54 ml (0.385 mmol) Triethylamin zugegeben und über Nacht bei RT gerührt. Das Dichlormethan wurde am Rotationsverdampfer entfernt und die verbliebene Lösung über eine präparative HPLC (Chromasil, Wasser/Acetonitril) gereinigt.

Ausbeute: 159 mg (65% d. Th.)

¹H-NMR (400 MHz, DMSO-d₆): δ = 8.12 (br s, 2H), 7.97-7.89 (m, 3H), 7.58 (d, 2H), 7.48 (d, 2H), 7.09 (d, 2H), 4.62 (s, 2H), 4.11 (t, 2H), 3.93 (t, 2H), 0.88 (s, 9H), 0.08 (s, 6H).

LC-MS (Methode 2): Rₜ = 3.37 min; MS (ESIpos): m/z = 634 [M+H]⁺.

### Beispiel 33A

### Methyl-3-amino-4-[4-(2-{[tert.-butyl(dimethyl)silyl]oxy}ethoxy)phenyl]-6-({[2-(4-chlorphenyl)-1,3-thiazol-4-yl]methyl}thio)-5-cyano-1-(2-methoxy-2-oxoethyl)-1H-pyrrolo[2,3-b]pyridin-2-carboxylat

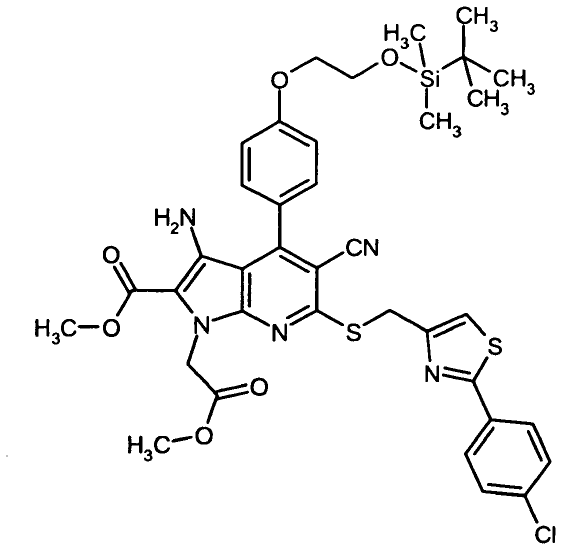

230 mg (0.363 mmol) der Verbindung aus Beispiel 32A wurden unter Argon in 4 ml DMF vorgelegt und portionsweise mit 17 mg (60%ig, 0.435 mmol) Natriumhydrid versetzt und anschließend 30 min bei RT gerührt. Anschließend wurde eine Lösung von 41 µl (0.435 mmol) Bromessigsäuremethylester in 1 ml DMF zugetropft und die resultierende Lösung wurde über Nacht bei RT gerührt. Das Reaktionsgemisch wurde mit Wasser versetzt und dreimal mit Essigsäureethylester extrahiert. Die vereinten organischen Phasen wurden mit gesättigter wässriger Natriumchlorid-Lösung gewaschen, über Natriumsulfat getrocknet und eingeengt. Der Rückstand wurde über eine präparative HPLC (Chromasil, Wasser/Acetonitril) gereinigt.

Ausbeute: 23 mg (8% d. Th.)

¹H-NMR (500 MHz, DMSO-d₆): δ = 7.97 (d, 2H), 7.64 (s, 1H), 7.59 (d, 2H), 7.49 (d, 2H), 7.18 (d, 2H), 5.31 (s, 2H), 4.93 (br s, 2H), 4.74 (s, 2H), 4.14 (t, 2H), 3.98 (t, 2H), 3.73 (s, 3H), 3.62 (s, 3H), 0.89 (s, 9H), 0.10 (s, 6H).

LC-MS (Methode 2): Rₜ = 3.47 min; MS (ESIpos): m/z = 778 [M+H]⁺.

### Beispiel 34A

### N-{6-({[2-(4-Chlorphenyl)-1,3-thiazol-4-yl]methyl}thio)-3,5-dicyano-4-[4-(2-hydroxyethoxy)phenyl]pyridin-2-yl} -N-methylglycin

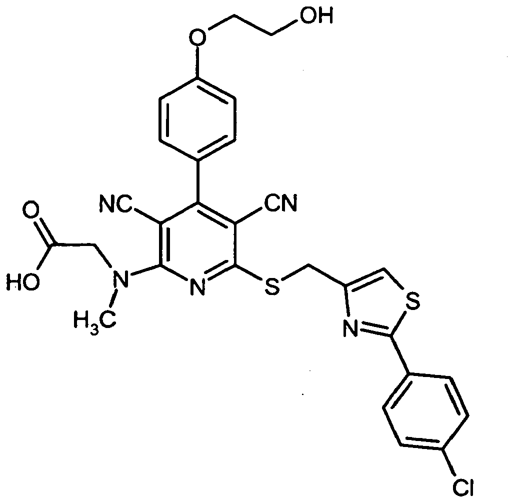

Zu einer Lösung von 300 mg (0.5 mmol) der Verbindung aus Beispiel 16A in 6 ml 1,4-Dioxan wurde eine Lösung von 89 mg (1 mmol) Sarcosin in 1 ml 1N wässriger Natriumhydroxid-Lösung zugegeben und anschließend 3h bei RT gerührt. Das Reaktionsgemisch wurde mit Wasser und dann mit 1N Salzsäure versetzt und dreimal mit Essigsäureethylester extrahiert. Die vereinigten organischen Phasen wurden mit gesättigter wässriger Natriumchlorid-Lösung gewaschen, über Natriumsulfat getrocknet und eingeengt. Der Rückstand wurde über eine präparative HPLC (Chromasil, Wasser/Acetonitril + 0.15% konz. Salzsäure) gereinigt.

Ausbeute: 211 mg (68% d. Th.)

¹H-NMR (500 MHz, DMSO-d₆): δ = 7.94 (d, 2H), 7.79 (s, 1H), 7.57 (d, 2H), 7.50 (d, 2H), 7.10 (d, 2H), 4.93 (br s, 1H), 4.63 (s, 2H), 4.38 (s, 2H), 4.09 (t, 2H), 3.73 (t, 2H), 3.44 (s, 3H).

LC-MS (Methode 2): Rₜ = 3.79 min; MS (ESIpos): m/z = 592 [M+H]⁺.

### Beispiel 35A

### Methyl N-{3,5-dicyano-6-[(3-cyanobenzyl)thio]-4-[4-(2-hydroxyethoxy)phenyl]pyridin-2-yl} -N-methylglycinat

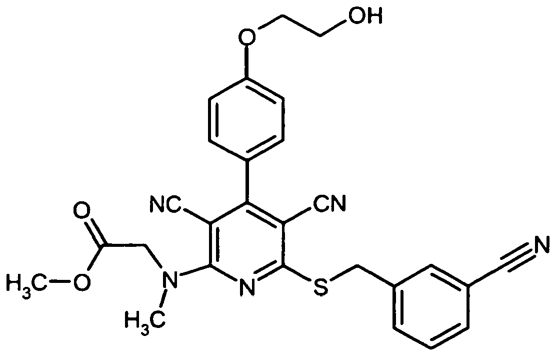

Zu einer Lösung von 300 mg (0.56 mmol) der Verbindung aus Beispiel 18A in 7.3 ml 1,4-Dioxan wurde mit eine Lösung von 155 mg (1.11 mmol) Sarcosinmethylester in 1.1 ml 1N wässriger Natriumhydroxid-Lösung zugegeben und anschließend über Nacht bei RT gerührt. Anschließend wurden nochmals dieselben Mengen Sarcosinmethylester und Natriumhydroxid-Lösung hinzugegeben und 4h bei RT gerührt. Das Reaktionsgemisch wurde mit Wasser versetzt und dreimal mit Essigsäureethylester extrahiert. Die vereinigten organischen Phasen wurden mit gesättigter wässriger Natriumchlorid-Lösung gewaschen, über Natriumsulfat getrocknet und eingeengt. Der Rückstand wurde über eine präparative HPLC (Chromasil, Wasser/Acetonitril + 0.15% konz. Salzsäure) gereinigt.

Ausbeute: 179 mg (62% d. Th.)

¹H-NMR (500 MHz, DMSO-d₆): δ = 7.87 (s, 1H), 7.79 (d, 1H), 7.74 (d, 1H), 7.59-7.53 (m, 3H), 7.11 (d, 2H), 4.92 (t, 1H), 4.54 (s, 2H), 4.52 (s, 2H), 4.09 (t, 2H), 3.75 (q, 2H), 3.61 (s, 3H), 3.44 (s, 3H).

LC-MS (Methode 7): Rₜ = 3.28 min; MS (ESIpos): m/z = 514 [M+H]⁺.

### Beispiel 36A

### N²-{6-({[2-(4-Chlorphenyl)-1,3-thiazol-4-yl]methyl}sulfanyl)-3,5-dicyano-4-[4-(2-hydroxyethoxy)-phenyl]pyridin-2-yl}-N,N²-dimethylglycinamid

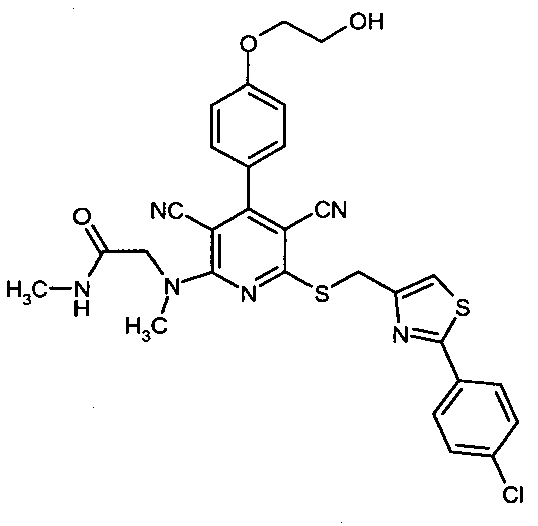

100 mg (0.162 mmol) der Verbindung aus Beispiel 34A wurden in 2 ml DMF vorgelegt, auf 0°C abgekühlt und mit 123 mg (0.324 mmol) HATU versetzt. Nach 20 min wurde mit 243 µl (0.486 mmol) Methylamin und 56 ml (0.324 mmol) N,N-Diisopropylethylamin versetzt und anschließend über Nacht bei RT gerührt. Das Reaktionsgemisch wurde mit Wasser versetzt und dreimal mit Essigsäureethylester extrahiert. Die vereinten organischen Phasen wurden mit gesättigter wässriger Natriumchlorid-Lösung gewaschen, über Natriumsulfat getrocknet und eingeengt. Der Rückstand wurde über eine präparative HPLC (Chromasil, Wasser/Acetonitril + 0.15% konz. Salzsäure) gereinigt. Es wurden 37 mg der gewünschten Verbindung erhalten. Zusätzlich fiel über Nacht aus der wässrigen Phase nochmals ein Feststoff aus, der abfitriert und mit wenig Wasser gewaschen wurde. Man erhielt nochmals 50 mg der gewünschten Verbindung.

Gesamtausbeute: 87 mg (86% d. Th.)

¹H-NMR (500 MHz, DMSO-d₆): δ = 8.03-7.92 (m, 3H), 7.70 (s, 1H), 7.59 (d, 2H), 7.51 (d, 2H), 7.12 (d, 2H), 4.61 (s, 2H), 4.39 (s, 2H), 4.09 (t, 2H), 3.73 (t, 2H), 3.44 (s, 3H), 2.61 (d, 3H).

LC-MS (Methode 3): Rₜ = 2.60 min; MS (ESIpos): m/z = 605 [M+H]⁺.

### Beispiel 37A

### 4-{[(4R)-2,2-Dimethyl-1,3-dioxolan-4-yl]methoxy}benzaldehyd

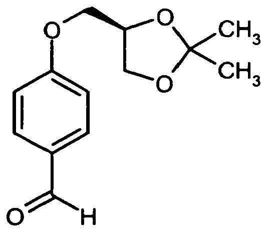

31.2 g (255.4 mmol) 4-Hydroxybenzaldehyd wurden in 400 ml trockenem DMF vorgelegt und bei RT mit 105.7 g (766.1 mmol) Kaliumcarbonat sowie 50.0 g (332.0 mmol) (S)-(-)-3-Chlor-1,2-propandiol-Acetonid versetzt. Es wurde 16h bei 160°C gerührt. Der Ansatz wurde dann mit 4000 ml Wasser versetzt und dreimal mit je 500 ml Ethylacetat extrahiert. Die vereinigten organischen Phasen wurden jeweils einmal mit 500 ml Wasser und 500 ml gesättigter wässriger Natriumchlorid-Lösung gewaschen. Nach Trocknen über Magnesiumsulfat wurde das Lösungsmittel am Rotationsverdampfer entfernt und der Rückstand mittels Säulenchromatographie an Kieselgel 60 aufgereinigt (Laufinittel-Gradient: Ethylacetat/Petrolether 1:9 → 2:8).

Ausbeute: 40.4 g (63% d. Th.)

¹H-NMR (400 MHz, DMSO-d₆): δ = 9.90 (s, 1H), 7.85 (d, 2H), 7.03 (d, 2H), 4.50 (q, 1H), 4.22-4.09 (m, 2H), 4.04 (dd, 1H), 3.92 (dd, 1H), 1.48 (s, 3H), 1.41 (s, 3H).

LC-MS (Methode 12): Rₜ = 3.97 min; MS (ESIpos): m/z = 254 [M+NH₄]⁺.

### Beispiel 38A

### 2-Amino-4-(4-{[(4R)-2,2-dimethyl-1,3-dioxolan-4-yl]methoxy}phenyl)-6-mercaptopyridin-3,5-dicarbonitril

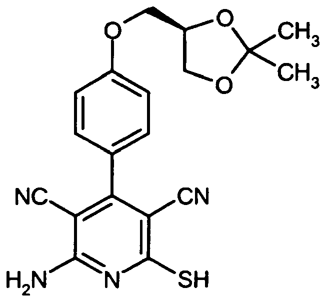

40.4 g (171.0 mmol) der Verbindung aus Beispiel 37A und 34.2 g (342.0 mmol) Cyanothioacetamid wurden in 700 ml Ethanol vorgelegt. Das Reaktionsgemisch wurde mit 34.5 g (342.0 mmol) 4-Methylmorpholin versetzt und 3h unter Rühren zum Rückfluss erhitzt. Nach Abkühlen auf RT wurde weitere 16h bei dieser Temperatur nachgerührt. Der ausgefallene Niederschlag wurde abgesaugt, mit ca. 100 ml Ethanol gewaschen und im Trockenschrank getrocknet. Das Produkt wurde ohne weitere Reinigung in den Folgereaktionen eingesetzt.

Ausbeute: 19.5 g (29% d. Th.)

¹H-NMR (400 MHz, DMSO-d₆): δ = 7.63-7.31 (br s, 2H), 7.41 (d, 2H), 7.09 (d, 2H), 4.49-4.38 (m, 1H), 4.15-3.99 (m, 2H), 3.78 (dd, 1H), 3.66 (dd, 1H), 2.77-2.68 (br s, 1H), 1.37 (s, 3H), 1.32 (s, 3H).

LC-MS (Methode 9): Rₜ = 1.95 min; MS (ESIpos): m/z = 424 [M+H+CH₃CN]⁺.

### Beispiele 39A

### 2-Amino-6-({[2-(4-chlorphenyl)-1,3-oxazol-4-yl]methyl}sulfanyl)-4-(4-{[(4R)-2,2-dimethyl-1,3-dioxolan-4-yl]methoxy}phenyl)pyridin-3,5-dicarbonitril

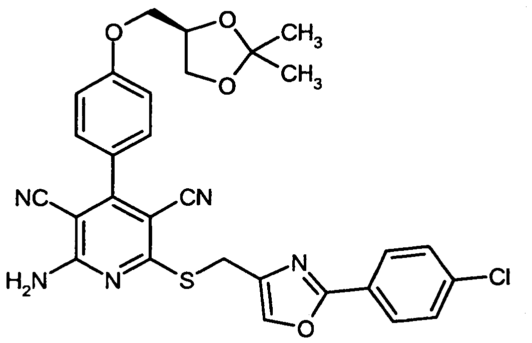

70 mg (0.18 mmol) der Verbindung aus Beispiel 38A und 46 mg (0.20 mmol) 4-(Chlormethyl)-2-(4-chlorphenyl)-1,3-oxazol wurden zusammen mit 46 mg (0.55 mmol) Natriumhydrogencarbonat in 1.9 ml trockenem DMF suspendiert. Das Reaktionsgemisch wurde 20h bei RT gerührt. Der Ansatz wurde danach am Rotationsverdampfer vom Lösungsmittel befreit und der Rückstand mittels präparativer HPLC gereinigt (Säule: YMC GEL ODS-AQ S-5 / 15 µm; LaufmittelGradient: Acetonitril/ Wasser 10:90 → 95:5).

Ausbeute: 79 mg (75% d. Th.)

¹H-NMR (400 MHz, DMSO-d₆): δ = 8.37 (s, 1H), 8.30-8.01 (br s, 2H), 7.97 (d, 2H), 7.60 (d, 2H), 7.48 (d, 2H), 7.12 (d, 2H), 4.48-4.40 (m, 1H), 4.42 (s, 2H), 4.16-4.03 (m, 3H), 3.78 (dd, 1H), 1.37 (s, 3H), 1.31 (s, 3H).

LC-MS (Methode 3): Rₜ = 2.99 min; MS (ESIpos): m/z = 574 [M+H]⁺.

### Beispiel 40A

### 2-Amino-6-({[2-(4-chlorphenyl)-1,3-oxazol-4-yl]methyl}sulfanyl)-4-(4-{[(2S)-2,3-dihydroxypropyl]oxy}phenyl)pyridin-3,5-dicarbonitril

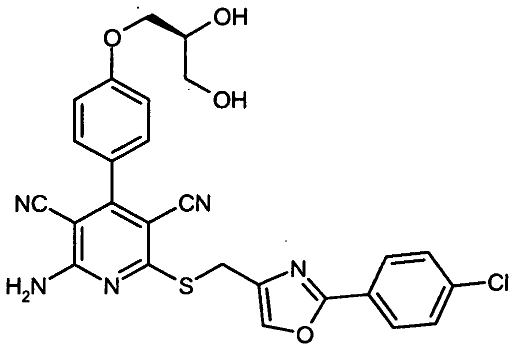

400 mg (0.70 mmol) der Verbindung aus Beispiel 39A wurden in 17 ml Essigsäure vorgelegt und anschließend vorsichtig mit 8.6 ml Wasser versetzt. Es wurde 12h bei RT gerührt. Nach Einengen des Reaktionsgemisches am Rotationsverdampfer wurde der Rückstand direkt mittels präparativer HPLC gereinigt (Säule: YMC GEL ODS-AQ S-5 / 15 µm; Laufinittel-Gradient: Acetonitril/Wasser 10:90 → 95:5). Nach Entfernen des Lösungsmittels am Rotationsverdampfer erhielt man das Produkt als Feststoff.

Ausbeute: 340 mg (91% d. Th.)

¹H-NMR (400 MHz, DMSO-d₆): δ = 8.37 (s, 1H), 8.27-7.91 (br s, 2H), 7.98 (d, 2H), 7.60 (d, 2H), 7.47 (d, 2H), 7.10 (d, 2H), 5.00 (d, 1H), 4.70 (t, 1H), 4.42 (s, 2H), 4.09 (dd, 1H), 3.96 (dd, 1H), 3.70 (q, 1H), 3.46 (t, 2H).

LC-MS (Methode 3): Rₜ = 2.48 min; MS (ESIpos): m/z = 534 [M+H]⁺.

### Beispiel 41A

### 2-Chlor-6-({[2-(4-chlorphenyl)-1,3-oxazol-4-yl]methyl}thio)-4-(4-{[(2S)-2,3-dihydroxypropyl]oxy} phenyl)pyridin-3,5-dicarbonitril

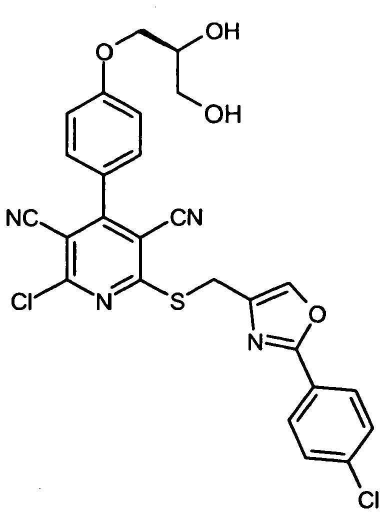

171 mg (1.46 mmol) Isopentylnitrit und 196 mg (1.46 mmol) Kupfer(II)-chlorid wurden in 18 ml Acetonitril vorgelegt. Nach Versetzen mit 389 mg (0.73 mmol) der Verbindung aus Beispiel 40A wurde 3h bei 60°C gerührt. Nach Abkühlen auf RT wurden 20 ml 1N Salzsäure zugegeben. Die wässrige Phase mit zweimal mit je 30 ml Essigsäureethylester extrahiert. Die vereinigten organischen Phasen wurden über Magnesiumsulfat getrocknet. Nach Entfernen des Lösungsmittels wurde das Produkt ohne weitere Reinigung in der Folgereatkion eingesetzt.

Ausbeute: 451 mg (77% d. Th., Reinheit 69%)

LC-MS (Methode 3): Rₜ = 2.84 min; MS (ESIpos): m/z = 553 [M+H]⁺.

### Beispiel 42A

### Methyl-N-[6-({[2-(4-chlorphenyl)-1,3-oxazol-4-yl]methyl}thio)-3,5-dicyano-4-(4-{[(2S)-2,3-dihydroxypropyl]oxy}phenyl)pyridin-2-yl]-N-methylglycinat

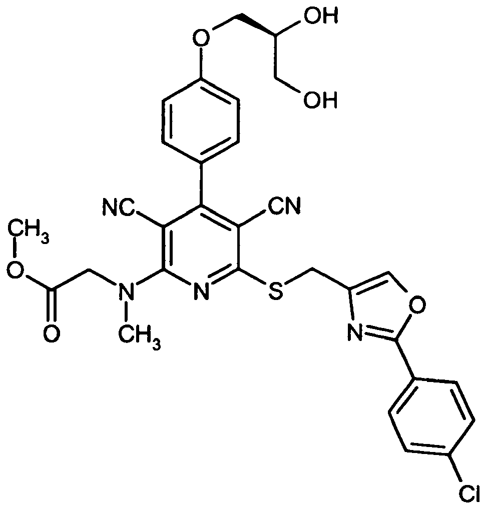

451 mg (0.51 mmol) der Verbindung aus Beispiel 40A wurden in 7.8 ml trockenem THF gelöst und nacheinander mit 141 mg (1.01 mmol) Sarcosinsäuremethylester-Hydrochlorid und 211 µl (1.52 mmol) Triethylamin versetzt. Das Reaktionsgemisch wurde 8h bei RT gerührt. Nach Entfernen des Lösungsmittels wurde der Ansatz direkt mittels präparativer HPLC (Säule: YMC GEL ODS-AQ S-5 /15 µm; Laufmittelgradient: Acetonitril/Wasser 10:90 → 95:5) gereinigt.

Ausbeute: 52 mg (15% d. Th.)

¹H-NMR (400 MHz, DMSO-d₆): δ = 8.19 (s, 1H), 7.98 (d, 2H), 7.62 (d, 2H), 7.55 (d, 2H), 7.11 (d, 2H), 5.01 (d, 1H), 4.70 (t, 1H), 4.61 (s, 2H), 4.42 (s, 2H), 4.09 (dd, 1H), 3.96 (dd, 1H), 3.87-3.78 (m, 1H), 3.65 (s, 3H), 3.52-3.43 (m, 5H).

LC-MS (Methode 3): Rₜ = 2.70 min; MS (ESIpos): m/z = 620 [M+H]⁺.

### Beispiel 43A

### 2-Amino-6-mercapto-4-(methylthio)pyridin-3,5-dicarbonitril

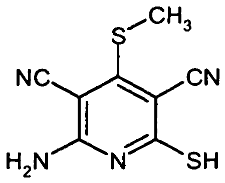

10 g (58.74 mmol) 2-(Di(methylthio))methylidenmalononitril und 7.1 g (70.48 mmol) Cyanothioacetamid wurden in 21 ml DMF vorgelegt und mit 16.4 ml (117.47 mmol) Triethylamin tropfenweise bei Raumtemperatur versetzt. Der Ansatz wurde 8h bei Raumtemperatur gerührt. Das Reaktionsgemisch wurde auf 300 ml 3N Salzsäure gegeben. Der ausfallende Niederschlag wurde abgesaugt, mit Wasser gewaschen und getrocknet. Man erhielt das Produkt als Pulver.

Ausbeute: 12.2 g (89 % d. Th., 96% Reinheit)

¹H-NMR (400 MHz, CDCl₃): δ = 3.98 (s, 1H), 2.72 (s, 3H).

LC-MS (Methode 7): Rₜ = 1.56 min; MS (ESIpos): m/z = 223 [M+H]⁺.

### Beispiel 44A

### 2-Amino-6-({[2-(4-chlorphenyl)-1,3-thiazol-4-yl]methyl} thio)-4-(methylthio)pyridin-3,5-dicarbonitril

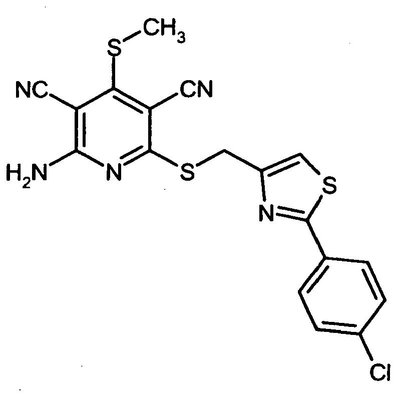

7.30 g (32.84 mmol) der Verbindung aus Beispiel 43A (2-Amino-6-mercapto-4-(methylthio)pyridin-3,5-dicarbonitril), 11.03 g (131.36 mmol) Natriumhydrogencarbonat und 9.62 g (39.41 mmol) 4-(Chlormethyl)-2-(4-chlorphenyl)-1,3-thiazol wurden in 150 ml absolutem DMF zusammengegeben und 12h bei Raumtemperatur gerührt. Es fiel ein Feststoff aus, welcher über eine Glasfritte abgesaugt wurde und dreimal mit Wasser und zweimal mit Diethylether gewaschen wurde. Der Rückstand wurde im Vakuum getrocknet.

Ausbeute: 14.2 g (99 % d. Th.)

¹H-NMR (400 MHz, CDCl₃): δ = 8.14-8.05 (br s, 2H), 7.96 (d, 2H), 7.87 (s, 1H), 7.58 (d, 2H), 4.58 (s, 2H), 2.72 (s, 3H).

LC-MS (Methode 2): Rₜ = 2.67 min; MS (ESIpos): m/z = 430 [M+H]⁺.

### Beispiel 45A

### 2-Amino-6-({[2-(4-chlorphenyl)-1,3-thiazol-4-yl]methyl}sulfanyl)-4-piperidin-1-ylpyridin-3,5-dicarbonitril

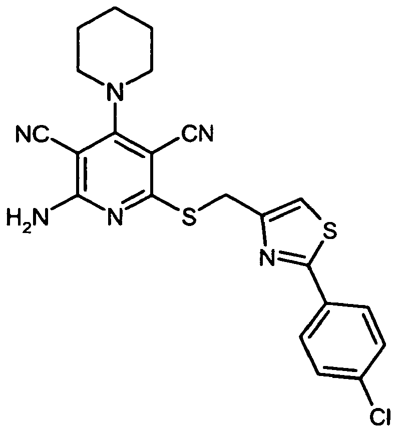

5.00 g (11.63 mmol) der Verbindung aus Beispiel 44A und 57.50 ml (581.43 mmol) Piperidin wurden in 120 ml Aceton vorgelegt und 8h zum Rückfluss erhitzt. Nach dem Abkühlen wurde der Ansatz auf ein Lösungsmittelgemisch von 50 ml gesättigter wässriger Ammoniumchlorid-Lösung und 50 ml Essigsäureethylester gegossen. Die Phasen wurden getrennt. Die organische Phase wurde zweimal mit je 20 ml gesättigter wässriger Natriumchlorid-Lösung gewaschen und anschließend über Magnesiumsulfat getrocknet. Nach Entfernen des Lösungsmittels wurde der Rückstand mit 100 ml Diethylether verrührt. Der Niederschlag wurde abgesaugt und im Vakuum bei 50°C getrocknet.

Ausbeute: 2.00 g (37 % d. Th.)

¹H-NMR (400 MHz, CDCl₃): δ = 7.94 (d, 2H), 7.82 (s, 1H), 7.75-7.59 (br s, 2H), 7.55 (d, 2H), 4.53 (s, 2H), 3.48 (br s, 4H), 1.61 (br s, 6H).

LC-MS (Methode 2): Rₜ = 2.90 min; MS (ESIpos): m/z = 467 [M+H]⁺.

### Beispiel 46A

### 2-Chlor-6-({[2-(4-chlorphenyl)-1,3-thiazol-4-yl]methyl}sulfanyl)-4-piperidin-1-ylpyridin-3,5-dicarbonitril

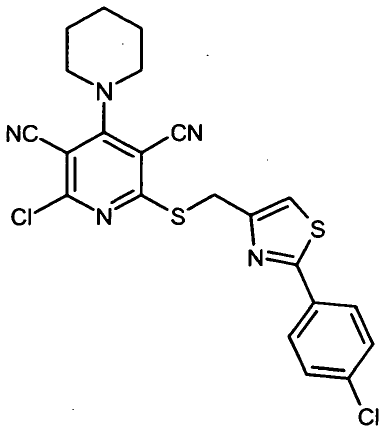

3.85 g (29.55 mmol) Isopentylnitrit und 3.97 g (29.55 mmol) Kupfer(II)-chlorid wurden in 40 ml Acetonitril vorgelegt und mit 2.30 g (4.93 mmol) der Verbindung aus Beispiel 45A versetzt. Das Reaktionsgemisch wurde 3h bei 60°C gerührt. Zur Reaktionslösung wurden 20 ml 1N Salzsäure gegeben. Die wässrige Phase wurde zweimal mit je 40 ml Essigsäureethylester extrahiert. Die vereinigten organischen Phasen wurden je einmal mit je 20 ml gesättigter wässriger Natriumhydrogencarbonat-Lösung und gesättigter wässriger Natriumchlorid-Lösung gewaschen und anschließend über Magnesiumsulfat getrocknet. Das Lösungsmittel wurde am Rotationsverdampfer entfernt. Der Rückstand wurde mittels Säulenchromatographie an Kieselgel 60 (Laufinittelgradient Cyclohexan:Essigsäureethylester = 10:1 → 1:4) aufgereinigt.

Ausbeute: 1.50 g (60 % d. Th.)

¹H-NMR (400 MHz, CDCl₃): δ = 7.94 (d, 2H), 7.67 (s, 1H), 7.57 (d, 2H), 4.53 (s, 2H), 3.68-3.59 (br s, 4H), 1.71-1.59 (br s, 6H).

LC-MS (Methode 2): Rₜ = 2.79 min; MS (ESIpos): m/z = 509 [M+H]⁺.

### Beispiel 47A

### Methyl-N-[6-({[2-(4-chlorphenyl)-1,3-thiazol-4-yl]methyl}sulfanyl)-3,5-dicyano-4-piperidin-1-ylpyridin-2-yl]-N-methylglycinat

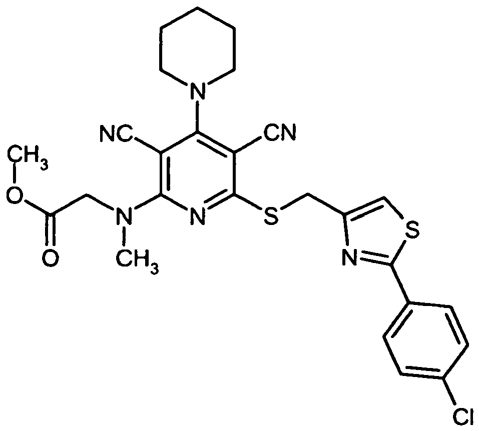

100 mg (0.21 mmol) der Verbindung aus Beispiel 46A wurden in 2 ml trockenem THF gelöst. Anschließend wurden nacheinander 57 mg (0.41 mmol) Sarcosinsäuremethylester-Hydrochlorid und 86 µl (0.62 mmol) Triethylamin zugegeben. Das Reaktionsgemisch wurde 10h bei RT gerührt. Der Ansatz wurde mit 1 ml Wasser versetzt und dreimal mit je 5 ml Essigsäureethylester extrahiert. Die vereinigten organischen Phasen wurden einmal mit 3 ml gesättigter wässriger Natriumchlorid-Lösung gewaschen und über Magnesiumsulfat getrocknet. Nach Entfernen des Lösungsmittels am Rotationsverdampfer wurde der Rückstand mittels präparativer HPLC (Säule: YMC GEL ODS-AQ S-5 / 15 µm; Laufmittelgradient: Acetonitril/Wasser 10:90 → 95:5) gereinigt.

Ausbeute: 80 mg (70 % d. Th.)

¹H-NMR (400 MHz, CDCl₃): δ = 7.95 (d, 2H), 7.62 (s, 1H), 7.58 (d, 2H), 4.51 (s, 2H), 4.46 (s, 2H), 3.13 (s, 3H), 3.07 (br s, 4H), 3.31 (s, 3H), 1.65 (br s, 6H).

LC-MS (Methode 3): Rₜ = 3.24 min; MS (ESIpos): m/z = 553 [M].

### Beispiel 48A

### 2-Amino-4-[4-(2-hydroxyethoxy)phenyl]-6-(phenylsulfanyl)pyridin-3,5-dicarbonitril

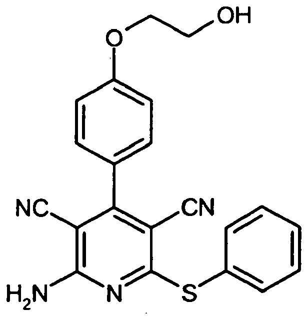

100.0 g (601.8 mmol) 4-(2-Hydroxyethoxy)benzaldehyd wurden in 600 ml absolutem Ethanol vorgelegt und mit 40 g (605.5 mmol) Malonsäuredinitril und 1 ml (7.22 mmol) Triethylamin versetzt und 2h zum Rückfluss erhitzt. Das Heizbad wurde entfernt und der Ansatz mit 43.5 g (658.2 mmol) Malonsäuredinitril, 1.5 ml (10.83 mmol) Triethylamin und 66.3 g (601.8 mmol) Thiophenol versetzt. Es wurde wieder 2h zum Rückfluss erhitzt und anschließend 8h bei RT gerührt. Die Reaktionslösung wurde auf 5°C gekühlt und der entstehende Niederschlag abgesaugt. Der Niederschlag wurde mit 500 ml tert.-Butylmethylether gewaschen. Der Rückstand wurde in 400 ml DMF aufgenommen und auf 70°C erhitzt. Es wurde dann eine Lösung aus 141.1 g (257.4 mmol) Ammonium-cer(IV)-nitrat in 250 ml 50°C warmem Wasser zugetropft. Während der Zugabe wurden weitere 500 ml DMF zugegeben. Nach Zugabe wurde 1h bei RT gerührt. Dann wurden 1000 ml Wasser zugegeben und das Reaktionsgemisch 16h bei RT nachgerührt. Der ausfallende Niederschlag wurde abgesaugt und mit Wasser gewaschen. Der Rückstand wurde im Vakuum getrocknet.

Ausbeute: 95.2 g (89 % d. Th.).

¹H-NMR (400 MHz, DMSO-d₆): δ = 7.83-7.19 (br s, 2H), 7.64-7.58 (m, 2H), 7.53-7.48 (m, 5H), 7.12 (d, 2H), 5.10-4.75 (br s, 1H), 4.10 (t, 2H), 3.75 (t, 2H).

LC-MS (Methode 5): Rₜ = 1.76 min; MS (ESIpos): m/z = 389 [M+H]⁺.

### Beispiele 49A

### 2-Amino-6-{[2-(4-chlorphenyl)-1,3-oxazol-4-yl]methoxy}-4-[4-(2-hydroxyethoxy)phenyl]pyridin-3,5-dicarbonitril

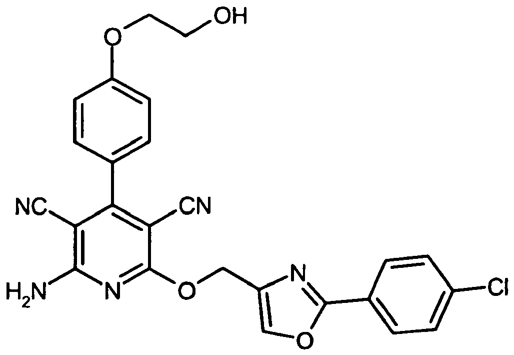

72 mg (0.64 mmol) Kalium-tert.-butylat wurden in 1 ml trockenem Dimethoxyethan suspendiert. Dann wurden nacheinander 270 mg (1.29 mmol) [2-(4-Chlorphenyl)-1,3-oxazol-4-yl]methanol und 50 mg (0.13 mmol) der Verbindung aus Beispiel 48A zugegeben. Das Reaktionsgemisch wurde 2h bei 60°C gerührt, anschließend auf RT abgekühlt und dann 8h bei dieser Temperatur gerührt. Das Reaktionsgemisch wurde mit 5 ml Wasser und 1 ml 2N Essigsäure versetzt. Es fällt ein Niederschlag aus, der abgesaugt wurde. Der Rückstand wurde mittels präparativer HPLC (Säule: YMC GEL ODS-AQ S-5 / 15 µm; Laufmittelgradient: Acetonitril/Wasser 10:90 → 95:5) gereinigt. Nach Entfernen des Lösungsmittels am Rotationsverdampfer erhielt man das Produkt als Feststoff.

Ausbeute: 44 mg (70 % d. Th.).

¹H-NMR (400 MHz, DMSO-d₆): δ = 8.48 (s, 1H), 8.18-7.85 (br s, 2H), 8.00 (d, 2H), 7.62 (d, 2H), 7.48 (d, 2H), 7.11 (d, 2H), 5.41 (s, 2H), 4.91 (t, 1H), 4.08 (t, 2H), 3.73 (q, 2H).

LC-MS (Methode 6): Rₜ = 1.22 min; MS (ESIpos): m/z = 488 [M+H]⁺.

### Beispiel 50A

### 2-Chlor-6-{[2-(4-chlorphenyl)-1,3-oxazol-4-yl]methoxy}-4-[4-(2-hydroxyethoxy)phenyl]pyridin-3,5-dicarbonitril

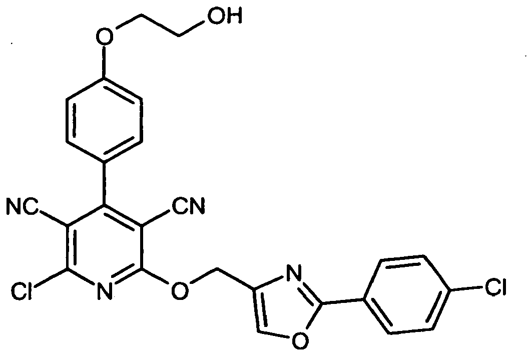

78 mg (0.67 mmol) Isopentylnitrit und 90 mg (0.67 mmol) Kupfer(II)-chlorid wurden in 8.5 ml Acetonitril vorgelegt und mit 72 mg (0.64 mmol) der Verbindung aus Beispiel 49A versetzt. Das Reaktionsgemisch wurde 3h bei 60°C gerührt. Zur Reaktionslösung wurden 8.5 ml 1N Salzsäure gegeben. Die wässrige Phase wurde zweimal mit je 20 ml Essigsäureethylester extrahiert und die vereinigten organischen Phasen über Magnesiumsulfat getrocknet. Nach Entfernen des Lösungsmittels am Rotationsverdampfer wurde der Rückstand im Vakuum getrocknet und ohne weitere Reinigung in der Folgereaktion eingesetzt.

Ausbeute: 231 mg (87 % d. Th., 64 % Reinheit).

LC-MS (Methode 6): Rₜ = 1.40 min; MS (ESIpos): m/z = 507 [M]⁺.

### Beispiel 51A

### Methyl-N-(6-{[2-(4-chlorphenyl)-1,3-oxazol-4-yl]methoxy}-3,5-dicyano-4-[4-(2-hydroxyethoxy)phenyl]pyridin-2-yl)-N-methylglycinat

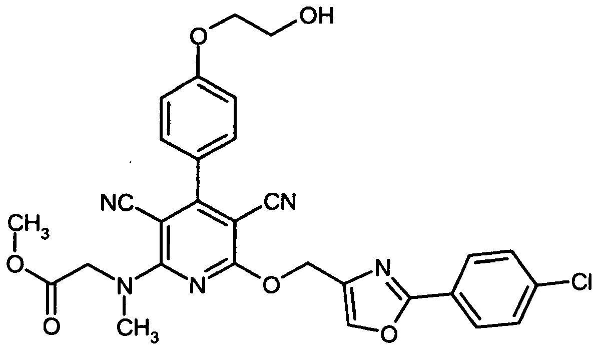

230 mg (ca. 0.45 mmol) des Rohproduktes aus Beispiel 50A wurden in 3 ml absolutem THF gelöst und mit 127 mg (0.91 mmol) Sarcosinsäuremethylester-Hydrochlorid und 138 mg (1.36 mmol) Triethylamin versetzt. Das Reaktionsgemisch wurde 10h bei RT gerührt. Nach Entfernen des Lösungsmittels am Rotationsverdampfers wurde der Rückstand direkt mittels präparativer HPLC (Säule: YMC GEL ODS-AQ S-5 / 15 µm; Laufmittelgradient: Acetonitril/Wasser 10:90 → 95:5) gereinigt. Nach Entfernen des Lösungsmittels am Rotationsverdampfer erhielt man das Produkt als weißen Feststoff.

Ausbeute: 24 mg (9 % d. Th.).

¹H-NMR (400 MHz, DMSO-d₆): δ = 8.34 (s, 1H), 8.00 (d, 2H), 7.62 (d, 2H), 7.53 (d, 2H), 7.11 (d, 2H), 5.39 (s, 2H), 4.98-4.86.(br s, 1H), 4.58 (s, 2H), 4.09 (t, 2H), 3.78-3.71 (m, 2H), 3.68 (s, 3H), 3.49 (s, 3H).

LC-MS (Methode 3): Rₜ = 2.59 min; MS (ESIpos): m/z = 574 [M+H]⁺.

### Ausführnugsbeispiele:

### Beispiel 1

### Methyl-3-amino-6-({[2-(4-chlorphenyl)-1,3-thiazol-4-yl]methyl}thio)-5-cyano-4-[4-(2-hydroxyethoxy)phenyl]-1-methyl-1H-pyrrolo[2,3-b]pyridin-2-carboxylat

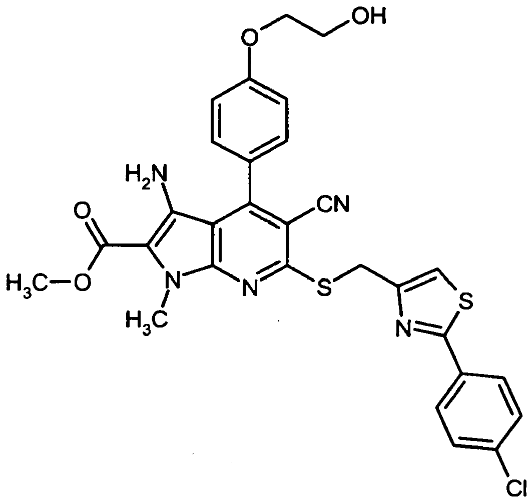

21 mg (0.035 mmol) der Verbindung aus Beispiel 21A wurden in 0.69 ml Acetonitril vorgelegt und mit 45 mg (0.139 mmol) Cäsiumcarbonat versetzt. Das Reaktionsgemisch wurde 2h bei 50°C gerührt und anschließend abgekühlt. Es wurde vom Feststoff abfiltriert und mit Acetonitril gewaschen. Das Filtrat wurde eingeengt und der Rückstand wurde im Hochvakuum getrocknet.

Ausbeute: 17 mg (81% d. Th.)

¹H-NMR (400 MHz, DMSO-d₆): δ = 7.96 (d, 2H), 7.72 (s, 1H), 7.58 (d, 2H), 7.48 (d, 2H), 7.16 (d, 2H), 4.96-4.87 (m, 3H), 4.79 (s, 2H), 4.10 (t, 2H), 3.96 (s, 3H), 3.80 (s, 3H), 3.78 (q, 2H).

LC-MS (Methode 3): Rₜ = 3.16 min; MS (ESIpos): m/z = 606 [M+H]⁺.

Die in Tabelle 4 aufgeführten Beispiele wurden aus den entsprechenden Ausgangsverbindungen analog zu Beispiel 1 mit anschließender Aufreinigung [präparative HPLC (Chromasil, Wasser/Acetonitril + 0.15% konz. Salzsäure)] hergestellt:

**Tabelle 4:**

| **Beispiel Nr.** | **Struktur (Ausbeute)** | **LC-MS: Rₜ [min] (Methode) ; MS (ESI): m/z [M+H]⁺** | **¹H-NMR (DMSO-d₆):** |
|---|---|---|---|
| **2** | 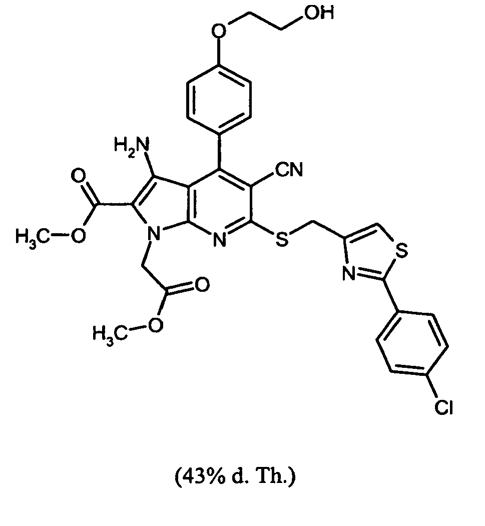 | 2.83 min (Methode 2); m/z = 664 | δ (500 MHz) = 7.96 (d, 2H), 7.64 (s, 1 H), 7.58 (d, 2H), 7.51 (d, 2H), 7.19 (d, 2H), 5.31 (s, 2H), 4.98-4.89 (m, 3H), 4.73 (s, 2H), 4.12-4.05 (m, 2H), 3.79-3.70 (m, 5H), 3.62 (s, 3H). |
| **3** | 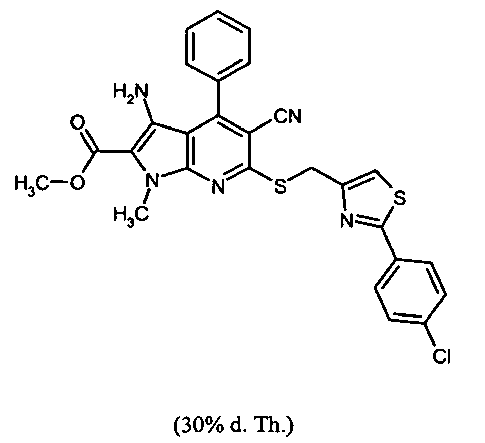 | 4.64 min (Methode 7); m/z = 546 | δ (500 MHz) = 7.98 (d, 2H), 7.73 (s, 1H), 7.65-7.60 (m, 3H), 7.59-7.50 (m, 4H), 4.81 (s, 2H), 4.79 (s, 2H), 3.98 (s, 3H), 3.80 (s, 3H). |
| **4** | 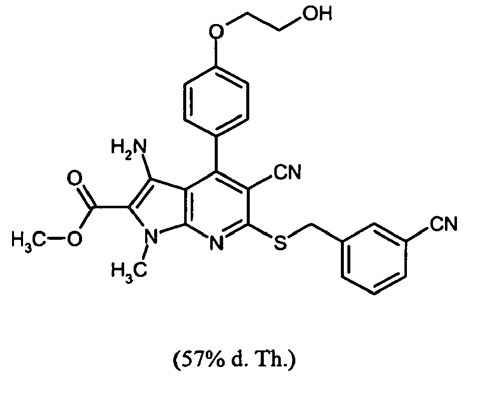 | 1.64 min (Methode 4); m/z = 514 | δ (400 MHz) = 8.02 (s, 1H), 7.88 (d, 1H), 7.73 (d, 1H), 7.56 (t, 1H), 7.48 (d, 2H), 7.16 (d, 2H), 5.01-4.82 (m, 3H), 4.68 (s, 2H), 4.09 (t, 2H), 3.95 (s, 3H), 3.80 (s, 3H), 3.78 (t, 2H). |
| **5** | 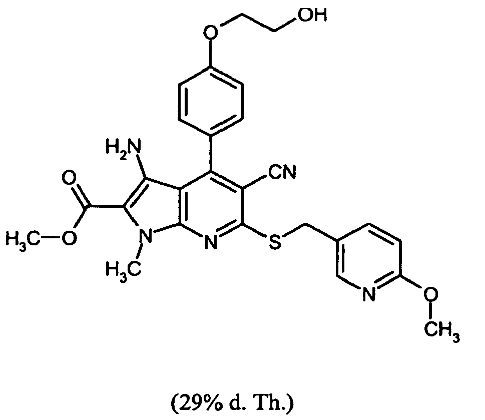 | 2.64 min (Methode 3); m/z = 520 | δ (400 MHz) = 8.30 (d, 1H), 7.84 (dd, 1H), 7.48 (d, 2H), 7.18 (d, 2H), 6.80 (d, 1H), 5.10-4.78 (m, 3H), 4.59 (s, 2H), 4.09 (t, 2H), 3.98 (s, 3H), 3.81 (s, 6H), 3.78 (t, 2H). |
| **6** | 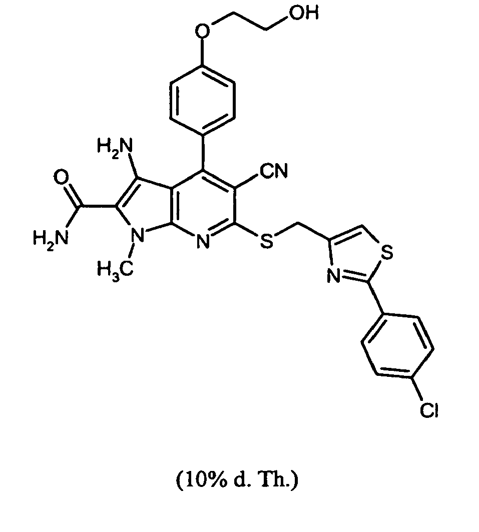 | 2.69 min (Methode 3); m/z = 591 | δ (400 MHz) = 7.97 (d, 2H), 7.71 (s, 1H), 7.58 (d, 2H), 7.48 (d, 2H), 7.32 (s, 2H), 7.14 (d, 2H), 4.79 (s, 2H), 4.55-4.28 (s, 2H), 4.09 (t, 2H), 3.92 (s, 3H), 3.76 (t, 2H). |
| **7** | 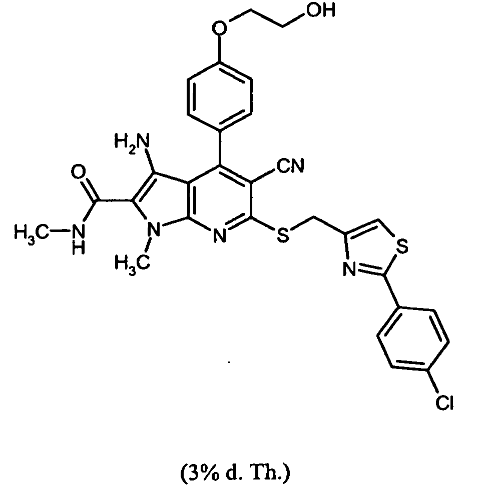 | 3.70 min (Methode 4); m/z = 605 | δ (400 MHz) = 8.02-7.92 (m, 3H), 7.70 (s, 1H), 7.59 (d, 2H), 7.50 (d, 2H), 7.11 (d, 2H), 5.05-4.80 (s, 1H), 4.61 (s, 2H), 4.39 (s, 2H), 4.09 (t, 2H), 3.76 (t, 2H) 3.42 (s, 3H), 2.60 (d, 3H). |
| **8** | 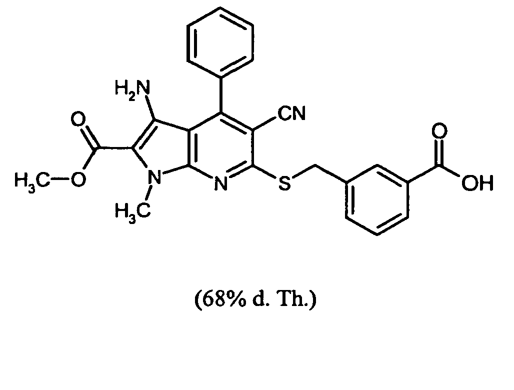 | 3.70min (Methode 7); m/z = 473 | δ (400 MHz)= 13.00 (s, 1H), 8.19 (s, 1H), 7.85-7.77 (m, 2H), 7.65-7.57 (m, 3H), 7.56-7.50 (m, 2H), 7.48 (t, 1H), 4.80 (s, 2H), 4.69 (s, 2H), 3.99 (s, 3H), 3.80 (s, 3H). |
| **9** | 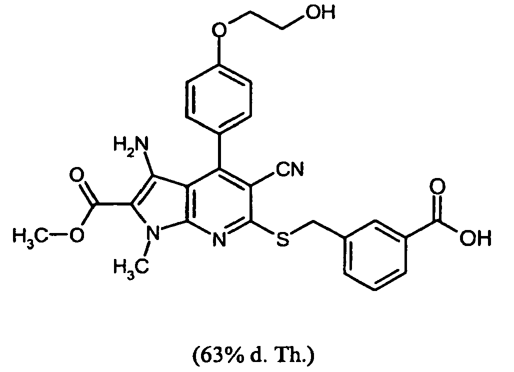 | 2.04 min (Methode 8); m/z = 533 | δ (400 MHz) = 12.98 (s, 1 H), 8.19 (s, 1H), 7.83-7.76 (m, 2H), 7.49-7.42 (m, 3H), 7.16 (d, 2H), 5.09-4.82 (m, 3H), 4.68 (s, 2H), 4.10 (t, 2H), 3.98 (s, 3H), 3.81 (s, 3H), 3.77 (t, 2H). |
| **10** | 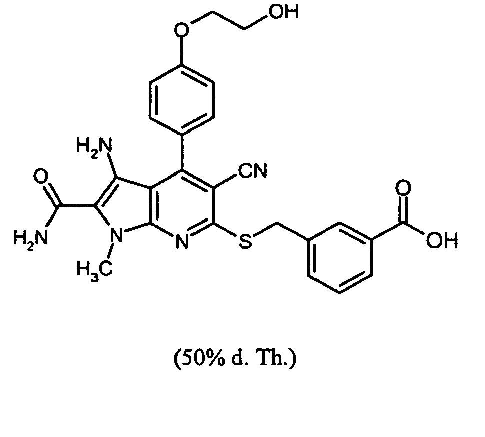 | 1.68 min (Methode 8);m/z= 518 | δ (400 MHz) = 12.98 (s, 1H), 12.70-9.10 (br s, 3H), 8.17 (s, 1H), 7.83-7.77 (m, 2H), 7.50-7.41 (m, 3H), 7.32 (s, 1H), 7.13 (d, 2H), 4.68 (s, 2H), 4.40 (s, 1H), 4.10 (t, 2H), 3.93 (s, 3H), 3.76 (t, 2H). |
| **11** | 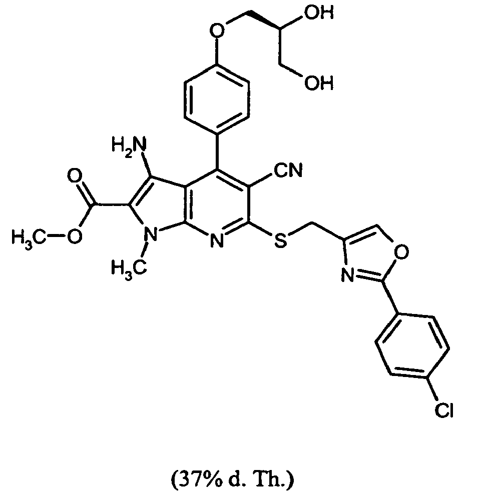 | 2.96 min (Methode 3); m/z = 620 | δ (400 MHz) = 8.21 (s, 1H), 7.98 (d, 2H), 7.60 (d, 2H), 7.47 (d, 2H), 7.18 (d, 2H), 5.03 (d, 1H), 4.92 (s, 2H), 4.71 (t, 1H), 4.59 (s, 2H), 4.11 (dd, 1H), 4.01-3.95 (m, 1H), 3.97 (s, 3H), 3.88-3.79 (m, 1H), 3.82 (s, 3H), 3.48 (t, 2H). |
| **12** | 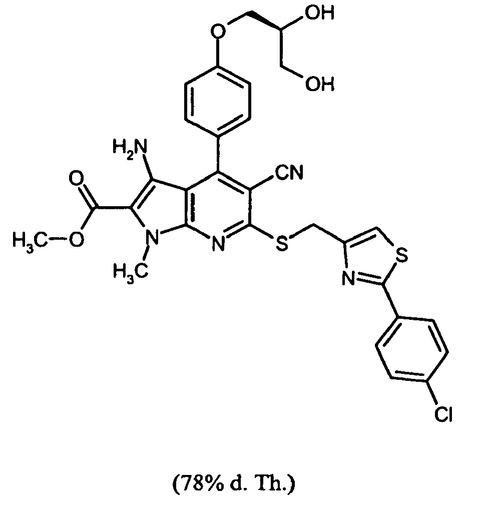 | 4.13 min (Methode 7); m/z = 636 | δ (400 MHz) = 7.96 (d, 2H), 7.73 (s, 1H), 7.57 (d, 2H), 7.47 (d, 2H), 7.16 (d, 2H), 5.10-4.97 (br s, 1H), 4.91 (br s, 1H), 4.78 (s, 2H), 4.10 (dd, 1H), 4.01-3.94 (m, 1H), 3.95 (s, 3H), 3.87-3.79 (m, 1H), 3.81 (s, 3H), 3.48 (d, 2H), NH2 fehlt. |
| **13** | 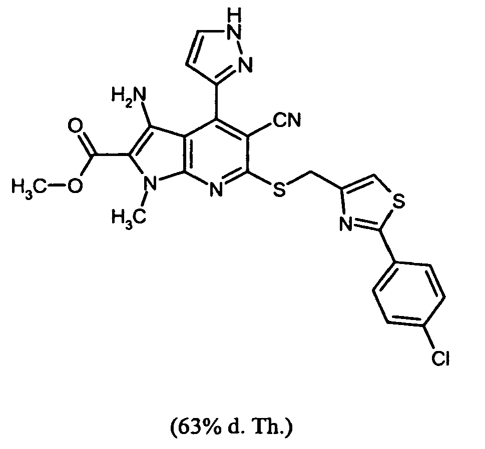 | 1.53 min (Methode 6); m/z = 536 | δ (400 MHz) = 13.79 (s, 1H), 8.11 (s, 1H), 7.96 (d, 2H), 7.72 (s, 1H), 7.57 (d, 2H), 6.98 (s, 1H), 6.42 (br s, 2H), 4.78 (s, 2H), 3.94 (s, 3H), 3.82 (s, 3H). |
| **14** | 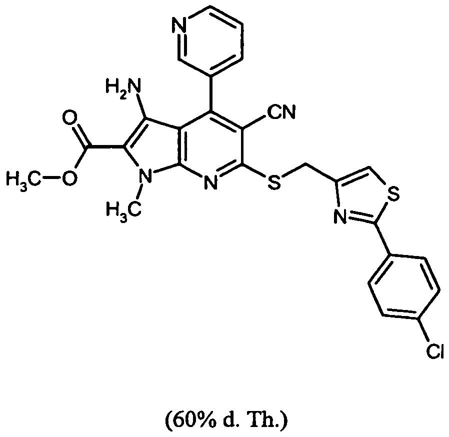 | 3.02 min (Methode 3); m/z = 547 | δ (400 MHz) = 8.80 (dd, 1H), 8.76 (d, 1H), 8.03 (d t, 1H), 7.95 (d, 2H), 7.74 (s, 1H), 7.63 (m, 1H), 7.58 (d, 2H), 4.90 (s, 2H), 4.79 (s, 2H), 3.98 (s, 3H), 3.82 (s, 3H). |

### Beispiel 15

### Methyl-3-amino-6-[(3-carbamoylbenzyl)thio]-5-cyano-4-[4-(2-hydroxyethoxy)phenyl]-1-methyl-1H-pyrrolo[2,3-b]pyridin-2-carboxylat

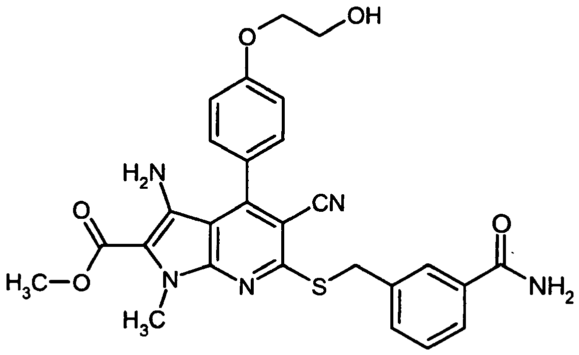

35 mg (0.066 mmol) der Verbindung aus Beispiel 9 wurden in 1.5 ml Acetonitril vorgelegt und mit 19 mg (0.1 mmol) 1-(3-Dimethylaminopropyl)-3-ethylcarbodiimidhydrochlorid und 13 mg (0.1 mmol) 1-Hydroxy-1H-benzotriazol-Hydrat versetzt. Nach 10 min bei RT wurden 18 mg (0.33 mmol) Ammoniumchlorid und 0.08 ml (0.46 mmol) N,N-Diisopropylethylamin zugegeben und der Ansatz wurde über Nacht bei RT gerührt.

Das Reaktionsgemisch wurde mit soviel Wasser versetzt bis eine klare Lösung entsteht. Diese wurde über eine präparative HPLC (Chromasil, Wasser/Acetonitril + 0.1% TFA) gereinigt.

Ausbeute: 34 mg (97 % d. Th.)

¹H-NMR (400 MHz, DMSO-d₆): δ = 8.09 (s, 1H), 7.98 (s, 1H), 7.76 (d, 1H), 7.68 (d, 1H), 7.49-7.32 (m, 4H), 7.15 (d, 2H), 4.90 (br s, 3H), 4.66 (s, 2H), 4.09 (t, 2H), 3.98 (s, 3H), 3.81 (s, 3H), 3.77 (t, 2H).

LC-MS (Methode 8): Rₜ = 1.84 min; MS (ESIpos): m/z = 532 [M+H]⁺.

### Beispiel 16

### 3-Amino-6-[(3-carbamoylbenzyl)thio]-5-cyano-4-[4-(2-hydroxyethoxy)phenyl]-1-methyl-1H-pyrrolo[2,3-b]pyridin-2-carboxamid

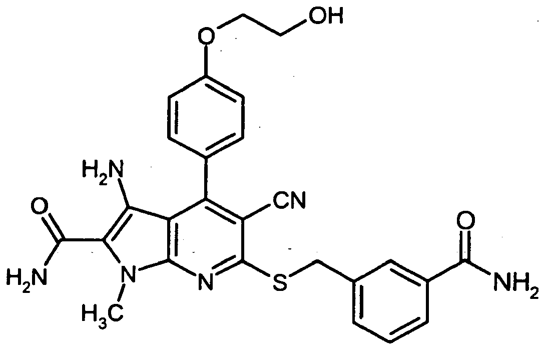

Die Zielverbindung wurde in analoger Weise zu Beispiel 15 aus Beispiel 10 hergestellt.

Ausbeute: 2 mg (5 % d. Th.)

¹H-NMR (400 MHz, DMSO-d₆): δ = 8.18 (br s, 2H), 8.08 (s, 1H), 7.97 (s, 1H), 7.77 (d, 1H), 7.68 (d, 1H), 7.48-7.30 (m, 4H), 7.12 (d, 2H), 5.40-4.60 (m, 4H), 4.40 (br s, 1H), 4.09 (t, 2H), 3.93 (s, 3H), 3.77 (t, 2H).

LC-MS (Methode 3): Rₜ = 1.92 min; MS (ESIpos): m/z = 517 [M+H]⁺.

### Beispiel 17

### Methyl-3-amino-6-({[2-(4-chlorphenyl)-1,3-thiazol-4-yl]methyl}thio)-5-cyano-4-[4-(2-hydroxyethoxy)phenyl]-1H-pyrrolo[2,3-b]pyridin-2-carboxylat

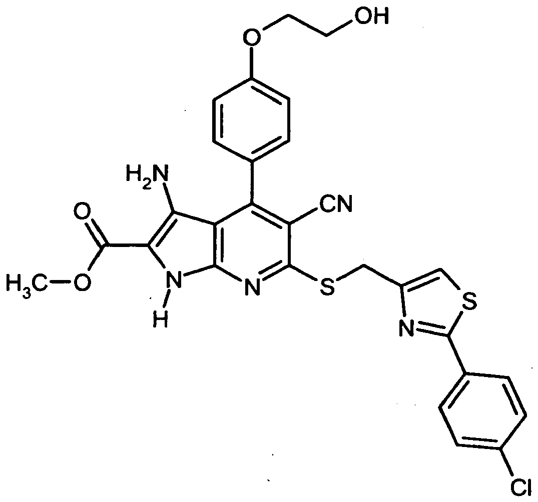

90 mg (0.114 mmol) der Verbindung aus Beispiel 30A wurden in 5 ml Acetonitril vorgelegt und mit 148 mg (0.454 mmol) Cäsiumcarbonat versetzt. Das Reaktionsgemisch wurde über Nacht bei 50°C gerührt und anschließend nochmals mit 74 mg (0.23 mmol) Cäsiumcarbonat versetzt. Nach Rühren über Nacht bei 50°C wurde vom Feststoff abfiltriert und mit Acetonitril gewaschen. Das Filtrat wurde eingeengt und der Rückstand (ca. 200 mg) ohne weitere Reinigung weiter umgesetzt.

Dieser Rückstand wurde in 2 ml Dichlormethan vorgelegt und mit 2 ml einer 4M Chlorwasserstoff-Lösung in 1,4-Dioxan versetzt und 2.5h bei RT gerührt. Das Reaktionsgemisch wurde eingedampft und der Rückstand wurde über präparative HPLC (Chromasil, Wasser/Acetonitril + 0.15% konz. Salzsäure) gereinigt.

Ausbeute: 5 mg (7 % d. Th.)

¹H-NMR (400 MHz, DMSO-d₆): δ = 12.05 (s, 1H), 8.00 (s, 1H), 7.95 (d, 2H), 7.58 (d, 2H), 7.48 (d, 2H), 7.15 (d, 2H), 4.91 (br s, 1H), 4.74 (s, 2H), 4.70 (s, 2H), 4.09 (t, 2H), 3.81 (s, 3H), 3.77 (t, 2H).

LC-MS (Methode 4): Rₜ = 4.00 min; MS (ESIpos): m/z = 592 [M+H]⁺.

### Beispiel 18

### Methyl-3-amino-6-({[2-(4-chlorphenyl)-1,3-thiazol-4-yl]methyl}sulfanyl)-5-cyano-1-methyl-4-piperidin-1-yl-1H-pyrrolo[2,3-b]pyridin-2-carboxylat

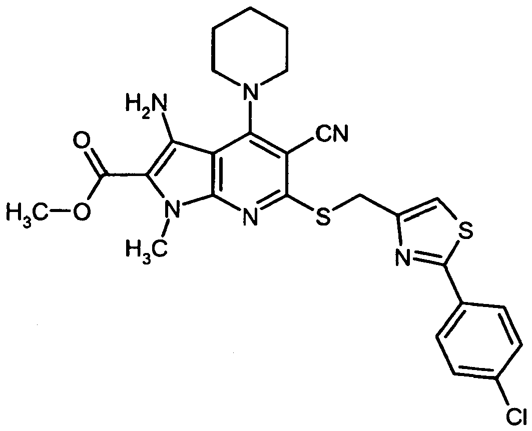

80 mg (0.15 mmol) der Verbindung aus Beispiel 47A wurden in 3 ml Acetonitril vorgelegt und mit 189 mg (0.58 mmol) Cäsiumcarbonat versetzt. Es wurde 4h bei 50°C gerührt. Nach dem Abkühlen wurde filtriert und das Filtrat am Rotationsverdampfer vom Lösungsmittel befreit. Der Rückstand wurde im Vakuum getrocknet.

Ausbeute: 68 mg (85 % d. Th.)

¹H-NMR (400 MHz, CDCl₃): δ = 7.95 (d, 2H), 7.68 (s, 1H), 7.57 (d, 2H), 5.71 (s, 2H), 4.68 (s, 2H), 3.88 (s, 3H), 3.83 (s, 3H), 3.48-3.42 (m, 4H), 1.76-1.68 (br s, 4H), 1.65-1.57 (m, 2H).

LC-MS (Methode 4): Rₜ = 5.00 min; MS (ESIpos): m/z = 554 [M+H]⁺.

### Beispiel 19

### Methyl-3-amino-6- {[2-(4-chlorphenyl)-1,3-oxazol-4-yl]methoxy} -5-cyano-4-[4-(2-hydroxyethoxy)phenyl]-1-methyl-1H-pyrrolo[2,3-b]pyridin-2-carboxylat

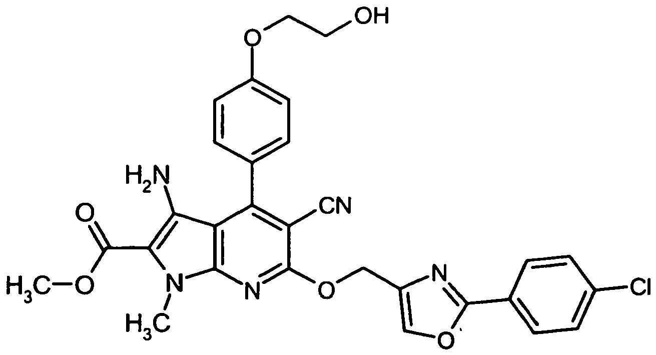

20 mg (0.04 mmol) der Verbindung aus Beispiel 51A wurden in 1 ml Acetonitril vorgelegt und mit 45 mg (0.14 mmol) Cäsiumcarbonat versetzt. Das Reaktionsgemisch wurde 4h bei 50°C gerührt. Nach Abkühlen auf RT wurde abfiltriert. Der Niederschlag wurde mit Acetonitril gewaschen und in Wasser suspendiert. Es wurde dreimal mit je 10 ml Essigsäureethylester extrahiert und die vereinigten organischen Phasen über Magnesiumsulfat getrocknet. Nach Entfernen des Lösungsmittels am Rotationsverdampfer wurde der Rückstand direkt mittels präparativer HPLC (Säule: YMC GEL ODS-AQ S-5 / 15 µm; Laufmittelgradient: Acetonitril/Wasser 10:90 → 95:5) gereinigt. Nach Entfernen des Lösungsmittels am Rotationsverdampfer erhielt man das Produkt als Feststoff.

Ausbeute: 10 mg (48 % d. Th.).

¹H-NMR (400 MHz, DMSO-d₆): δ = 8.41 (s, 1H), 8.02 (d, 2H), 7.62 (d, 2H), 7.48 (d, 2H), 7.17 (d, 2H), 5.56 (s, 2H), 4.96-4.90 (m, 3H), 4.09 (t, 2H), 3.91 (s, 3H), 3.79 (s, 3H), 3.78 (q, 2H).

LC-MS (Methode 6): Rₜ = 1.44 min; MS (ESIpos): m/z = 574 [M+H]⁺.

### Beispiel 20

### Methyl-3-amino-6-({[2-(4-chlorphenyl)-1,3-thiazol-4-yl]methyl}thio)-5-cyano-4-[4-(2-hydroxyethoxy)phenyl]-1-(2-methoxy-2-oxoethyl)-1H-pyrrolo[2,3-b]pyridin-2-carboxylat

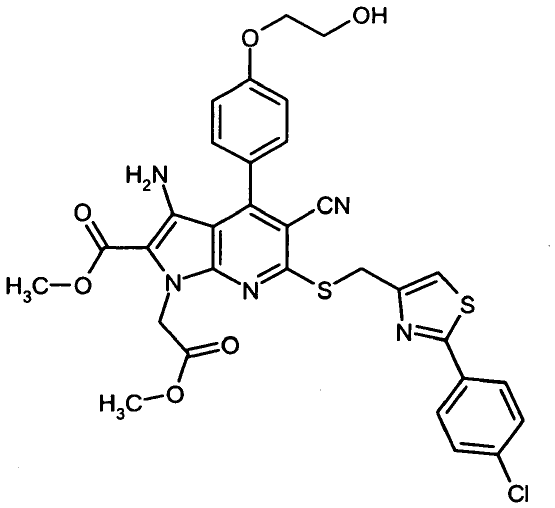

19 mg (0.024 mmol) der Verbindung aus Beispiel 33A wurden in 1 ml THF vorgelegt und mit 0.122 ml (0.122 mmol) einer 1N Tetrabutylammoniumfluorid-Lösung in THF versetzt. Das Reaktionsgemisch wurde 2h bei RT gerührt und anschließend mit Essigsäureethylester verdünnt. Es wurde mit gesättigter wässriger Natriumhydrogencarbonat-Lösung gewaschen. Die organische Phase wurde über Natriumsulfat getrocknet, eingeengt und der Rückstand wurde über eine präparative HPLC gereinigt (Chromasil, Wasser/Acetonitril). Es wurden 15 mg des Produktes erhalten, die über präparative Dünnschichtchromatographie (Dichlormethan/Methanol = 20/1) gereinigt wurden.

Ausbeute: 7 mg (43% d. Th.)

¹H-NMR (500 MHz, DMSO-d₆): δ = 7.96 (d, 2H), 7.64 (s, 1H), 7.58 (d, 2H), 7.51 (d, 2H), 7.19 (d, 2H), 5.31 (s, 2H), 4.98-4.89 (m, 3H), 4.73 (s, 2H), 4.12-4.05 (m, 2H), 3.79-3.70 (m, 5H), 3.62 (s, 3H).

LC-MS (Methode 2): Rₜ = 2.83 min; MS (ESIpos): m/z = 664 [M+H]⁺.

### Beispiel 21

### 3-Amino-6-({[2-(4-chlorphenyl)-1,3-thiazol-4-yl]methyl}sulfanyl)-4-[4-(2-hydroxyethoxy)phenyl]-1H-pyrazolo[3,4-b]pyridin-5-carbonitril

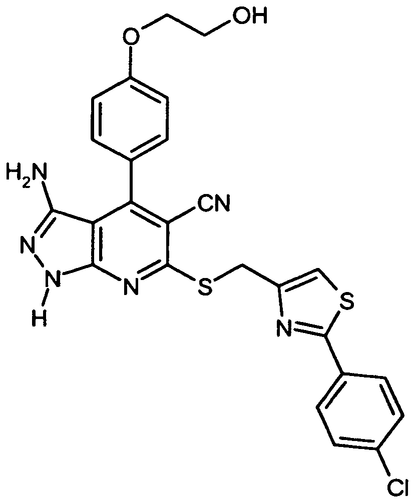

100 mg (0.165 mmol) der Verbindung aus Beispiel 16A wurden in 3.2 ml N-Methylpyrrolidon gelöst und mit 0.20 ml (0.412 mmol) Hydrazinhydrat 1.5h bei RT gerührt. Es wurde mit sehr wenig Acetonitril, THF und Wasser verdünnt und über eine präparative HPLC (Chromasil, Wasser/Acetonitril + 0.1% TFA) gereinigt.

Ausbeute: 30 mg (34 % d. Th.)

¹H-NMR (400 MHz, DMSO-d₆): δ = 12.81 (br s, 1H), 7.98 (d, 2H), 7.76 (s, 1H), 7.58 (d, 2H), 7.51 (d, 2H), 7.18 (d, 2H), 5.30-4.48 (m, 5H), 4.10 (t, 2H), 3.76 (t, 2H).

LC-MS (Methode 3): Rₜ = 2.522 min; MS (ESIpos): m/z = 535 [M+H]⁺.

### Beispiel 22

### 3-Amino-6-({[2-(4-chlorphenyl)-1,3-thiazol-4-yl]methyl}sulfanyl)-4-phenyl-1H-pyrazolo[3,4-b]pyridin-5-carbonitril

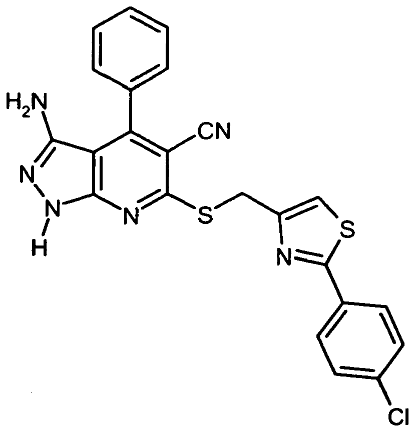

Die Zielverbindung wurde in analoger Weise wie Beispiel 21 aus Beispiel 14A hergestellt.

Ausbeute: 16 mg (20 % d. Th.)

¹H-NMR (400 MHz, DMSO-d₆): δ = 12.88 (br s, 1H), 7.98 (d, 2H), 7.78 (s, 1H), 7.65-7.55 (m, 7H), 5.32-4.42 (m, 4H).

LC-MS (Methode 3): Rₜ = 2.77 min; MS (ESIpos): m/z = 475 [M+H]⁺.

### Beispiel 23

### 6-({[2-(4-Chlorphenyl)-1,3-thiazol-4-yl]methyl}sulfanyl)-4-[4-(2-hydroxyethoxy)phenyl]-1H-pyrazolo[3,4-b]pyridin-5-carbonitril

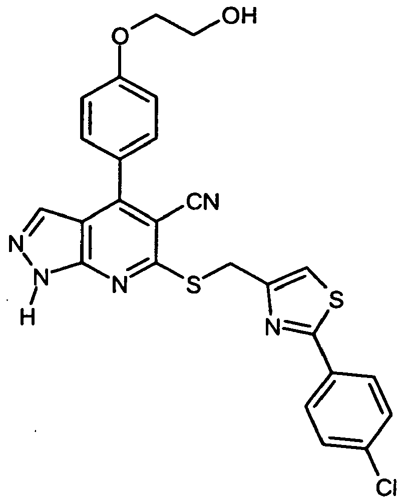

280 mg (0.359 mmol) der Verbindung aus Beispiel 21 wurden in 3 ml Wasser und 6 ml Eisessig vorgelegt, auf 0°C abgekühlt und mit einer Lösung aus 50 mg Natriumnitrit in 3 ml Wasser versetzt. Das Reaktionsgemisch wurde über Nacht bei RT gerührt. Anschließend wurde auf 0°C abgekühlt und vom Niederschlag abfiltriert. Dieser wurde mit eiskaltem Wasser gewaschen und mit 9 ml 1,2-Dimethoxyethan und 12 ml 0.1 N Salzsäure-Lösung versetzt. Das Reaktionsgemisch wurde 1.5 h bei 80°C gerührt und anschließend abgekühlt. Es wurde mit so wenig THF verdünnt, so dass eine klare Lösung entsteht. Diese wurde portionsweise über eine präparative HPLC (Chromasil, Wasser/Acetonitril + 0.1% TFA) gereinigt.

Ausbeute: 102 mg (55 % d. Th.)

¹H-NMR (400 MHz, DMSO-d₆): δ = 14.18 (s, 1H), 8.11 (s, 1H), 7.97 (d, 2H), 7.78 (s, 1H), 7.74 (d, 2H), 7.58 (d, 2H), 7.19 (d, 2H), 4.99-4.87 (m, 1H), 4.78 (s, 2H), 4.11 (t, 2H), 3.80-3.72 (m, 2H).

LC-MS (Methode 4): Rₜ = 2.58 min; MS (ESIpos): m/z = 520 [M+H]⁺.

### B. Bewertung der pharmakologischen und physiologischen Wirksamkeit

Die pharmakologische und physiologische Wirkung der erfindungsgemäßen Verbindungen kann in folgenden Assays gezeigt werden:

### B-1. Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:
Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 5 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den A1-Zellen zugegeben und anschließend werden alle Kulturen für vier Stunden bei 37°C inkubiert. Danach wird zu den Testkulturen 35 µl einer Lösung, bestehend zu 50% aus Lyse-Reagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der A1-Zelle 50% der Luciferase-Antwort inhibiert bzw. bei den A2b- und A2a-Zellen 50% der maximalen Stimulierbarkeit mit der entsprechenden Substanz erreicht sind. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol. 357, 1-9 (1998)].

In der folgenden Tabelle 1 sind die EC₅₀-Werte repräsentativer Ausführungsbeispiele für die Rezeptorstimulation an Adenosin A1-, A2a- und A2b-Rezeptor-Subtypen aufgeführt:

**Tabelle 1**

| **Beispiel Nr.** | **EC50 A1 [nM] (1µM Forskolin)** | **EC50 A2a [nM]** | **EC50 A2b [nM]** |
|---|---|---|---|
| **1** | 0.8 | 170 | 550 |
| **2** | 1.7 | 150 | 1540 |
| **3** | 5.3 | >3000 | >3000 |
| **4** | 3.0 | 73 | 320 |
| **5** | 1.5 | 200 | 1000 |
| **6** | 0.3 | 1120 | >3000 |
| **7** | 5.0 | 350 | 660 |
| **11** | 14 | 600 | >3000 |
| **12** | 11 | 1600 | >3000 |
| **15** | 3.7 | 440 | 1330 |
| **16** | 4.9 | 330 | 760 |
| **17** | 3.7 | 680 | >3000 |
| **21** | 10 | >3000 | >3000 |

### B-2. Untersuchung an isolierten Gefäßen

Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.

### B-3. Blutdruck- und Herzfrequenz-Messungen an wachen Ratten

Wachen SHR (spontaneously hypertensive rats)-Ratten, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend werden über 24 Stunden Blutdruck und Herzfrequenz und deren Veränderungen aufgezeichnet.

### B-4. Blutdruck- und Herzfreguenz-Messunpen an wachen Krallenaffen

Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend werden über 6-24 Stunden Blutdruck und Herzfrequenz und deren Veränderungen aufgezeichnet.

### B-5. Indirekte Bestimmung des Adenosin-Antagonismus über Genexpression

Zellen der permanenten Linie CHO K1 (Chinese Hamster Ovary) werden stabil mit einem Reporterkonstrukt (CRE-Luciferase) und der cDNA für die Adenosin-Rezeptorsubtypen A2a oder A2b transfiziert. A2a- bzw. A2b-Rezeptoren sind über Gas-Proteine an die Adenylatcyclase gekoppelt. Durch Rezeptoraktivierung wird die Adenylatcyclase aktiviert und damit das cAMP-Level in der Zelle erhöht. Über das Reporterkonstrukt, einem cAMP-abhängigen Promotor, ist die Änderung des cAMP-Levels an die Luciferase-Expression gekoppelt.

Für die Bestimmung von Adenosin-Antagonismus am Adenosin-Rezeptorsubtyp A1 werden ebenfalls CHO K1-Zellen stabil transfiziert, diesmal allerdings mit einem Ca²⁺-sensitiven Reporterkonstrukt (NFAT-TA-Luc; Clontech) und einem A1-Gα16 Fusionskonstrukt. Diese Rezeptorchimäre ist im Gegensatz zum nativen A1-Rezeptor (Gai-Kopplung) an die Phospholipase C gekoppelt. Die Luciferase wird hier in Abhängigkeit von der cytosolischen Ca²⁺-Konzentration exprimiert.

Die permanenten Zelllinien werden in DMEM/F12 (Cat.-No BE04-687Q; BioWhittaker) mit 10% FCS (Fetales Kälberserum) und diversen Zusätzen (20 ml/Liter 1M HEPES (Cat.-No 15630; Gibco), 20 ml/Liter GlutaMAX (Cat.-No 35050-038, Gibco), 14 ml/Liter MEM Natriumpyruvat (Cat.-No 11360-039; Gibco), 10 ml/Liter PenStrep (Cat.-No 15070-063; Gibco)) bei 37°C unter 5% Kohlendioxid kultiviert und zweimal pro Woche gesplittet.

Für die Testung im 384-well Plattenformat werden die Zellen mit 2000 Zellen/Well in 25µl/Well Aussaatmedium ausgesät und bis zur Substanztestung bei 37°C unter 5% Kohlendioxid kultiviert. Die A2a- und A2b-Zellen werden 24 h vor der Substanztestung in Medium mit Zusätzen und 5% FCS ausgesät, wobei für die A2a-Zellen als Basismedium DMEM/F12 und für die A2b-Zellen OptiMEM (Cat.-No 31985-047; Gibco) verwendet wird. Die A1-Gα16-Zellen werden 48 h vor Substanztestung in OptiMEM mit 2.5% dialysiertem FCS und Zusätzen ausgesät. Am Testtag wird vor der Substanzzugabe das Medium durch 25 µl Cafty-Puffer (Cat.-No T21-154; PAA) mit 2 mM Calciumchlorid und 0.1% BSA (Rinderserumalbumin) ersetzt. Von den zu testenden, in DMSO gelösten Substanzen werden Verdünnungsreihen in Cafty-Puffer mit 2 mM Calciumchlorid und 0.1% BSA (Rinderserumalbumin) und einer geeigneten Konzentration des Agonisten angesetzt. Die Substanzen werden in einer Endkonzentration von 5 x 10⁻⁵ M bis 2.56 x 10⁻¹¹ M zu den Testkulturen pipettiert, wobei der DMSO-Gehalt auf den Zellen 0.5% nicht überschreiten darf. Als Agonist wird für die A2a- und A2b-Zellen NECA (5-N-Ethylcarboxamido-adenosin) in einer Endkonzentration von 30 nM, was etwa der EC₅₀-Konzentration entspricht, eingesetzt. Für die A1-Gα16-Zellen wird 25 nM CPA (N6-cyclopentyl-adenosine) als Agonist eingesetzt, was etwa der EC₇₅-Konzentration entspricht. Nach der Substanzzugabe werden die Zellplatten 3-4 h bei 37°C unter 5% Kohlendioxid inkubiert. Anschließend werden die Zellen direkt vor der Messung mit 25 µl einer Lösung, bestehend zu 50% aus Lyse-Reagenz (30 mM Dinatriumhydrogenphosphat, 10% Glycerin, 3% Triton X-100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) versetzt. Die Luciferase-Aktivität wird mit einem Lumineszenz-Reader detektiert. Bestimmt werden die IC₅₀-Werte, d.h. die Konzentration, bei denen die Luciferase-Antwort, hervorgerufen durch den jeweiligen Agonisten zu 50% inhibiert wird. Als Referenz-Antagonist wird für die A2a- und A2b-Zellen ZM241385 und für die A1-Ga16-Zellen DPCPX (1,3-dipropyl-8-cyclopentylxanthine) verwendet.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel® (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 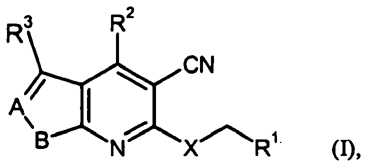 in welcher
entweder
A für CR⁴ oder N steht,
wobei
R⁴ für (C₁-C₄)-Alkoxycarbonyl, Aminocarbonyl, Mono-(C₁-C₄)-alkylaminocarbonyl oder Di-(C₁-C₄)-alkylaminocarbonyl steht,
und
B für NR⁵ steht,
wobei
R⁵ für Wasserstoff oder (C₁-C₄)-Alkyl steht, worin (C₁-C₄)-Alkyl mit einem Substituenten (C₁-C₄)-Alkoxycarbonyl substituiert sein kann,
X für O oder S steht,
R¹ für (C₆-C₁₀)-Aryl oder 5- bis 10-gliedriges Heteroaryl steht,
wobei (C₆-C₁₀)-Aryl und 5- bis 10-gliedriges Heteroaryl mit 1 oder 2 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Hydroxycarbonyl, (C₁-C₆)-Alkoxycarbonyl, Aminocarbonyl, Mono-(C₁-C₆)-alkylaminocarbonyl, Di-(C₁-C₆)-alkylamino-carbonyl, Pyrrolidino, Piperidino, Morpholino, Piperazino und *N*'-(C₁-C₄)- Alkylpiperazino, Phenyl und 5- oder 6-gliedriges Heteroaryl, substituiert sein können, worin Phenyl und 5- oder 6-gliedriges Heteroaryl mit 1 bis 3 Substituenten unabhängig voneinander ausgewählt aus der Gruppe Halogen, Nitro, Cyano, (C₁-C₆)-Alkyl, Difluormethyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, Hydroxycarbonyl und (C₁-C₆)-Alkoxy-carbonyl substituiert sein können,
R² für (C₅-C₆)-Cycloalkyl, 5- oder 6-gliedriges Heterocyclyl, Phenyl oder 5- oder 6-gliedriges Heteroaryl steht,
wobei (C₅-C₆)-Cycloalkyl mit 1 oder 2 Substituenten unabhängig voneinander ausgewählt aus der Gruppe (C₁-C₆)-Alkyl, Hydroxy, Oxo, (C₁-C₆)-Alkoxy, Amino, Mono-(C₁-C₆)-alkylamino und Di-(C₁-C₆)-alkylamino substituiert sein kann,
worin (C₁-C₆)-Alkyl und (C₁-C₆)-Alkoxy mit 1 oder 2 Substituenten unabhängig voneinander ausgewählt aus der Gruppe Hydroxy, (C₁-C₄)-Alkoxy und (C₃-C₇)-Cycloalkyl substituiert sein können,
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
wobei 5- oder 6-gliedriges Heterocyclyl mit 1 bis 3 Substituenten unabhängig voneinander ausgewählt aus der Gruppe Oxo, Thioxo, Hydroxy, (C₁-C₆)-Alkyl, (C₁-C₆)-Alkoxy, (C₁-C₆)-Alkylcarbonyl, Amino, Mono-(C₁-C₆)-alkylamino, Di-(C₁-C₆)-alkylamino und (C₃-C₇)-Cycloalkyl substituiert sein kann,
worin (C₁-C₆)-Alkyl mit 1 bis 3 Substituenten unabhängig voneinander ausgewählt aus der Gruppe Fluor, Oxo, Hydroxy, Trifluormethyl, (C₁-C₄)-Alkoxy, (C₁-C₄)-Alkylcarbonyloxy, Amino, Mono-(C₁-C₄)-alkylamino, Di-(C,-C₄)-alkylamino und (C₃-C₇)-Cycloalkyl substituiert sein kann,
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
worin (C₁-C₆)-Alkylcarbonyl mit einem Substituenten ausgewählt aus der Gruppe Hydroxy und (C₁-C₄)-Alkoxy substituiert sein kann,
und
worin (C₃-C₇)-cycloalkyl mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
wobei Phenyl und 5- oder 6-gliedriges Heteroaryl mit 1 bis 3 Substituenten unabhängig voneinander ausgewählt aus der Gruppe Halogen, Cyano, Hydroxy, (C₁-C₆)-Alkyl, (C₁-C₆)-Alkoxy, (C₃-C₇)-Cycloalkoxy und -NR^{A}R^{B} substituiert sein können,
worin (C₁-C₆)-Alkyl mit 1 bis 3 Substituenten ausgewählt aus der Gruppe Fluor substituiert sein kann,
und
worin (C₁-C₆)-Alkoxy mit 1 bis 3 Substituenten unabhängig voneinander ausgewählt aus der Gruppe Fluor, Trifluormethyl, (C₃-C₇)-Cycloalkyl, Oxo, Hydroxy, (C₁-C₄)-Alkoxy, Hydroxycarbonyl, Amino, Mono-(C₁-C₄)-alkylamino und Di-(C₁-C₄)-alkylamino substituiert sein kann,
und
worin (C₃-C₇)-Cycloalkoxy mit 1 oder 2 Substituenten unabhängig voneinander ausgewählt aus der Gruppe (C₁-C₄)-Alkyl, Hydroxy, Oxo und (C₁-C₄)-Alkoxy substituiert sein können,
und
worin
R^{A} für Wasserstoff oder (C₁-C₆)-Alkyl steht, worin (C₁-C₆)-Alkyl seinerseits mit einem Substituenten ausgewählt aus der Gruppe Hydroxy und (C₁-C₄)-Alkoxy substituiert sein kann,
R^{B} für Wasserstoff, (C₁-C₆)-Alkyl, (C₃-C₇)-Cycloalkyl, (C₁-C₄)-Alkylsulfonyl oder (C₃-C₇)-Cycloalkylsulfonyl steht,
worin (C₁-C₆)-Alkyl seinerseits mit 1 oder 2 Substituenten unabhängig voneinander ausgewählt aus der Gruppe (C₃-C₇)-Cycloalkyl, Oxo, Hydroxy, (C₁-C₄)-Alkoxy, Hydroxycarbonyl, Amino, Mono-(C₁-C₄)-alkylamino und Di-(C₁-C₄)-alkylamino substituiert sein kann,
und
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
oder
worin zwei benachbarte Substituenten am Phenyl zusammen mit den Kohlenstoffatomen, an die sie gebunden sind, ein 1,3-Dioxolan oder 2,2-Difluor-1,3-dioxolan bilden können,
R³ für Wasserstoff, (C₁-C₄)-Alkyl, (C₁-C₄)-Alkoxy, Amino, Mono-(C₁-C₄)-alkylamino,-Di-(C₁-C₄)-alkylamino oder (C₁-C₄)-Alkylcarbonyl steht
sowie ihre *N*-Oxide, Salze, Solvate, Salze der *N*-Oxide und Solvate der *N*-Oxide.

2. Verbindung der Formel (I) nach Anspruch 1, in welcher
A für CR⁴ oder N steht,
wobei
R⁴ für (C₁-C₄)-Alkoxycarbonyl, Aminocarbonyl oder Mono-(C₁-C₄)-alkylaminocarbonyl steht,
B für NR⁵ steht,
wobei
R⁵ ür Wasserstoff, Methyl oder Ethyl steht,
worin Methyl und Ethyl mit einem Substituenten ausgewählt aus der Gruppe Methoxycarbonyl und Ethoxycarbonyl substituiert sein können,
X für O oder S steht,
R¹ für Phenyl oder 5- oder 6-gliedriges Heteroaryl steht,
wobei Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, Hydroxy, (C₁-C₄)-Alkoxy, Amino, Hydroxycarbonyl, (C₁-C₄)-Alkoxycarbonyl, Aminocarbonyl, Phenyl und 5- oder 6-gliedriges Heteroaryl substituiert sein können,
worin Phenyl und 5- oder 6-gliedriges Heteroaryl mit 1 bis 3 Substituenten unabhängig voneinander ausgewählt aus der Gruppe Fluor, Chlor, Nitro, Cyano, (C₁-C₄)-Alkyl, Difluormethyl, Trifluormethyl, Hydroxy, Methoxy, Ethoxy, Amino, Hydroxycarbonyl und (C₁-C₄)-Alkoxycarbonyl substituiert sein können,
R² für Cyclohexyl, Tetrahydropyranyl, Piperidinyl, Piperazinyl, Morpholinyl, Phenyl, Pyrazolyl, Imidazolyl, Oxazolyl, Thiazolyl oder Pyridyl steht,
wobei Cyclohexyl mit einem Substituenten ausgewählt aus der Gruppe Hydroxy und (C₁-C₄)-Alkoxy substituiert sein kann,
worin (C₂-C₄)-Alkoxy mit 1 oder 2 Substituenten unabhängig voneinander ausgewählt aus der Gruppe Hydroxy und Methoxy substituiert sein kann,
und wobei Piperidinyl, Piperazinyl und Morpholinyl mit einem Substituenten ausgewählt aus der Gruppe (C₁-C₄)-Alkyl, Hydroxy, (C₁-C₄)-Alkoxy und (C₁-C₄)-Alkylcarbonyl substituiert sein können,
worin (C₁-C₄)-Alkyl mit 1 oder 2 Substituenten unabhängig voneinander ausgewählt aus der Gruppe Hydroxy, Methoxy, Ethoxy, Methylcarbonyloxy und Ethylcarbonyloxy substituiert sein kann,
und
worin (C₁-C₄)-Alkylcarbonyl mit einem Substituenten ausgewählt aus der Gruppe Hydroxy, Methoxy und Ethoxy substituiert sein kann,
und
wobei Phenyl und Pyridyl mit 1 bis 3 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus oder Gruppe Oxo, Hydroxy, (C₁-C₄)-Alkoxy, Hydroxycarbonyl und Amino substituiert sein kann,
und
wobei Pyrazolyl, Imidazolyl, Oxazolyl und Thiazolyl mit 1 oder 2 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
R³ für Wasserstoff, Amino, Methylamino oder Dimethylamino steht,
sowie ihre Salze, Solvate und Solvate der Salze.

3. Verbindung der Formel (I) nach Anspruch 1, in welcher
A für CR⁴ oder N steht,
wobei
R⁴ für (C₁-C₄)-Alkoxycarbonyl, Aminocarbonyl oder Mono-(C₁-C₄)-alkylaminocarbonyl steht,
und
B für NR⁵ steht,
wobei
R⁵ für Wasserstoff, Methyl oder Ethyl steht,
worin Methyl und Ethyl mit einem Substituenten ausgewählt aus der Gruppe Methoxycarbonyl und Ethoxycarbonyl substituiert sein können,
X für O oder S steht,
R¹ für Phenyl oder 5- oder 6-gliedriges Heteroaryl steht,
wobei Phenyl und 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, Hydroxy, (C₁-C₄)-Alkoxy, Amino, Hydroxycarbonyl, (C₁-C₄)-Alkoxycarbonyl, Aminocarbonyl, Phenyl und 5- oder 6-gliedriges Heteroaryl substituiert sein können,
worin Phenyl und 5- oder 6-gliedriges Heteroaryl mit 1 bis 3 Substituenten unabhängig voneinander ausgewählt aus der Gruppe Fluor, Chlor, Nitro, Cyano, (C₁-C₄)-Alkyl, Difluormethyl, Trifluormethyl, Hydroxy, Methoxy, Ethoxy, Amino, Hydroxycarbonyl und (C₁-C₄)-Alkoxycarbonyl substituiert sein können,
R² für eine Gruppe der Formel 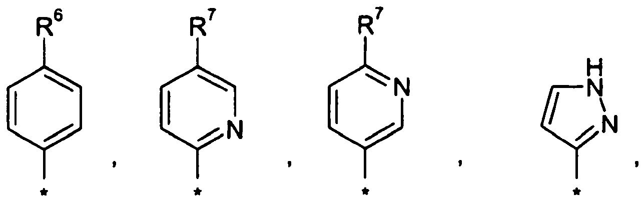 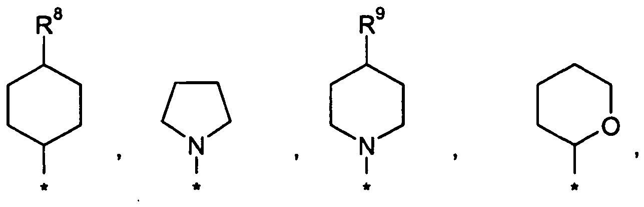 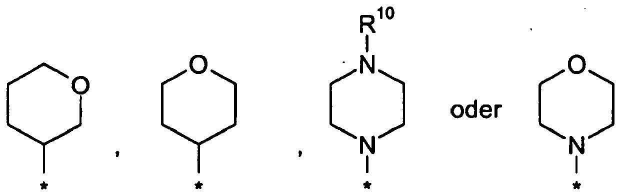 steht, wobei
* die Anknüpfstelle an den Bicyclus bedeutet,
R⁶ für Wasserstoff oder (C₁-C₄)-Alkoxy steht,
worin (C₂-C₄)-Alkoxy mit 1 oder 2 Substituenten Hydroxy substituiert sein kann,
R⁷ für Wasserstoff oder (C₁-C₄)-Alkoxy steht,
worin (C₂-C₄)-Alkoxy mit 1 oder 2 Substituenten Hydroxy substituiert sein kann,
R⁸ für Wasserstoff, Hydroxy, Methoxy, Ethoxy oder 2-Hydroxyethoxy steht,
R⁹ für Wasserstoff oder Hydroxy steht,
und
R¹⁰ für Wasserstoff oder Methyl steht,
R³ für Wasserstoff, Amino, Methylamino oder Dimethylamino steht,
sowie ihre Salze, Solvate und Solvate der Salze.

4. Verbindung der Formel (I) nach Anspruch 1, 2 oder 3, in welcher
A für CR⁴ oder N steht,
wobei
R⁴ für Methoxycarbonyl, Aminocarbonyl oder Methylaminocarbonyl steht,
und
B für NR⁵ steht,
wobei
R⁵ für Wasserstoff oder Methyl steht,
worin Methyl mit einem Substituenten Methoxycarbonyl substituiert sein kann,
X für O oder S steht
R¹ für Thiazolyl, Oxazolyl, Phenyl oder Pyridyl steht,
wobei Phenyl und Pyridyl mit 1 oder 2 Substituenten unabhängig voneinander ausgewählt aus der Gruppe Fluor, Chlor, Cyano, Methyl, Ethyl, Methoxy, Amino, Hydroxycarbonyl, Methoxycarbonyl, Ethoxycarbonyl und Aminocarbonyl substituiert sein können,
und
wobei Thiazolyl und Oxazolyl mit einem Substituenten Gruppe Phenyl substituiert sind,
worin Phenyl mit einem Substituenten ausgewählt aus der Gruppe Fluor, Chlor, Cyano, Methyl, Methoxy und Hydroxycarbonyl substituiert sein kann,
und
wobei Thiazolyl und Oxazolyl mit einem Substituenten ausgewählt aus der Gruppe Fluor, Chlor, Cyano, Methyl, Ethyl, Methoxy, Amino, Hydroxycarbonyl, Methoxycarbonyl, Ethoxycarbonyl und Aminocarbonyl substituiert sein können,
R² für eine Gruppe der Formel 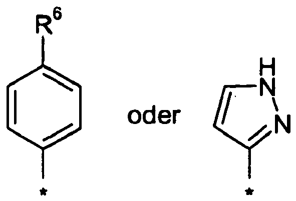 steht, wobei
* die Anknüpfstelle an den Bicyclus bedeutet,
R⁶ für Wasserstoff oder (C₁-C₄)-Alkoxy steht,
worin (C₂-C₄)-Alkoxy mit 1 oder 2 Substituenten Hydroxy substituiert sein kann,
R³ für Amino steht,
sowie ihre Salze, Solvate und Solvate der Salze.

5. Verfahren zur Herstellung von Verbindungen der Formel (I), wie in den Ansprüchen 1 bis 4 definiert und in welcher R³ für Amino steht, **dadurch gekennzeichnet, dass** man eine Verbindung der Formel (II) 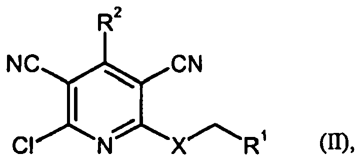 in welcher X, R¹ und R² jeweils die in den Ansprüchen 1 bis 4 angegebenen Bedeutungen haben,
[A] in einem inerten Lösungsmittel in Gegenwart einer geeigneten Base mit einer Verbindung der Formel (III-A) 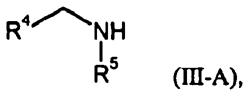 in welcher R⁴ und R⁵ jeweils die in den Ansprüchen 1 bis 4 angegebenen Bedeutungen haben,
zu einer Verbindung der Formel (IV-A) 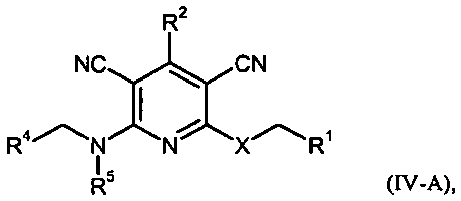 in welcher X, R¹, R², R⁴ und R⁵ jeweils die in den Ansprüchen 1 bis 4 angegebenen Bedeutungen haben,
umsetzt, diese anschliessend in einem inerten Lösungsmittel und in Gegenwart einer geeigneten Base zu Verbindungen der Formel (I-A) 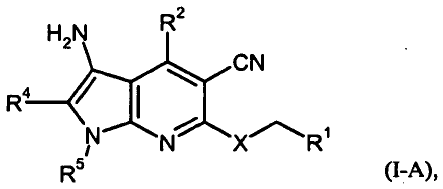 in welcher X, R¹, R², R⁴ und R⁵ jeweils die in den Ansprüchen 1 bis 4 angegebenen Bedeutungen haben,
cyclisiert,
oder
[B] in einem inerten Lösungsmittel in Gegenwart einer geeigneten Base mit einer Verbindung der Formel (III-B) 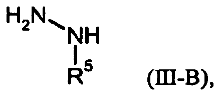 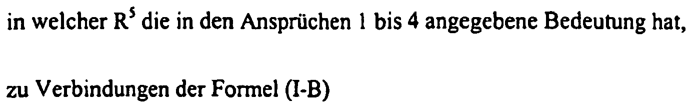 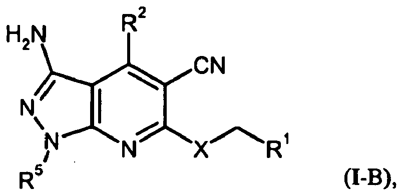 in welcher X, R¹, R² und R⁵ jeweils die in den Ansprüchen 1 bis 4 angegebenen Bedeutungen haben,
cyclisiert,
anschließend gegebenenfalls vorhandene Schutzgruppen abspaltet und die resultierenden Verbindungen der Formeln (I-A) und (I-B) gegebenenfalls mit den entsprechenden (*i*) Lösungsmitteln und/oder (*ii*) Basen oder Säuren in ihre Solvate, Salze und/oder Solvate der Salze überführt.

6. Verbindung der Formel (I), wie in einem der Ansprüche 1 bis 4 definiert, zur Behandlung und/oder Prävention von Krankheiten.

7. Verbindung der Formel (I), wie in einem der Ansprüche 1 bis 4 definiert, zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

8. Verbindung der Formel (I), wie in einem der Ansprüche 1 bis 4 definiert, zur Verwendung in einem Verfahren zur Behandlung und/oder Prophylaxe von Diabetes, Metabolischem Syndrom und Dyslipidämien.

9. Verwendung einer Verbindung der Formel (I), wie in einem der Ansprüche 1 bis 4 definiert, zur Herstellung eines Arzneimittels zur Behandlung und/oder Prävention von koronarer Herzerkrankung, akutem Koronarsyndrom, Angina pectoris, Herzinsuffizienz, Myokardinfarkt, Vorhofflimmern und Hypertonie.

10. Verwendung einer Verbindung der Formel (I), wie in einem der Ansprüche 1 bis 4 definiert, zur Herstellung eines Arzneimittels zur Behandlung und/oder Prävention von Diabetes, Metabolischem Syndrom und Dyslipidämien.

11. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

12. Arzneimittel enthaltend eine Verbindung der Formel (I), wie in einem der Ansprüche 1 bis 4 definiert, in Kombination mit einem oder mehreren weiteren Wirkstoffen ausgewählt aus der Gruppe bestehend aus den Fettstoffwechsel verändernden Wirkstoffen, Antidiabetika, blutdrucksenkenden Wirkstoffen und antithrombotisch wirkenden Mitteln.

13. Arzneimittel nach Anspruch 11 oder 12 zur Behandlung und/oder Prävention von koronarer Herzerkrankung, akutem Koronarsyndrom, Angina pectoris, Herzinsuffizienz, Myokardinfarkt, Vorhofflimmern und Hypertonie.

14. Arzneimittel nach Anspruch 11 oder 12 zur Behandlung und/oder Prävention von Diabetes, Metabolischem Syndrom und Dyslipidämien.

15. Verwendung mindestens einer Verbindung der Formel (I), wie in einem der Ansprüche 1 bis 5 definiert, oder eines Arzneimittels, wie in einem der Ansprüche 11 bis 13 definiert zur Herstellung eines Medikamentes zur Behandlung und/oder Prävention von koronarer Herzerkrankung, akutem Koronarsyndrom, Angina pectoris, Herzinsuffizienz, Myokardinfarkt, Vorhofflimmern und Hypertonie in Menschen und Tieren.

16. Verwendung mindestens einer Verbindung der Formel (I), wie in einem der Ansprüche 1 bis 4 definiert, oder eines Arzneimittels, wie in einem der Ansprüche 11, 12 und 14 definiert zur Herstellung eines Medikamentes zur Behandlung und/oder Prävention von Diabetes, Metabolischem Syndrom und Dyslipidämien in Menschen und Tieren.

## Claims

1. Compound of the formula (I) 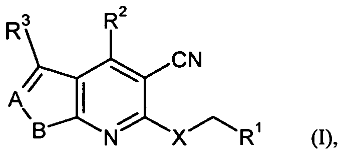 in which
either
A represents CR⁴ or N,
where
R⁴ represents (C₁-C₄)-alkoxycarbonyl, aminocarbonyl, mono-(C₁-C₄)-alkylaminocarbonyl or di-(C₁-C₄)-alkylaminocarbonyl,
B represents NR⁵,
where
R⁵ represents hydrogen or (C₁-C₄)-alkyl,
in which (C₁-C₄)-alkyl may be substituted by a (C₁-C₄)-alkoxycarbonyl substituent,
X represents O or S,
R¹ represents (C₆-C₁₀)-aryl or 5- to 10-membered heteroaryl,
where (C₆-C₁₀)-aryl and 5- to 10-membered heteroaryl may be substituted by 1 or 2 substituents independently of one another selected from the group consisting of halogen, nitro, cyano, (C₁-C₆)-alkyl, trifluoromethyl, hydroxyl, (C₁-C₆)-alkoxy, amino, mono-(C₁-C₆)-alkylamino, di-(C₁-C₆)-alkylamino, hydroxycarbonyl, (C₁-C₆)-alkoxycarbonyl, aminocarbonyl, mono-(C₁-C₆)-alkylaminocarbonyl, di-(C₁-C₆)-alkylaminocarbonyl, pyrrolidino, piperidino, morpholino, piperazino and N'-(C₁-C₄)-alkylpiperazino, phenyl and 5- or 6-membered heteroaryl,
in which phenyl and 5- or 6-membered heteroaryl may be substituted by 1 to 3 substituents independently of one another selected from the group consisting of halogen, nitro, cyano, (C₁-C₆)-alkyl, difluoromethyl, trifluoromethyl, hydroxyl, (C₁-C₆)-alkoxy, difluoromethoxy, trifluoromethoxy, amino, mono-(C₁-C₆)-alkylamino, di-(C₁-C₆)-alkyl-amino, hydroxycarbonyl and (C₁-C₆)-alkoxycarbonyl,
R² represents (C₅-C₆)-cycloalkyl, 5- or 6-membered heterocyclyl, phenyl or 5- or 6-membered heteroaryl,
where (C₅-C₆)-cycloalkyl may be substituted by 1 or 2 substituents independentlyof one another selected from the group consisting of (C₁-C₆)-alkyl, hydroxyl, oxo, (C₁-C₆)-alkoxy, amino, mono-(C₁-C₆)-alkylamino and di-(C₁-C₆)-alkylamino,
in which (C₁-C₆)-alkyl and (C₁-C₆)-alkoxy may be substituted by 1 or 2 substituents independently of one another selected from the group consisting of hydroxyl, (C₁-C₄)-alkoxy and (C₃-C₇)-cycloalkyl, in which (C₃-C₇)-cycloalkyl for its part may be substituted by 1 or 2 substituents independently of one another selected from the group consisting of (C₁-C₄)-alkyl, hydroxyl, oxo and (C₁-C₄)-alkoxy,
and
where 5- or 6-membered heterocyclyl may be substituted by 1 to 3 substituents independently of one another selected from the group consisting of oxo, thioxo,hydroxyl, (C₁-C₆)-alkyl, (C₁-C₆)-alkoxy, (C₁-C₆)-alkylcarbonyl, amino, mono-(C₁-C₆)-alkylamino, di-(C₁-C₆)-alkylamino and (C₃-C₇)-cycloalkyl,
in which (C₁-C₆)-alkyl may be substituted by 1 to 3 substituents independently of one another selected from the group consisting of fluorine, oxo, hydroxyl, trifluoromethyl, (C₁-C₄)-alkoxy, (C₁-C₄)-alkylcarbonyloxy, amino, mono-(C₁-C₄)-alkylamino, di-(C₁-C₄)-alkylamino and (C₃-C₇)-cycloalkyl,
in which (C₃-C₇)-cycloalkyl for its part may be substituted by 1 or 2 substituents independently of one another selected from the group consisting of (C₁-C₄)-alkyl, hydroxyl, oxo and (C₁-C₄)-alkoxy, and
in which (C₁-C₆)-alkylcarbonyl may be substituted by a substituent selected from the group consisting of hydroxyl and (C₁-C₄)-alkoxy,
and
in which (C₃-C₇)-cycloalkyl may be substituted by 1 or 2 substituents independently of one another selected from the group consisting of (C₁-C₄)-alkyl, hydroxyl, oxo and (C₁-C₄)-alkoxy,
and
where phenyl and 5- or 6-membered heteroaryl may be substituted by 1 to 3 substituents independently of one another selected from the group consisting of halogen, cyano, hydroxyl, (C₁-C₆)-alkyl, (C₁-C₆)-alkoxy, (C₃-C₇)-cycloalkoxy and -NR^{A}R^{B},
in which (C₁-C₆)-alkyl may be substituted by 1 to 3 substituents selected from the group consisting of fluorine, and
in which (C₁-C₆)-alkoxy may be substituted by 1 to 3 substituents independently of one another selected from the group consisting of fluorine, trifluoromethyl, (C₃-C₇)-cycloalkyl, oxo, hydroxyl, (C₁-C₄)-alkoxy, hydroxycarbonyl, amino, mono-(C₁-C₄)-alkylamino and di-(C₁-C₄)-alkylamino,
and
in which (C₃-C₇)-cycloalkoxy may be substituted by 1 or 2 substituents independently of one another selected from the group consisting of (C₁-C₄)-alkyl, hydroxyl, oxo and (C₁-C₄)-alkoxy,
and
in which
R^{A} represents hydrogen or (C₁-C₆)-alkyl,
in which (C₁-C₆)-alkyl for its part may be substituted by a substituent selected from the group consisting of hydroxyl and (C₁-C₄)-alkoxy,
R^{B} represents hydrogen, (C₁-C₆)-alkyl, (C₃-C₇)-cycloalkyl, (C₁-C₄)-alkylsulfonyl or (C₃-C₇)-cycloalkylsulfonyl, in which (C₁-C₆)-alkyl for its part may be substituted by 1 or 2 substituents independently of one another selected from the group consisting of (C₃-C₇)-cycloalkyl, oxo, hydroxyl, (C₁-C₄)-alkoxy, hydroxycarbonyl, amino, mono-(C₁-C₄)-alkylamino and di-(C₁-C₄)-alkylamino,
and
in which (C₃-C₇)-cycloalkyl for its part may be substituted by 1 or2 substituents independently of one another selected from the group consisting of (C₁-C₄)-alkyl, hydroxyl, oxo and (C₁-C₄)-alkoxy,
or
in which two adjacent substituents at the phenyl together with the carbon atoms to which they are attached may form a 1,3-dioxolane or 2,2-difluoro-1,3-dioxolane,
R³ represents hydrogen, (C₁-C₄)-alkyl, (C₁-C₄)-alkoxy, amino, mono-(C₁-C₄)-alkylamino, di-(C₁-C₄)-alkylamino or (C₁-C₄)-alkylcarbonyl
and the N-oxides, salts, solvates, salts of the N-oxides and solvates of the N-oxides thereof.

2. Compound of the formula (I) according to Claim 1 in which
A represents CR⁴ or N,
where
R⁴ represents (C₁-C₄)-alkoxycarbonyl, aminocarbonyl or mono-(C₁-C₄)-alkylaminocarbonyl,
and
B represents NR⁵,
where
R⁵ represents hydrogen, methyl or ethyl,
in which methyl and ethyl may be substituted by a substituent selected from the group consisting of methoxycarbonyl and ethoxycarbonyl,
X represents O or S,
R¹ represents phenyl or 5- or 6-membered heteroaryl,
where phenyl and 5- or 6-membered heteroaryl may be substituted by 1 or 2 substituents independently of one another selected from the group consisting of fluorine, chlorine, cyano, (C₁-C₄)-alkyl, trifluoromethyl, hydroxyl, (C₁-C₄)-alkoxy, amino, hydroxycarbonyl, (C₁-C₄)-alkoxycarbonyl, aminocarbonyl, phenyl and 5- or 6-membered heteroaryl,
in which phenyl and 5- or 6-membered heteroaryl may be substituted by 1 to 3 substituents independently of one another selected from the group consisting of fluorine, chlorine, nitro, cyano, (C₁-C₄)-alkyl, difluoromethyl, trifluoromethyl, hydroxyl, methoxy, ethoxy, amino, hydroxycarbonyl and (C₁-C₄)-alkoxycarbonyl,
R² represents cyclohexyl, tetrahydropyranyl, piperidinyl, piperazinyl, morpholinyl, phenyl, pyrazolyl, imidazolyl, oxazolyl, thiazolyl or pyridyl,
where cyclohexyl may be substituted by a substituent selected from the group consisting of hydroxyl and (C₁-C₄)-alkoxy,
in which (C₂-C₄)-alkoxy may be substituted by 1 or 2 substituents independently of one another selected from the group consisting of hydroxyl and methoxy,
where
where piperidinyl, piperazinyl and morpholinyl may be substituted by a substituent selected from the group consisting of (C₁-C₄)-alkyl, hydroxyl, (C₁-C₄)-alkoxy and (C₁-C₄)-alkylcarbonyl
in which (C₁-C₄)-alkyl may be substituted by 1 or 2 substituents independently of one another selected from the group consisting of hydroxyl, methoxy, ethoxy, methylcarbonyloxy and ethylcarbonyloxy,
and
in which (C₁-C₄)-alkylcarbonyl may be substituent selected from the group consisting of hydroxyl, methoxy and ethoxy,
andwhere phenyl and pyridyl may be substituted by 1 to 3 substituents independently of one another selected from the group consisting of fluorine, chlorine, cyano, hydroxyl, (C₁-C₄)-alkyl and (C₁-C₄)-alkoxy,
in which (C₂-C₄)-alkoxy may be substituted by 1 or 2 substituents independently of one another selected from the group consisting of oxo, hydroxyl, (C₁-C₄)-alkoxy, hydroxycarbonyl and amino,
and
where pyrazolyl, imidazolyl, oxazolyl and thiazolyl may be substituted by 1 or 2 substituents independently of one another selected from the group consisting of fluorine, chlorine, cyano, hydroxyl, (C₁-C₄)-alkyl and (C₁-C₄)-alkoxy,
in which (C₂-C₄)-alkoxy may be substituted by 1 or 2 substituents independently of one another selected from the group consisting of oxo, hydroxyl, (C₁-C₄)-alkoxy, hydroxycarbonyl and amino,
R³ represents hydrogen, amino, methylamino or dimethylamino,
and the salts, solvates and solvates of the salts thereof.

3. Compound of the formula (I) according to Claim I in which
A represents CR⁴ or N,
where
R⁴ represents (C₁-C₄)-alkoxycarbonyl, aminocarbonyl or mono-(C₁-C₄)-alkylaminocarbonyl,
and
B represents NR⁵,
where
R⁵ represents hydrogen, methyl or ethyl,
in which methyl and ethyl may be substituted by a substituent selected from the group consisting of methoxycarbonyl and ethoxycarbonyl,
X represents O or S,
R¹ represents phenyl or 5- or 6-membered heteroaryl,
where phenyl and 5- or 6-membered heteroaryl may be substituted by 1 or 2 substituents independently of one another selected from the group consisting of fluorine, chlorine, cyano, (C₁-C₄)-alkyl, trifluoromethyl, hydroxyl, (C₁-C₄)-alkoxy, amino, hydroxycarbonyl, (C₁-C₄)-alkoxycarbonyl, aminocarbonyl, phenyl and 5- or 6-membered heteroaryl,
in which phenyl and 5- or 6-membered heteroaryl may be substituted by 1 to 3 substituents independently of one another selected from the group consisting of fluorine, chlorine, nitro, cyano, (C₁-C₄)-alkyl, difluoromethyl, trifluoromethyl, hydroxyl, methoxy, ethoxy, amino, hydroxycarbonyl and (C₁-C₄)-alkoxycarbonyl,
R² represents a group of the formula 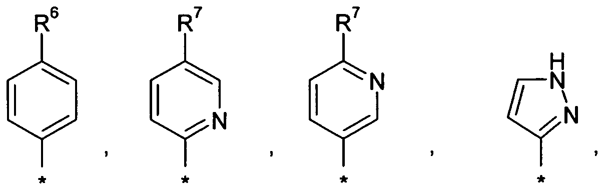 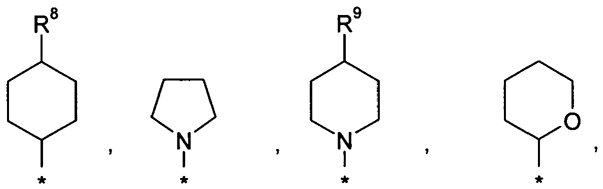 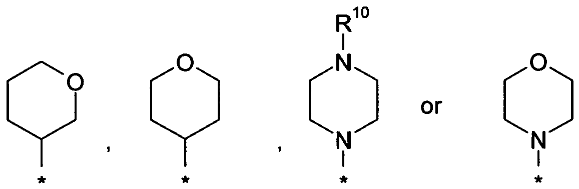 where
* represents the point of attachment to the bicycle,
R⁶ represents hydrogen or (C₁-C₄)-alkoxy,
in which (C₂-C₄)-alkoxy may be substituted by 1 or 2 hydroxyl substituents,
R⁷ represents hydrogen or (C₁-C₄)-alkoxy,
in which (C₂-C₄)-alkoxy may be substituted by 1 or 2 hydroxyl substituents,
R⁸ represents hydrogen, hydroxyl, methoxy, ethoxy or 2-hydroxyethoxy,
R⁹ represents hydrogen or hydroxyl,
and
R¹⁰ represents hydrogen or methyl,
R³ represents hydrogen, amino, methylamino or dimethylamino,
and the salts, solvates and solvates of the salts thereof.

4. Compound of the formula (I) according to Claim 1, 2 or 3 in which
A represents CR⁴ or N,
where
R⁴ represents methoxycarbonyl, aminocarbonyl or methylaminocarbonyl,
and
B represents NR⁵,
where
R⁵ represents hydrogen or methyl, in which methyl may be substituted by a methoxycarbonyl substituent,
X represents O or S,
R¹ represents thiazolyl, oxazolyl, phenyl or pyridyl, where phenyl and pyridyl may be substituted by 1 or 2 substituents independently of one another selected from the group consisting of fluorine, chlorine, cyano, methyl, ethyl, methoxy, amino, hydroxycarbonyl, methoxycarbonyl, ethoxycarbonyl and aminocarbonyl,
and
where thiazolyl and oxazolyl are substituted by a phenyl group substituent, in which phenyl may be substituted by a substituent selected from the group consisting of fluorine, chlorine, cyano, methyl, methoxy and hydroxycarbonyl,
and
where thiazolyl and oxazolyl may be substituted by a substituent selected from the group consisting of fluorine, chlorine, cyano, methyl, ethyl, methoxy, amino, hydroxycarbonyl, methoxycarbonyl, ethoxycarbonyl and aminocarbonyl,represents thiazolyl, oxazolyl, phenyl or pyridyl, where phenyl and pyridyl may be substituted by 1 or 2 substituents independently of one another selected from the group consisting of fluorine, chlorine, cyano, methyl, ethyl, methoxy, amino, hydroxycarbonyl, methoxycarbonyl, ethoxycarbonyl and aminocarbonyl,
and
where thiazolyl and oxazolyl are substituted by a phenyl group substituent, in which phenyl may be substituted by a substituent selected from the group consisting of fluorine, chlorine, cyano, methyl, methoxy and hydroxycarbonyl,
and
where thiazolyl and oxazolyl may be substituted by a substituent selected from the group consisting of fluorine, chlorine, cyano, methyl, ethyl, methoxy, amino, hydroxycarbonyl, methoxycarbonyl, ethoxycarbonyl and aminocarbonyl,
R² represents a group of the formula 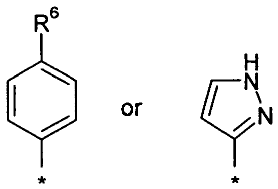 where
* represents the point of attachment to the bicycle,
R⁶ represents hydrogen or (C₁-C₄)-alkoxy,
in which (C₂-C₄)-alkoxy may be substituted by I or 2 hydroxyl substituents,
R³ represents amino,
and the salts, solvates and solvates of the salts thereof.

5. Process for preparing compounds of the formula (I) as defined in Claims 1 to 4 and in
which R³ represents amino, **characterized in that** a compound of the formula (II) 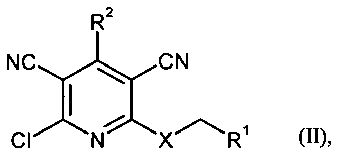 in which X, R¹ and R² each have the meanings given in Claims 1 to 4,
[A] is reacted in an inert solvent in the presence of a suitable base with a compound of the formula (III-A) 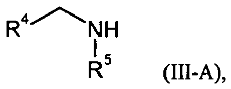 in which R⁴ and R⁵ each have the meanings given in Claims I to 4,
to give a compound of the formula (IV-A) 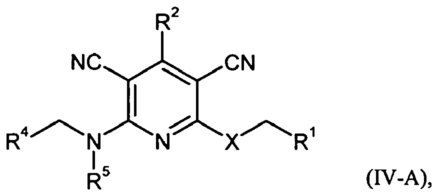 in which X, R¹, R², R⁴ and R⁵ each have the meanings given in Claims 1 to 4,
and this is then cyclized in an inert solvent and in the presence of a suitable base to give compounds of the formula (I-A) 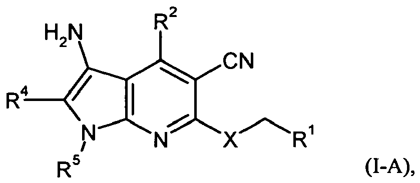 in which X, R¹, R², R⁴ and R⁵ each have the meanings given in Claims 1 to 4,
or
[B] is cyclized in an inert solvent in the presence of a suitable base with a compound of the formula (III-B) 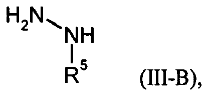 in which R⁵ has the meaning given in Claims 1 to 4, to give compounds of the formula (I-B) 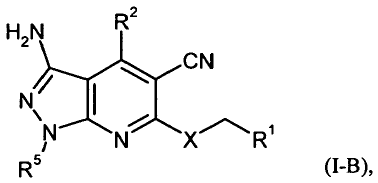 in which X, R¹, R² and R⁵ each have the meanings given in Claims I to 4,
any protective groups present are then cleaved off and the resulting compounds of the formulae (I-A) and (I-B) are, if appropriate, converted with the appropriate (i) solvents and/or (ii) bases or acids into their solvates, salts and/or solvates of the salts.

6. Compound of the formula (I) as defined in any of Claims 1 to 4 for the treatment and/or prevention of diseases.

7. Compound of the formula (I) as defined in any of Claims 1 to 4 for use in a method for the treatment and/or prophylaxis of coronary heart disease, acute coronary syndrome, angina pectoris, heart failure, myocardial infarction and atrial fibrillation.

8. Compound of the formula (I) as defined in any of Claims 1 to 4 for use in a method for the treatment and/or prophylaxis of diabetes, metabolic syndrome and dyslipidemias.

9. Use of a compound of the formula (I) as defined in any of Claims 1 to 4 for preparing a medicament for the treatment and/or prevention of coronary heart disease, acute coronary syndrome, angina pectoris, heart failure, myocardial infarction, atrial fibrillation and hypertension.

10. Use of a compound of the formula (I) as defined in any of Claims 1 to 4 for preparing a medicament for the treatment and/or prevention of diabetes, metabolic syndrome and dyslipidemias.

11. Medicament comprising a compound of the formula (I) as defined in any of Claims 1 to 4 in combination with an inert, nontoxic, pharmaceutically suitable auxiliary.

12. Medicament comprising a compound of the formula (I) as defined in any of Claims 1 to 4 in combination with one or more further active compounds selected from the group consisting of lipid metabolism-modifying active compounds, antidiabetics, antihypertensive drugs and antithrombotic drugs.

13. Medicament according to Claim 11 or 12 for the treatment and/or prevention of coronary heart disease, acute coronary syndrome, angina pectoris, heart failure, myocardial infarction, atrial fibrillation and hypertension.

14. Medicament according to Claim 11 or 12 for the treatment and/or prevention of diabetes, metabolic syndrome and dyslipidemias.

15. Use of at least one compound of the formula (I) as defined in any of Claims 1 to 5, or of a medicament as defined in any of Claims 11 to 13 for preparing a medicine for the treatment and/or prevention of coronary heart disease, acute coronary syndrome, angina pectoris, heart failure, myocardial infarction, atrial fibrillation and hypertension in humans and animals.

16. Use of at least one compound of the formula (I) as defined in any of Claims 1 to 4, or of a medicament as defined in any of Claims 11, 12 and 14 for preparing a medicine for the treatment and/or prevention of diabetes, metabolic syndrome and dyslipidemias in humans and animals.

## Revendications

1. Composé de la formule (I) 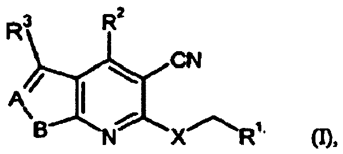 dans laquelle
soit
A est CR⁴ ou N,
dans laquelle
R⁴ est un alcoxycarbonyle (C₁-C₄), aminocarbonyle, mono-(C₁-C₄)-alkylaminocarbonyle ou di-alkylaminocarbonyle-(C₁-C₄),
et
B est NR⁵,
dans laquelle
R⁵ est un hydrogène ou alkyle-(C₁-C₄),
dans laquelle l'alkyle-(C₁-C₄) peut être substitué avec un substituant alcoxycarbonyle-(C₁-C₄),
X est O ou S,
R¹ est un aryle-(C₆-C₁₀) ou hétéroaryle avec 5 à 10 maillons,
dans laquelle aryle-(C₆-C₁₀) et hétéroaryle avec 5 à 10 maillons peuvent être substitués avec 1 ou 2 substituants sélectionnés indépendamment l'un de l'autre dans le groupe halogène, nitro, cyano, alkyle-(C₁-C₆), trifluorométhyle, hydroxy, alcoxy-(C₁-C₆), amino, mono-alkylamino-(C₁-C₆), di-alkylamino-(C₁-C₆), hydroxycarbonyle, alcoxycarbonyle-(C₁-C₆), aminocarbonyle, mono-alkylaminocarbonyle-(C₁-C₆), di-alkylaminocarbonyle-(C₁-C₆), pyrrolidino, pipéridino, morpholino, pipérazino et N'-alkylpipérazino-(C₁-C₄), phényle et hétéroaryle avec 5 ou 6 maillons,
dans laquelle phényle et hétéroaryle avec 5 ou 6 maillons peuvent être substitués avec 1 à 3 substituants sélectionnés indépendamment l'un de l'autre dans le groupe halogène, nitro, cyano, alkyle-(C₁-C₆), difluorométhyle, trifluorométhyle, hydroxy, alcoxy-(C₁-C₆), difluorométhoxy, trifluorométhoxy, amino, mono-alkylamino-(C₁-C₆), di-alkylamino-(C₁-C₆), hydroxycarbonyle et alkoxycarbonyle-(C₁-C₆),
R² est un cycloalkyle-(C₅-C₆), hétérocyclyle avec 5 ou 6 maillons, phényle ou hétéroaryle avec 5 ou 6 maillons,
dans laquelle cycloalkyle-(C₅-C₆) peut être substitué avec 1 ou 2 substituants sélectionnés indépendamment l'un de l'autre dans le groupe alkyle-(C₁-C₆), hydroxy, oxo, alcoxy-(C₁-C₆), amino, mono-alkylamino-(C₁-C₆) et di-alkylamino-(C₁-C₆),
dans laquelle alkyle-(C₁-C₆) et alcoxy-(C₁-C₆) peuvent être substitués avec 1 ou 2 substituants sélectionnés indépendamment l'un de l'autre dans le groupe hydroxy, alcoxy-(C₁-C₄) et cycloalkyle-(C₃-C₇),
dans laquelle cycloalkyle-(C₃-C₇) peut de son côté être substitué avec 1 ou 2 substituants sélectionnés indépendamment l'un de l'autre dans le groupe alkyle-(C₁-C₄), hydroxy, oxo et alcoxy-(C₁-C₄),
et
dans laquelle hétérocyclyle avec 5 ou 6 maillons peut être substitué avec 1 à 3 substituants sélectionnés indépendamment l'un de l'autre dans le groupe oxo, thioxo, hydroxy, alkyle-(C₁-C₆), alcoxy-(C₁-C₆), alkylcarbonyle-(C₁-C₆), amino, mono-alkylamino-(C₁-C₆), di-alkylamino-(C₁-C₆) et cycloalkyle-(C₃-C₇),
dans laquelle alkyle-(C₁-C₆) peut être substitué avec 1 à 3 substituants sélectionnés indépendamment l'un de l'autre dans le groupe fluor, oxo, hydroxy, trifluorométhyle, alcoxy-(C₁-C₄), alkylcarbonyloxy-(C₁-C₄), amino, mono-alkylamino-(C₁-C₄), di-alkylamino-(C₁-C₄) et cycloalkyle-(C₃-C₇),
dans laquelle cycloalkyle-(C₃-C₇) peut de son côté être substitué avec 1 ou 2 substituants sélectionnés indépendamment l'un de l'autre dans le groupe alkyle-(C₁-C₄), hydroxy, oxo et alcoxy-(C₁-C₄),
et
dans laquelle alkylcarbonyle-(C₁-C₆) peut être substitué avec un substituant sélectionné dans le groupe hydroxy et alcoxy-(C₁-C₄)
et
dans laquelle cycloalkyle-(C₃-C₇) peut être substitué avec 1 ou 2 substituants sélectionnés indépendamment l'un de l'autre dans le groupe alkyle-(C₁-C₄), hydroxy, oxo et alcoxy-(C₁-C₄),
et
dans laquelle phényle et hétéroaryle avec 5 ou 6 maillons peuvent être substitués avec 1 à 3 substituants sélectionnés indépendamment l'un de l'autre dans le groupe halogène, cyano, hydroxy, alkyle-(C₁-C₆), alcoxy-(C₁-C₆), cycloalcoxy-(C₃-C₇) et -NR^{A}R^{B},
dans laquelle alkyle-(C₁-C₆) peut être substitué avec 1 à 3 substituants sélectionnés dans le groupe fluor,
etdans laquelle alcoxy-(C₁-C₆) peut être substitué avec 1 à 3 substituants sélectionnés indépendamment l'un de l'autre dans le groupe fluor, trifluorométhyle, cycloalkyle-(C₃-C₇), oxo, hydroxy, amcoxy-(C₁-C₄), hydroxycarbonyle, amino, mono-alkylamino-(C₁-C₄) et di-alkylamino-(C₁-C₄),
et
dans laquelle cycloalcoxy-(C₃-C₇) peut être substitué avec 1 ou 2 substituants sélectionnés indépendamment l'un de l'autre dans le groupe alkyle-(C₁-C₄), hydroxy, oxo et alcoxy-(C₁-C₄),
et
dans laquelle
R^{A} est un hydrogène ou alkyle-(C₁-C₆),
dans laquelle alkyle-(C₁-C₆) peut de son côté être substitué avec un substituant sélectionné dans le groupe hydroxy et alcoxy-(C₁-C₄),
R^{B} est un hydrogène, alkyle-(C₁-C₆), cycloalkyle-(C₃-C₇), alkylsulfonyle-(C₁-C₄) ou cycloalkylsulfonyl-(C₃-C₇),
dans laquelle alkyle-(C₁-C₆) peut de son côté être substitué avec 1 ou 2 substituants sélectionnés indépendamment l'un de l'autre dans le groupe cycloalkyle-(C₃-C₇), oxo, hydroxy, alcoxy-(C₁-C₄), hydroxycarbonyle, amino, mono-alkylamino-(C₁-C₄) et di-alkylamino-(C₁-C₄),
et
dans laquelle cycloalkyle-(C₃-C₇) peut de son côté être substitué avec 1 ou 2 substituant sélectionnés indépendamment l'un de l'autre dans le groupe alkyle-(C₁-C₄), hydroxy, oxo et alcoxy-(C₁-C₄),
ou
dans laquelle deux substituants voisins sur le phényle peuvent former, avec les atomes de carbone auxquels ils sont liés, un 1,3-dioxolane ou 2,2-difluoro-1,3-dioxolane,
R³ est un hydrogène, alkyle-(C₁-C₄), alcoxy-(C₁-C₄), amino, mono-alkylamino-(C₁-C₄), di-alkylamino-(C₁-C₄) ou alkylcarbonyle-(C₁-C₄),
ainsi que ses N-oxydes, sels, solvates, sels des N-oxydes et solvates des N-oxydes.

2. Composé de la formule (I) selon la revendication 1, dans laquelle
A est CR⁴ ou N,
dans laquelle
R⁴ est alcoxycarbonyle-(C₁-C₄), aminocarbonyle ou mono-alkylaminocarbonyle-(C₁-C₄),
et
B est NR⁵,
dans laquelle
R⁵ est un hydrogène, méthyle ou éthyle,
dans laquelle méthyle et éthyle peuvent être substitués avec un substituant sélectionné dans le groupe méthoxycarbonyle et éthoxycarbonyle,
X est O ou S,
R¹ est un phényle ou hétéroaryle avec 5 ou 6 maillons,
dans laquelle phényle et hétéroaryle avec 5 ou 6 maillons peuvent être substitués avec 1 ou 2 substituants sélectionnés indépendamment l'un de l'autre dans le groupe fluor, chlore, cyano, alkyle-(C₁-C₄), trifluorométhyle, hydroxy, alcoxy-(C₁-C₄), amino, hydroxycarbonyle, alcoxycarbonyle-(C₁-C₄), aminocarbonyle, phényle et hétéroaryle avec 5 ou 6 maillons,
dans laquelle phényle et hétéroaryle avec 5 ou 6 maillons peuvent être substitués avec 1 à 3 substituants sélectionnés indépendamment l'un de l'autre dans le groupe fluor, chlore, nitro, cyano, alkyle-(C₁-C₄), difluorométhyle, trifluorométhyle, hydroxy, méthoxy, éthoxy, amino, hydroxycarbonyle et alcoxycarbonyle-(C₁-C₄),
R² est cyclohexyle, tétrahydropyranyle, pipéridinyle, pipérazinyle, morpholinyle, phényle, pyrazolyle, imidazolyle, oxazolyle, thiazolyle ou pyridyle,
dans laquelle cyclohexyle peut êtyre substitué avec un substituant sélectionné dans le groupe hydroxy et alcoxy-(C₁-C₄),
dans laquelle alcoxy-(C₂-C₄) peut être substitué avec 1 ou 2 substituants sélectionnés indépendamment l'un de l'autre dans le groupe hydroxy et méthoxy,
et
dans laquelle pipéridinyle, pipérazinyle et morpholinyle peuvent être substitués avec un substituant sélectionné dans le groupe alkyle-(C₁-C₄), hydroxy, alcoxy-(C₁-C₄) et alkylcarbonyle-(C₁-C₄),
dans laquelle alkyle-(C₁-C₄) peut être substitué avec 1 ou 2 substituants sélectionnés indépendamment l'un de l'autre dans le groupe hydroxy, méthoxy, éthoxy, méthylcarbonyloxy et éthylcarbonyloxy,
et
dans laquelle alkylcarbonyle-(C₁-C₄) peut être substitué avec un substituant sélectionné dans le groupe hydroxy, méthoxy et éthoxy,
et
dans laquelle phényle et pyridyle peuvent être substitués avec 1 à 3 substituants sélectionnés indépendamment l'un de l'autre dans le groupe fluor, chlore, cyano, hydroxy, alkyle-(C₁-C₄) et alcoxy-(C₁-C₄),
dans laquelle alcoxy-(C₂-C₄) peut être substitué avec 1 ou 2 substituants sélectionnés indépendamment l'un de l'autre dans le groupe oxo, hydroxy, alcoxy-(C₁-C₄) hydroxycarbonyle et amino, et
dans laquelle pyrazolyle, imidazolyle, oxazolyle et thiazolyle peuvent être substitués avec 1 ou 2 substituants sélectionnés indépendamment l'un de l'autre dans le groupe fluor, chlore, cyano, hydroxy, alkyle-(C₁-C₄) et alcoxy- (C₁-C₄),
dans laquelle alcoxy-(C₂-C₄) peut être susbtitué avec 1 ou 2 substituants sélectionnés indépendamment l'un de l'autre dans le groupe oxo, hydroxy, alcoxy-(C₁-C₄), hydroxycarbonyle et amino,
R³ est un hydrogène, amino, méthylamino ou diméthylamino,
ainsi que ses sels, solvates et solvates des sels.

3. Composé de la formule (I) selon la revendication 1,
dans laquelle
A est CR⁴ ou N,
dans laquelle
R⁴ est alkoxycarbonyle-(C₁-C₄), aminocarbonyle ou mono-alkylaminocarbonyle-(C₁-C₄),
et
B est NR⁵,
dans laquelle
R⁵ est un hydrogène, méthyle ou éthyle, dans laquelle méthyle et éthyle peuvent être substitués avec un substituant sélectionné dans le groupe méthoxycarbonyle et éthoxycarbonyle,
X est O ou S,
R¹ est phényle ou hétéroaryle avec 5 ou 6 maillons,
dans laquelle phényle et hétéroaryle avec 5 ou 6 maillons peuvent être substitués avec 1 ou 2 substituants sélectionnés indépendamment l'un de l'autre dans le groupe fluor, chlore, cyano, alkyle-(C₁-C₄), trifluorométhyle, hydroxy, alcoxy-(C₁-C₄), amino, hydroxycarbonyle, alcoxycarbonyle-(C₁-C₄), aminocarbonyle, phényle et hétéroaryle avec 5 ou 6 maillons,
dans laquelle phényle et hétéroaryle avec 5 ou 6 maillons peuvent être substitués avec 1 à 3 substituants sélectionnés indépendamment l'un de l'autre dans le groupe fluor, chlore, nitro, cyano, alkyle-(C₁-C₄), difluorométhyle, trifluorométhyle, hydroxy, méthoxy, éthoxy, amino, hydroxycarbonyle et alcoxycarbonyle-(C₁-C₄),
R² est un groupe de la formule 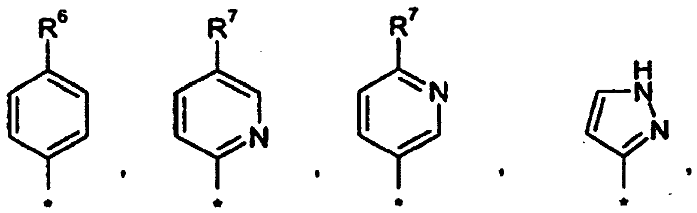 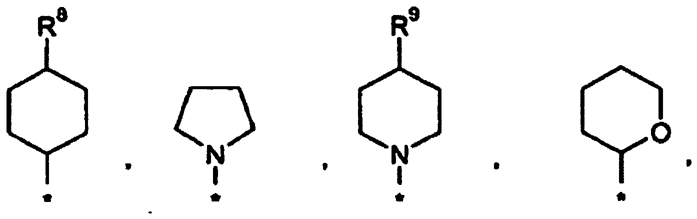 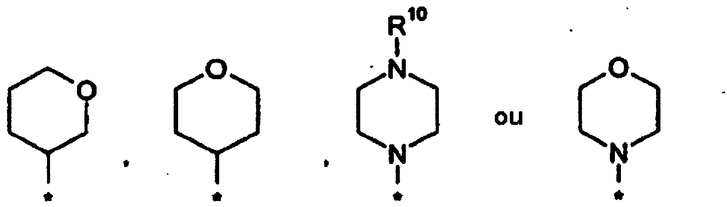 dans laquelle
* signifie le point de liaison au bicycle,
R⁶ est un hydrogène ou alcoxy-(C₁-C₄), dans laquelle alcoxy-(C₂-C₄) peut être substitué avec 1 ou 2 substituants hydroxy,
R⁷ est un hydrogène ou alcoxy-(C₁-C₄), dans laquelle alcoxy-(C₂-C₄) peut être substitué avec 1 ou 2 substituants hydroxy,
R⁸ est un hydrogène, hydroxy, méthoxy, éthoxy ou 2-hydroxyéthoxy,
R⁹ est un hydrogène ou hydroxy,
et
R¹⁰
est un hydrogène ou méthyle,
R³ est un hydrogène, amino, méthylamino ou diméthylamino,
ainsi que ses sels, solvates et solvates des sels.

4. Composé de la formule (I) selon la revendication 1, 2 ou 3, dans laquelle
A est CR⁴ ou N,
dans laquelle
R⁴ est méthoxycarbonyle, aminocarbonyle ou méthylaminocarbonyle,
et
B est NR⁵,
dans laquelle
R⁵ est un hydrogène ou méthyle,
dans laquelle méthyle peut être substitué avec un substituant méthoxycarbonyle,
X est O ou S
R¹ est thiazolyle, oxazolyle, phényle ou pyridyle, dans laquelle phényle et pyridyle peuvent être substitués avec 1 ou 2 substituants sélectionnés indépendamment l'un de l'autre dans le groupe fluor, chlore, cyano, méthyle, éthyle, méthoxy, amino, hydroxycarbonyle, méthoxycarbonyle, éthoxycarbonyle et aminocarbonyle,
et
dans laquelle thiazolyle et oxazolyle sont substitués avec un substituant du groupe phényle,
dans laquelle phényle peut être substitué avec un substituant sélectionné dans le groupe fluor, chlore, cyano, méthyle, méthoxy et hydroxycarbonyle,
et
dans laquelle thiazolyle et oxazolyle peuvent être substitués avec un substituant sélectionné dans le groupe fluor, chlore, cyano, méthyle, éthyle, méthoxy, amino, hydroxycarbonyle, méthoxycarbonyle, éthoxycarbonyle et aminocarbonyle,
R² est un groupe de la formule 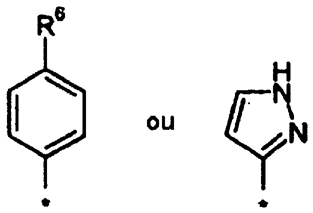 dans laquelle
* signifie le point de liaison au bicycle,
R⁶ est un hydrogène ou alkoxy-(C₁-C₄),
dans laquelle alcoxy-(C₂-C₄) peut êre substitué avec 1 ou 2 substituants hydroxy,
R³ est un amino,
ainsi que ses sels, solvates et solvates des sels.

5. Procédé pour la fabrication de composés de la formule (I), définie dans les revendications 1 à 4 et dans laquelle R³ est un amino, **caractérisé en ce que**
l'on convertit un composé de la formule (II) 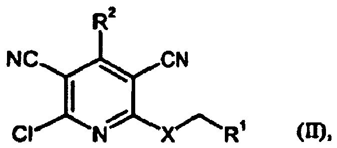 dans laquelle X, R¹ et R² ont respectivement les significations indiquées dans les revendications 1 à 4,
[A] dans un solvant inerte en présence d'une base appropriée avec un composé de la formule (III-A) 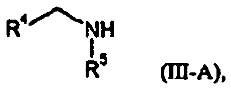 dans laquelle R⁴ et R⁵ ont respectivement les significqations indiquées dans les revendications 1 à 4,
en un composé de la formule (IV-A) 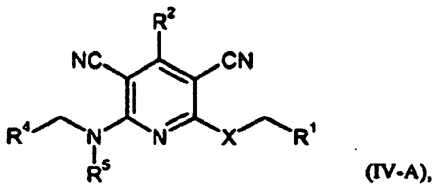 dans laquelle X, R¹, R², R⁴ et R⁵ ont respectivement les significations indiquées dans les revendications 1 à 4,
on cyclise ensuite celui-ci dans un solvant inerte en présence d'une base appropriée en composés de la formule (I-A) 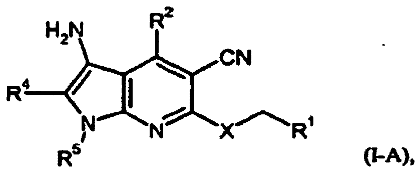 dans laquelle X, R¹, R², R⁴ et R⁵ ont respectivement les significations indiquées dans les revendications 1 à 4,
ou
[B] on le cyclise dans un solvant inerte en présence d'une base appropriée avec un composé de la formule (III-B) 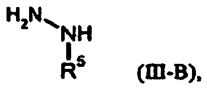 dans laquelle R⁵ a la signification indiquée dans les revendications 1 à 4,
en composés de la formule (I-B) 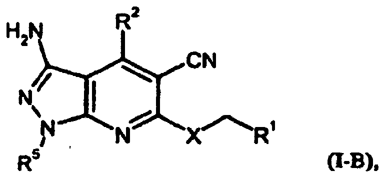 dans laquelle X, R¹, R² et R⁵ ont respectivement les significations indiquées dans les revendications 1 à 4,
on élimine ensuite les groupes de protection éventuellement présents et on transforme les composés résultants des formules (I-A) et (I-B) éventuellement avec les (i) solvants et/ou (ii) bases ou acides correspondants en leurs solvates, sels et/ou solvates des sels.

6. Composé de la formule (I), défini dans l'une quelconque des revendications 1 à 4, pour le traitement et/ou la prévention de maladies.

7. Composé de la formule (I), défini dans l'une quelconque des revendications 1 à 4, destiné à être utilisé dans un procédé de traitement et/ou la prophylaxie de maladies cardiaques coronariennes, du syndrome coronarien aigu, de l'angine de poitrine, de l'insuffisance cardiaque, de l'infarctus du myocarde et de la fibrillation auriculaire.

8. Composé de la formule (I), défini dans l'une quelconque des revendications 1 à 4, destiné à être utilisé dans un procédé de traitement et/ou de prophylaxie du diabète, du syndrome métabolique et de la dyslipidémie.

9. Utilisation d'un composé de la formule (I), défini dans l'une quelconque des revendications 1 à 4, pour la fabrication d'un médicament pour le traitement et/ou la prévention des maladies cardiaques coronariennes, du syndrome coronarien aigu, de l'angine de poitrine, de l'insuffisance cardiaque, de l'infarctus du myocarde, de la fibrillation auriculaire et de l'hypertonie.

10. Utilisation d'un composé de la formule (I), défini dans l'une quelconque des revendications 1 à 4, pour la fabrication d'un médicament pour le traitement et/ou la prévention du diabète, du syndrome métabolique et de la dyslipidémie.

11. Médicament contenant un composé de la formule (I), défini dans l'une quelconque des revendications 1 à 4, en combinaison avec un adjuvant inerte, non toxique, approprié au point de vue pharmaceutique.

12. Médicament contenant un composé de la formule (I), défini dans l'une quelconque des revendications 1 à 4, en combinaison avec un ou plusieurs autres principes actifs sélectionnés dans le groupe composé des principes actifs modifiant le métabolisme des graisses, des antidiabétiques, des principes actifs abaissant la pression sanguine et des agents à action antithrombotique.

13. Médicament selon la revendication 11 ou 12 pour le traitement et/ou la prévention de maladies cardiaques coronariennes, du syndrome coronarien aigu, de l'angine de poitrine, de l'insuffisance cardiaque, de l'infarctus du myocarde, de la fibrillation auriculaire et de l'hypertonie.

14. Médicament selon la revendication 11 ou 12 pour le traitement et/ou la prévention du diabète, du syndrome métabolique et de la dyslipidémie.

15. Utilisation d'au moins un composé de la formule (I), défini dans l'une quelconque des revendications 1 à 5, ou d'un médicament défini dans l'une quelconque des revendications 11 à 13, pour la fabrication d'un médicament pour le traitement et/ou la prévention des maladies cardiaques coronariennes, du syndrome coronarien aigu, de l'angine de poitrine, de l'insuffisance cardiaque, de l'infarctus du myocarde, de la fibrillation auriculaire et de l'hypertonie chez l'homme et chez l'animal.

16. Utilisation d'au moins un composé de la formule (I), défini dans l'une quelconque des revendications 1 à 4, ou d'un médicament, défini dans l'une quelconque des revendications 11, 12 et 14, pour la fabrication d'un médicament pour le traitement et/ou la prévention du diabète, du syndrome métabolique et de la dyslipidémie chez l'homme et chez l'animal.
